# EUROPEAN PATENT APPLICATION

(11) **EP 1 731 905 A1**
(43) Date of publication of application: **13.12.2006**
(21) Application number: 05727895.4
(22) Date of filing: 29.03.2005
(51) Int. Cl.: G01N 27/62, C07C 323/25, G01N 33/483, C07D 339/04, C07H 15/04, C07H 15/08

(54) **MASS ANALYSIS METHOD USING FINE METAL PARTICLES**

(30) Priority: 31.03.2004 JP 2004108285; 11.06.2004 JP 2004174847; 27.01.2005 JP 2005020442
(71) Applicant: Shionogi Co., Ltd., Osaka-shi, Osaka 541-0045 (JP)
(72) Inventor: NAGAHORI, Noriko, Sapporo-shi, Hokkaido 0010012 (JP); NIIKURA, Kenichi, Sapporo-shi, Hokkaido 0640806 (JP); NISHIMURA, Shinichiro, Sapporo-shi, Hokkaido 0600008 (JP)
(74) Representative: UEXKÜLL & STOLBERG
(86) International application number: PCT/JP2005/005940
(87) International publication number: WO 2005/095941

(57) **Abstract**

It is an object of the present invention to provide a novel mass spectrometry method which overcomes the conventional problems mentioned above, which can analyze at a high sensitivity and high accuracy a chemical reaction on a surface of a self-organized membrane bound to a metal, and which can be applied to analysis of structures of a sugar chain in future. According to the present invention, a method for performing mass spectrometry of sulfur atom-containing derivatives of an organic residue, **characterized in that** the method includes ionizing a metal-organic residue complex into the derivatives, wherein the complex has the organic residue bound through a sulfur atom to the metal is provided, thereby solving the above problems.

## Description

### TECHNICAL FIELD

The present invention relates to a method for performing mass spectrometry of sulfur atom-containing derivatives of an organic residue using a metal-organic residue complex wherein the complex has the organic residue bound through a sulfur atom to the metal. The present invention also relates to a metal-organic residue complex used for such a mass spectrometry method and to a method for producing the metal-organic residue complex. The present invention also relates to a method for trapping a sugar chain or a sugar chain-containing substance, using a metal-organic residue complex of the present invention and to a method for measuring the molecular weight of a substance which may interact with an organic residue of a metal-organic residue complex. The present invention further relates to a composition for trapping a sugar chain or a kit for mass spectrometry of a sugar chain or a sugar chain-containing substance.

### BACKGROUND ART

A sugar chain usually exists as a complex with a protein, lipid or the like. A sugar chain structure is extremely complex due to diversity of the monosaccharides comprising the sugar chain, branching, linkage, anomericity, and the like. In addition to the diversity of the sugar chain structures, biosynthesis of the sugar chains by various enzymes can be accompanied by the difficulty in the identification of an expression pattern of the sugar chain using expression analysis of a gene or protein. Further, formation of multiple sugar chains, which have been constructed for a single protein, can frequently lead to heterogeneity of the sugar chain structure when bound to the same protein or lipid, resulting in the inevitable necessity for high-sensitive analysis, than proteins.

A self-organized film which has a sugar chain or allows trapping a sugar chain, will hold great promise for a novel system for analyzing a sugar chain structure. It is generally known that, when a metal substrate (for example, Au, Ag, Cu or the like) is immersed into a solution of a thiol derivative, the thiol derivatives adsorb a surface of the metal, and interactions between the molecules (for example, Van der Waals forces) cause the molecules to be regularly arranged in two-dimensional directions at the level of nanometers. Since the molecules will spontaneously assemble to form an arranged system, the formed film is called "self-assembled monomolecular film" or "self-assembled film". Many researchers have performed studies for fixing functional molecules or biomolecules (such as a sugar chain or a protein) on the surface of a metal or for modifying the surface of the metal, by utilizing the available organic film formation on the metal surface.

An approach has been reported so far wherein oligosaccharide chains are fixed on a gold substrate to monitor them using surface plasmon resonance technique (see, for example, patent document 1). However, according to this approach, the fixation of the sugar chain is indirectly monitored only based on the change in the index of refraction of a solution in the vicinity of the gold-liquid interface. This means that accurate information of the sugar chains can not be obtained from the above observation.

Recently, mass spectrometry has been regarded as an important approach for analyzing the structure of a surface, particularly, in the field of biochemistry and molecular biology. This approach eliminates cumbersome procedures before measurement, such as directly linking an analyte to a fluorochrome or radioactive labels for subsequent analysis. Further, the approach has the advantage of allowing for a rapid acquisition of accurate information for the analyte. So far, Mrksich et al. reported that thiol compounds, which have been likened to a gold substrate, experiences cleavage caused by a MALDI-TOF Mass laser beam, and the cleaved compounds are detected (see, for example, Non-Patent Document 1). In this situation, since a monomolecular film composed of thiol compounds as arranged in a two-dimensional monomolecular layer on the surface of the gold substrate is cleaved by laser beam irradiation, the absolute amount of the detected thiol compounds is extremely low, and is susceptible to contaminants (for example, a buffer, a salt or the like) during detection, possibly resulting in decreased sensitivity of detection.

There has not yet been a technique which enables the high-sensitivity and accurate analysis of a sugar chain. Such a technique is very important in glycomics, including next-generation proteomics, such as, post translational modification analysis. Thus developments of such a technique are ardently desired.
Patent Document 1: Japanese Laid-Open Patent Publication No. 2003-83969
Patent Document 2: Japanese Laid-Open Patent Publication No. 2003-254959
Patent Document 3: WO 03/019178
Patent Document 4: WO 03/091724
Non-Patent Document 1: Angew. Chem. Int. Ed. 41, 4715-4718, 2002
Non-Patent Document 2: J. Comb. Chem. 2002, 4, 120-124

### DISCLOSURE OF THE INVENTION

### PROBLEMS TO BE SOLVED BY THE INVENTION

It is an object of the present invention to provide a novel mass spectrometry method which overcomes the conventional problems mentioned above, which can analyze at a high sensitivity and high accuracy a chemical reaction on a surface of a self-organized membrane bound to a metal, and which can be applied to the analysis of structure of a sugar chain in future.

The present inventors have found, as a result of wholehearted studies, that enhancement of sensitivity can be achieved by providing a surface of a metal which enables a diffuse reflection of a laser beam (that is, the reflected laser provides a number of additional irradiation to the surface of the metal). Further, we confirmed that not only laser irradiation by the MALDI-TOF MS method, but also laser irradiation by the LDI-TOF MS method which does not require a low-molecular matrix, could cleave and ionize linkage parts of thiol compounds which had been introduced to the surface of such a metal, and that the parts could be observed at a high sensitivity. Thus the above problems have been solved.

Accordingly, the present invention provides the following.
(Item 1) A method for performing mass spectrometry of sulfur atom-containing derivatives of an organic residue, characterized in that the method includes ionizing a metal-organic residue complex into the derivatives, wherein the complex has the organic residue bound through a sulfur atom to the metal.
(Item 2) A method for performing mass spectrometry of a compound or salt thereof, characterized in that the method includes ionizingametal-organic residue complex into sulfur atom-containing derivatives,
   wherein the metal-organic residue complex is represented by the general formula (I)

   (R-S)ₙ-M¹ (I),

   wherein R is an organic residue, S is a sulfur atom and n indicates a stoichiometric ratio of (R-S) group with respect to M¹ and is an integer equal to or greater than 1; and
   wherein the compound is represented by the general formulae (II) and/or (III):

   R-SH (II)

   and/or

   R-S-S-R (III),

   wherein R and S are the same as defined above.
(Item 3) A method for performing mass spectrometry of a compound or salt thereof, characterized in that the method includes ionizing a metal-organic residue complex into sulfur atom-containing derivatives,
   wherein the metal-organic residue complex is represented by the general formula (IV):

   M¹-S-X-CH(R)-S-M¹ (IV),

   wherein R is an organic residue, S is a sulfur atom, M¹ at both ends are same metal entities, X is a lower alkylene or a lower alkenylene;
   wherein the compound is represented by the general formulae (V) and/or (VI):

   HS-X-CH(R)-SH (V)

   and/or 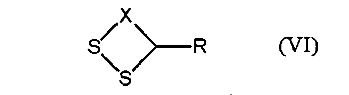 wherein R, S and X are the same as defined above.
(Item 4) A method for performing mass spectrometry of a sugar chain or a sugar chain-containing substance, comprising the following steps of:
   1) contacting a metal-organic residue complex with a sugar chain or a sugar chain-containing substance under the conditions where the metal-organic residue complex and the sugar chain or sugar chain-containing substance may react with each other, wherein the metal-organic residue complex contains a metal bound to a group represented by the following formula :
      -S-Y- (OCH₂CH₂)ₙ-NH-C(=O) -CH₂-O-NH₂,
      -S-Y- (OCH₂CH₂)ₙ-NH-C(=O) -CH₂-O-NH(CH₃),
      -S-Y- (OCH₂CH₂)ₙ-NH-C(=O) -CH(NH₂) -W⁴-SH,
      -S-Y- (OCH₂CH₂)ₙ-NH-C(=S) -CH(NH₂) -W⁴-SH,
      -S-W¹-O-NH₂,
      -S-W¹-O-NH(CH₃),
      -S-W¹-O-W²-O-NH₂,
      -S-W¹-O-W²-O-NH(CH₃),
      -S- (CH₂CH₂O)ₙ-W¹-O-W²-O-NH₂,
      -S- (CH₂CH₂O)ₙ-W¹-O-W²-O-NH(CH₃),
      -S-W¹-C(=O)-NH-NH₂,
      -S-W¹-C(=S)-NH-NH₂,
      -S-W¹-NH-C(=O)-CH (NH₂)-W⁴-SH,
      -S-W¹-NH-C(=S) -CH (NH₂)-W⁴-SH
      -S-Z¹-Z²-Z³-Z⁴-Z⁵-O-NH₂,
      -S-Z¹-Z²-Z³-Z⁴-Z⁵-O-NH(CH₃),
      -S-Z¹-Z²-Z³-Z⁴-CH(NH₂)-O-Z⁶-SH,
      -S-Z¹-Z²-Z³-Z⁴-CH(NH₂)-Z⁶-SH,
      -S-Z¹-O-Z³-CH(NH₂)-Z⁶-SH,
      -S-Z¹-O-Z³-O-NH₂,
      -S-Z¹-O-Z³-O-NH(CH₃),
      -S-Z¹-O-Z³-Z⁴-Z⁵-O-NH₂,
      -S-Z¹-O-Z³-Z⁴-Z⁵-O-NH(CH₃),
      -S-Z¹-O-Z³-Z⁴-CH(NH₂)-O-Z⁶-SH
      -S-Z¹-O-Z³-Z⁴-CH (NH₂) -Z⁶-SH
      -S-Z¹-Z³-Z⁴-Z⁵-O-NH₂,
      -S-Z¹-Z³-Z⁴-Z⁵-O-NH(CH₃),
      -S-Z¹-Z³-Z⁴-CH(NH₂)-O-Z⁶-SH,
      -S-Z¹-Z³-Z⁴-CH (NH₂)-Z⁶-SH,
      or 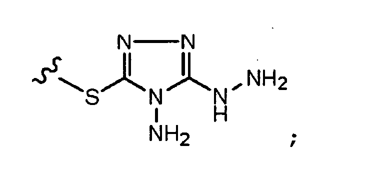
   2) obtaining the metal-organic residue complex bound to the sugar chain or the sugar chain-containing substance; and
   3) ionizing the metal-organic residue complex bound to the sugar chain or the sugar chain-containing substance into sulfur atom-containing derivatives of the organic residue.
(Item 5) A method according to any one of items 1 to 4, wherein the metal has a surface enough to cause a diffuse reflection of a laser beam.
(Item 6) Amethod according to item 5, wherein the metal is a fine metal particle.
(Item 7) A method according to any one of items 1 to 6, wherein the metal is a simple substance of gold, silver, cadmium or selenium.
(Item 8) A method according to any one of items 1 to 6, wherein the mass spectrometry is carried out by MALDI-TOF MS method.
(Item 9) A method according to item 8, wherein the mass spectrometry is carried out in the presence of a matrix reagent.
(Item 10) A method according to item 9, wherein the matrix reagent is 2,5-dihydroxybenzoic acid.
(Item 11) A method according to any one of items 1 to 3, wherein the organic residue is a group including a sugar chain or a sugar chain-containing substance.
(Item 12) A method for performing mass spectrometry of a sulfur atom-containing analyte, characterized in that the method includes ionizing a metal-analyte complex wherein the metal is bound through a sulfur atom to an analyte into a sulfur atom-containing analyte.
(Item 13) A method for performing mass spectrometry of a sulfur atom-containing analyte including the steps of:
   1) reacting tetrachloroauric acid with a sulfur atom-containing analyte in the presence of a reducing agent;
   2) obtaining a gold-analyte complex particle which has the analyte bound through the sulfur atom to the gold; and
   3) ionizing the obtained gold-analyte complex particles into a sulfur atom-containing analyte derivative.
(Item 14) A method according to item 13, wherein the reducing agent is sodium borohydride or sodium citrate.
(Item 15) A method according to item 13, wherein the step 1) is carried out in a solvent selected from the group consisting of water, alcoholic solvent, ethyl acetate, ethereal solvent or a mixture solvent thereof.
(Item 16) A method according to item 13, wherein the step 1) is carried out at a temperature between -20°C and 200°C, inclusive.
(Item 17) A method according to item 13, wherein the step 1) is carried out for 0.1 hour to 24 hours.
(Item 18) A method according to item 13, wherein a metal-analyte complex particle wherein an analyte is bound through a sulfur atom to a metal is purified by centrifugal filtration.
(Item 19) A method according to item 13, wherein the mass spectrometry is carried out by MALDI-TOF MS method.
(Item 20) A method according to item 13, wherein the step 3) is carried out in the presence of a matrix reagent.
(Item 21) A method for confirming development of chemical reaction or enzymatic reaction, characterized in that the method includes providing the metal-analyte complex particle obtained in the step 2) described in item 13 to a chemical reaction or enzymatic reaction, and ionizing the metal-analyte complex particle in a reaction system into a sulfur atom-containing analyte.
(Item 22) A method according to item 21, wherein the enzyme reaction is a sugar chain elongation reaction.
(Item 23) A method according to item 21, wherein an activity of a glycosyltransferase is confirmed utilizing development of the sugar chain elongation reaction.
(Item 24) A metal-organic residue complex containing a metal bound to a group represented by the following formula:
   -S-Y-(OCH₂CH₂)ₙ-NH-C(=O)-CH₂-O-NH₂,
   -S-Y- (OCH₂CH₂)ₙ-NH-C(=O)-CH₂-O-NH(CH₃),
   -S-Y-(OCH₂CH₂)ₙ-NH-C(=O)-CH(NH₂)-W⁴-SH,
   -S-Y-(OCH₂CH₂)ₙ-NH-C (=S) -CH (NH₂) -W⁴-SH,
   -S-W¹-O-NH₂,
   -S-W¹-O-NH(CH₃),
   -S-W¹-O-W²-O-NH₂,
   -S-W¹-O-W²-O-NH(CH₃),
   -S-(CH₂CH₂O)ₙ-W¹-O-W²-O-NH₂,
   -S-(CH₂CH₂O)ₙ-W¹-O-W²-O-NH(CH₃),
   -S-W¹-C(=O)-NH-NH₂,
   -S-W¹-C(=S)-NH-NH₂,
   -S-W¹-NH-C(=O)-CH(NH₂)-W⁴-SH,
   -S-W¹-NH-C(=S)-CH(NH₂)-W⁴-SH,
   -S-Z¹-Z²-Z³-Z⁴-Z⁵-O-NH₂,
   -S-Z¹-Z²-Z³-Z⁴-Z⁵-O-NH(CH₃),
   -S-Z¹-Z²-Z³-Z⁴-CH(NH₂)-O-Z⁶-SH,
   -S-Z¹-Z²-Z³-Z⁴-CH(NH₂)-Z⁶-SH,
   -S-Z¹-O-Z³-CH(NH₂)-Z⁶-SH,
   -S-Z¹-O-Z³-O-NH₂,
   -S-Z¹-O-Z³-O-NH(CH₃),
   -S-Z¹-O-Z³-Z⁴-Z⁵-O-NH₂,
   -S-Z¹-O-Z³-Z⁴-Z⁵-O-NH(CH₃),
   -S-Z¹-O-Z³-Z⁴-CH(NH₂)-O-Z⁶-SH
   -S-Z¹-O-Z³-Z⁴-CH(NH₂)-Z⁶-SH
   -S-Z¹-Z³-Z⁴-Z⁵-O-NH₂,
   -S-Z¹-Z³-Z⁴-Z⁵-O-NH(CH₃),
   -S-Z¹-Z³-Z⁴-CH(NH₂)-O-Z⁶-SH,
   -S-Z¹-Z³-Z⁴-CH(NH₂)-Z⁶-SH,
   or 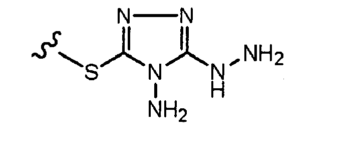 wherein, Y, W¹ and W² are independently C1-C12 alkylene, C2-C12 alkenylene or C2-C12 alkynylene;
   W⁴ is C1-C2 alkylene;
   Z¹ is substituted or unsubstituted arylen or heteroarylen;
   Z² is a nitrogen-containing heterocycle;
   Z³ and Z⁵ are independently C1-C12 alkylene;
   Z⁴ is -O-C (=O), -O-C(=S), -NH-C (=O), -NH-C (=S) , -O- or -S-;
   Z⁶ is C1-C2 alkylene; and
   n is an integer between 1 and 10, inclusive.
(Item 25) A complex according to item 24, wherein the metal has a surface enough to cause a diffuse reflection of a laser beam.
(Item 26) A complex according to item 25, wherein the metal is a fine metal particle.
(Item 27) A method for producing metal-organic residue complex particles, wherein the method includes reacting tetrachloroauric acid with a compound represented by the following formula:
   -(S-Y-(OCH₂CH₂)ₙ-NH-C(=O)-CH₂-O-NH₂)₂,
   -(S-Y-(OCH₂CH₂)ₙ-NH-C(=O)-CH₂-O-NH(CH₃))₂,
   -(S-Y-(OCH₂CH₂)ₙ-NH-C(=O)-CH(NH₂)-W⁴-SH)₂,
   -(S-Y-(OCH₂CH₂)ₙ-NH-C(=S)-CH(NH₂)-W⁴-SH)₂,
   -(S-W¹-O-NH₂)₂,
   -(S-W¹-O-NH(CH₃))₂,
   -(S-W¹-O-W²-O-NH₂)₂,
   -(S-W¹-O-W²-O-NH(CH₃))₂,
   -(S-(CH₂CH₂O)ₙ-W¹-O-W²-O-NH₂)₂,
   -(S-(CH₂CH₂O)ₙ-W¹-O-W²-O-NH(CH₃))₂,
   -(S-W¹-C(=O)-NH-NH₂)₂,
   -(S-W¹-C(=S)-NH-NH₂)₂,
   -(S-W¹-NH-C(=O)-CH(NH₂)-W⁴-SH)₂,
   -(S-W¹-NH-C(=S)-CH(NH₂)-W⁴-SH)₂,
   -(S-Z¹-Z²-Z³-Z⁴-Z⁵-O-NH₂)₂,
   -(S-Z¹-Z²-Z³-Z⁴-Z⁵-O-NH(CH₃))₂,
   -(S-Z¹-Z²-Z³-Z⁴-CH(NH₂)-O-Z⁶-SH)₂,
   -(S-Z¹-Z²-Z³-Z⁴-CH(NH₂)-Z⁶-SH)₂,
   -(S-Z¹-O-Z³-CH(NH₂)-Z⁶-SH)₂,
   -(S-Z¹-O-Z³-O-NH₂)₂,
   -(S-Z¹-O-Z³-O-NH(CH₃))₂,
   -(S-Z¹-O-Z³-Z⁴-Z⁵-O-NH₂)₂,
   -(S-Z¹-O-Z³-Z⁴-Z⁵-O-NH(CH₃))₂,
   -(S-Z¹-O-Z³-Z⁴-CH(NH₂)-O-Z⁶-SH)₂,
   -(S-Z¹-O-Z³-Z⁴-CH(NH₂)-Z⁶-SH)₂,
   -(S-Z¹-Z³-Z⁴-Z⁵-O-NH₂)₂,
   -(S-Z¹-Z³-Z⁴-Z⁵-O-NH(CH₃))₂,
   -(S-Z¹-Z³-Z⁴-CH(NH₂)-O-Z⁶-SH)₂,
   -(S-Z¹-Z³-Z⁴-CH(NH₂)-Z⁶-SH)₂
   or 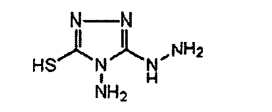 ,or a salt thereof, in the presence of a reducing agent,
   wherein, Y, W¹ and W² are independently C1-C12 alkylene, C2-C12 alkenylene or C2-C12 alkynylene;
   W⁴ is C1-C2 alkylene;
   Z¹ is substituted or unsubstituted arylen or heteroarylen;
   Z² is a nitrogen-containing heterocycle;
   Z³ and Z⁵ are independently C1-C12 alkylene;
   Z⁴ is -O-C (=O), -O-C(=S), -NH-C (=O), -NH-C (=S) , -O- or -S-;
   Z⁶ is C1-C2 alkylene; and
   n is an integer between 1 and 10, inclusive.
(Item 28) A producing method according to item 27, wherein the reducing agent is sodium borohydride or sodium citrate.
(Item 29) A producing method according to item 28, carried out in water, alcoholic solvent, ethyl solvent ethereal solvent, or a mixture solvent thereof.
(Item 30) A producing method according to item 28, carried out at a temperature between -20°C to 200°C, inclusive.
(Item 31) A method according to item 28, carried out for 0.1 hour to 24 hours.
(Item 32) A producing method according to item 28, wherein a gold-analyte complex particle wherein an analyte is bound through a sulfur atom to a metal is purified by centrifugal filtration.
(Item 33) A method for trapping a sugar chain or a sugar chain-containing substance, characterized in that the method includes contacting a metal-organic residue complex with a sugar chain or a sugar chain-containing substance, under conditions where the metal-organic residue complex and the sugar chain or the sugar chain-containing substance may react with each other,
   the metal-organic residue complex has a metal bound to a group represented by the following formula:
   -S-Y- (OCH₂CH₂)ₙ-NH-C (=O) -CH₂-O-NH₂,
   -S-Y- (OCH₂CH₂)ₙ-NH-C (=O) -CH₂-O-NH (CH₃),
   -S-Y- (OCH ₂CH₂)ₙ-NH-C (=O) -CH (NH₂) -W⁴-SH,
   -S-Y- (OCH₂CH₂)ₙ-NH-C (=S) -CH (NH₂) -W⁴-SH,
   -S-W¹-O-NH₂,
   -S-W¹-O-NH(CH₃),
   -S-W¹-O-W²-O-NH₂,
   -S-W¹-O-W²-O-NH(CH₃),
   -S- (CH₂CH₂O)ₙ-W¹-O-W²-O-NH₂,
   -S- (CH₂CH₂O)ₙ-W¹-O-W²-O-NH(CH₃),
   -S-W¹-C(=O)-NH-NH₂,
   -S-W¹-C(=S)-NH-NH₂,
   -S-W¹-NH-C(=O)-CH (NH₂)-W⁴-SH,
   -S-W¹-NH-C(=S) -CH (NH₂) -W⁴-SH,
   -S-Z¹-Z²-Z³-Z⁴-Z⁵-O-NH₂,
   -S-Z¹-Z²-Z³-Z⁴-Z⁵-O-NH(CH₃),
   -S-Z¹-Z²-Z³-Z⁴-CH(NH₂)-O-Z⁶-SH,
   -S-Z¹-Z²-Z³-Z⁴-CH(NH₂)-Z⁶-SH,
   -S-Z¹-O-Z³-CH(NH₂)-Z⁶-SH,
   -S-Z¹-O-Z³-O-NH₂,
   -S-Z¹-O-Z³-O-NH(CH₃),
   -S-Z¹-O-Z³-Z⁴-Z⁵-O-NH₂,
   -S-Z¹-O-Z³-Z⁴-Z⁵-O-NH(CH₃),
   -S-Z¹-O-Z³-Z⁴-CH(NH₂)-O-Z⁶-SH
   -S-Z¹-O-Z³-Z⁴-CH(NH₂)-Z⁶-SH
   -S-Z¹-Z³-Z⁴-Z⁵-O-NH₂,
   -S-Z¹-Z³-Z⁴-Z⁵-O-NH(CH₃),
   -S-Z¹-Z³-Z⁴-CH(NH₂)-O-Z⁶-SH,
   -S-Z¹-Z³-Z⁴-CH(NH₂)-Z⁶-SH,
   or 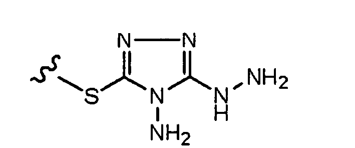 wherein, Y, W¹ and W² are independently C1-C12 alkylene, C2-C12 alkenylene or C2-C12 alkynylene;
   W⁴ is C1-C2 alkylene;
   Z¹ is substituted or unsubstituted arylen or heteroarylen;
   Z² is a nitrogen-containing heterocycle;
   Z³ and Z⁵ are independently C1-C12 alkylene;
   Z⁴ is -O-C (=O), -O-C (=S) , -NH-C (=O), -NH-C (=S) , -O- or -S-;
   Z⁶ is C1-C2 alkylene; and
   n is an integer between 1 and 10, inclusive.
(Item 34) A method for measuring the molecular weight of a substance which may interact with an organic residue of a metal-organic residue complex, including the steps of:
   1) contacting the metal-organic residue complex with a substance which may interact with the organic residue, wherein the metal is bound through a sulfur atom to organic residue;
   2) obtaining the metal-organic residue complex bound to the substance which may interact; and
   3) ionizing the obtained metal-organic residue complex into derivatives of the organic residue, wherein the organic residue contains a sulfur atom.
(Item 35) A method according to item 34, wherein the metal has a surface enough to cause a diffuse reflection of a laser beam.
(Item 36) A method according to item 35, wherein the metal is a fine metal particle.
(Item 37) A method for performing mass spectrometry of a sugar chain or a sugar chain-containing substance, including the steps of:
   1) contacting a compound with a metal, wherein the compound is represented by the following formula: 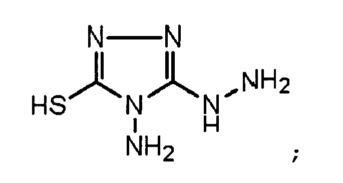
   2) contacting the metal-organic residue complex obtained in 1) with a sugar chain or a sugar chain-containing substance under conditions where the metal-organic residue complex and the sugar chain or the sugar chain-containing substance may react with each other; and
   3) ionizing the metal-organic residue complex obtained in 2) into derivatives of the organic residue, wherein the organic residue contains a sulfur atom.
(Item 38) A method for performing mass spectrometry of a sugar chain or a sugar chain-containing substance, including the steps of:
   1) contacting a compound represented by the following formula: 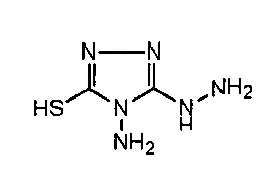 with a sugar chain or a sugar chain-containing substance under conditions where the compound and the sugar chain or the sugar chain-containing substance may react with each other;
   2) contacting the compound obtained in 1) with a metal; and
   3) ionizing the metal-organic residue complex obtained in 2) into derivatives of the organic residue, wherein the organic residue contains a sulfur atom.
(Item 39) A method according to item 37 or 38, wherein the metal has a surface enough to cause a diffuse reflection of a laser beam.
(Item 40) A method according to item 39, wherein the metal is a fine metal particle.
(Item 41) A composition for trapping a sugar chain, including:
   a compound represented by the general formula (II): R-SH (II) or a salt thereof, wherein R is an organic residue; and S is a sulfur atom;
   a compound represented by the general formula (III): R-S-S-R (III) or a salt thereof, wherein, R and S are the same as defined above;
   a compound represented by the general formula (V): HS-X-CH(R)-SH (V) or a salt thereof, wherein R and S are the same as defined above; and X is lower alkylene or lower alkenylene; or
   a compound represented by the general formula (VI): 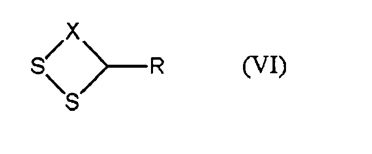
   or a salt thereof , wherein, R, S and X are the same as defined above; or a mixture thereof.
(Item 42) A composition for trapping a sugar chain, including a compound represented by the following formula:
   -(S-Y-(OCH₂CH₂)ₙ-NH-C(=O)-CH₂-O-NH₂)₂,
   -(S-Y-(OCH₂CH₂)ₙ-NH-C(=O)-CH₂-O-NH(CH₃))₂,
   -(S-Y-(OCH₂CH₂)ₙ-NH-C(=O)-CH(NH₂) -W⁴-SH)₂,
   -(S-Y-(OCH₂CH₂)ₙ-NH-C(=S)-CH(NH₂)-W⁴-SH)₂,
   -(S-W¹-O-NH₂)₂,
   -(S-W¹-O-NH(CH₃))₂,
   -(S-W¹-O-W²-O-NH₂)₂,
   -(S-W¹-O-W²-O-NH(CH₃))₂,
   -(S-(CH₂CH₂O)ₙ-W¹-O-W²-O-NH₂)₂,
   -(S-(CH₂CH₂O)ₙ-W¹-O-W²-O-NH(CH₃))₂.
   -(S-W¹-C(=O)-NH-NH₂)₂,
   -(S-W¹-C(=S)-NH-NH₂)₂,
   -(S-W¹-NH-C(=O)-CH(NH₂)-W⁴-SH)₂,
   -(S-W¹-NH-C(=S)-CH(NH₂)-W⁴-SH)₂,
   -(S-Z¹-Z²-Z³-Z⁴-Z⁵-O-NH₂)₂,
   -(S-Z¹-Z²-Z³-Z⁴-Z⁵-O-NH(CH₃))₂,
   -(S-Z¹-Z²-Z³-Z⁴-CH(NH₂)-O-Z⁶-SH)₂,
   -(S-Z¹-Z²-Z³-Z⁴-CH(NH₂)-Z⁶-SH)₂,
   -(-S-Z¹-O-Z³-CH(NH₂)-Z⁶-SH)₂,
   -(S-Z¹-O-Z³-O-NH₂)₂,
   -(S-Z¹-O-Z³-O-NH(CH₃))₂,
   -(S-Z¹-O-Z³-Z⁴-Z⁵-O-NH₂)₂,
   -(S-Z¹-O-Z³-Z⁴-Z⁵-O-NH(CH₃))₂,
   -(S-Z¹-O-Z³-Z⁴-CH(NH₂)-O-Z⁶-SH)₂,
   -(S-Z¹-O-Z³-Z⁴-CH(NH₂)-Z⁶-SH)₂,
   -(S-Z¹-Z³-Z⁴-Z⁵-O-NH₂)₂.
   -(S-Z¹-Z³-Z⁴-Z⁵-O-NH(CH₃))₂,
   -(S-Z¹-Z³-Z⁴-CH(NH₂)-O-Z⁶-SH)₂,
   -(S-Z¹-Z³-Z⁴-CH(NH₂)-Z⁶-SH)₂
   or 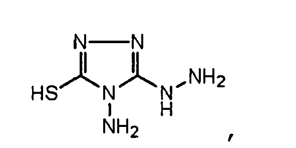 wherein Y, W¹ and W² are independently C1-C12 alkylene, C2-C12 alkenylene or C2-C12 alkynylene;
   W⁴ is C1-C2 alkylene;
   Z¹ is substituted or unsubstituted arylen or heteroarylen;
   Z² is a nitrogen-containing heterocycle;
   Z³ and Z⁵ are independently C1-C12 alkylene;
   Z⁴ is -O-C (=O) , -O-C (=S) , -NH-C (=O), -NH-C (=S) , -O- or -S-;
   Z⁶ is C1-C2 alkylene; and
   n is an integer between 1 and 10, inclusive.
(Item 43) A metal-organic residue complex represented by the following formula:
   M²-(S-Y-(OCH₂CH₂)ₙ-NH-C(=O)-CH₂-O-NH₂)ₘ,
   M²-(S-Y-(OCH₂CH₂)ₙ-NH-C(=O)-CH₂-O-NH(CH₃))ₘ,
   M²-(S-Y-(OCH₂CH₂)ₙ-NH-C(=O)-CH (NH₂)-W⁴-SH)ₘ,
   M²-(S-Y-(OCH₂CH₂)ₙ-NH-C(=S)-CH (NH₂)-W⁴-SH)ₘ,
   M²-(S-W¹-O-NH₂)ₘ,
   M²-(S-W¹-O-NH(CH₃))m,
   M²-(S-W¹-O-W²-O-NH₂)ₘ,
   M²-(S-W¹-O-W²-O-NH(CH₃))ₘ,
   M²- (S- (CH₂CH₂O)ₙ-W¹-O-W²-O-NH₂)ₘ,
   M²-(S- (CH₂CH₂O)ₙ-W¹-O-W²-O-NH(CH₃))ₘ,
   M²-(S-W¹-C(=O)-NH-NH₂)ₘ,
   M²-(S-W¹-C(=S)-NH-NH₂)ₘ,
   M²-(S-W¹-NH-C(=O)-CH (NH₂)-W⁴-SH)ₘ,
   M²-(S-W¹-NH-C(=S)-CH (NH₂)-W⁴-SH)ₘ,
   M²-(S-Z¹-Z²-Z³-Z⁴-Z⁵-O-NH₂)ₘ,
   M²-(S-Z¹-Z²-Z³-Z⁴-Z⁵-O-NH(CH₃))ₘ,
   M²-(S-Z¹-Z²-Z³-Z⁴-CH(NH₂)-O-Z⁶-SH)ₘ,
   M²-(S-Z¹-Z²-Z³-Z⁴-CH(NH₂)-Z⁶-SH)ₘ,
   M²- (S-Z¹-O-Z³-CH (NH₂) -Z⁶-SH)ₘ,
   M²- (S-Z¹-O-Z³-O-NH₂)ₘ,
   M²- (S-Z¹-O-Z³-O-NH (CH₃))ₘ,
   M²- (S-Z¹-O-Z³-Z⁴-Z⁵-O-NH₂)ₘ,
   M²- (S-Z¹-O-Z³-Z⁴-Z⁵-O-NH (CH₃))ₘ,
   M²- (S-Z¹-O-Z³-Z⁴-CH (NH₂) -O-Z⁶-SH)ₘ,
   M²-(S-Z¹-O-Z³-Z⁴-CH (NH₂) -Z⁶-SH)ₘ,
   M²- (S-Z¹-Z³-Z⁴-Z⁵-O-NH₂)ₘ,
   M²- (S-Z¹-Z³-Z⁴-Z⁵-O-NH (CH₃))ₘ,
   M²- (S-Z¹-Z³-Z⁴-CH(NH₂)-O-Z⁶-SH)ₘ,
   M²-(S-Z¹-Z³-Z⁴-CH(NH₂)-Z⁶-SH)ₘ,
   or the general formula (VII): 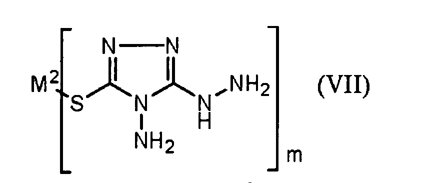
   wherein, M² is a metal;
   m indicates a stoichiometric ratio of an organic residue with respect to M² and is an integer equal to or greater than 1, wherein the organic residue contains a sulfur atom;
   Y, W¹ and W² are independently C1-C12 alkylene, C2-C12 alkenylene or C2-C12 alkynylene;
   W⁴ is C1-C2 alkylene;
   Z¹ is substituted or unsubstituted arylen or heteroarylen ;
   Z² is a nitrogen-containing heterocycle;
   Z³ and Z⁵ are independently C1-C12 alkylene;
   Z⁴ is -O-C (=O), -O-C (=S) , -NH-C (=O), -NH-C (=S) , -O- or -S-;
   Z⁶ is C1-C2 alkylene; and
   n is an integer between 1 and 10, inclusive.
(Item 44) A complex according to item 43, wherein the metal has a surface enough to cause a diffuse reflection.
(Item 45) A complex according to item 44, wherein the metal is a fine metal particle.
(Item 46) A composition for trapping a sugar chain, including: a metal-organic residue complex represented by the general formula (I):

   (R-S)ₙ-M¹ (I),

   wherein R is an organic residue; S is a sulfur atom; M¹ is a metal; and n indicates a stoichiometric ratio of (R-S) group with respect to M¹ and is an integer equal to or greater than 1; or
   a metal-organic residue complex represented by the general formula (IV):

   M¹-S-X-CH(R)-S-M¹ (IV),

   wherein R and S are the same as defined above, M¹ at both ends are a metal of the same substance and X is lower alkylene or lower alkenylene.
(Item 47) A composition for trapping a sugar chain, comprising a metal-organic residue complex, represented by the following formula:
   M²-(S-Y-(OCH₂CH₂)ₙ-NH-C(=O)-CH₂-O-NH₂)ₘ,
   M²-(S-Y-(OCH₂CH₂)ₙ-NH-C(=O)-CH₂-O-NH(CH₃))ₘ,
   M²-(S-Y-(OCH₂CH₂)ₙ-NH-C(=O)-CH(NH₂)-W⁴-SH)ₘ,
   M²-(S-Y-(OCH₂CH₂)ₙ-NH-C(=S)-CH(NH₂)-W⁴-SH)ₘ,
   M²-(S-W¹-O-NH₂)ₘ,
   M²-(S-W¹-O-NH(CH₃))ₘ,
   M²-(S-W¹-O-W²-O-NH₂)ₘ,
   M²-(S-W¹-O-W²-O-NH(CH₃))ₘ,
   M²-(S-(CH₂CH₂O)ₙ-W¹-O-W²-O-NH₂)ₘ,
   M²-(S-(CH₂CH₂O)ₙ-W¹-O-W²-O-NH(CH₃))ₘ,
   M²-(S-W¹-C(=O)-NH-NH₂)ₘ,
   M²-(S-W¹-C(=S)-NH-NH₂)ₘ,
   M²-(S-W¹-NH-C(=O)-CH (NH₂)-W⁴-SH)ₘ,
   M²-(S-W¹-NH-C(=S)-CH (NH₂)-W⁴-SH)ₘ,
   M²-(S-Z¹-Z²-Z³-Z⁴-Z⁵-O-NH₂)ₘ,
   M²-(S-Z¹-Z²-Z³-Z⁴-Z⁵-O-NH(CH₃))ₘ,
   M²-(S-Z¹-Z²-Z³-Z⁴-CH(NH₂)-O-Z⁶-SH)ₘ,
   M²-(S-Z¹-Z²-Z³-Z⁴-CH(NH₂)-Z⁶-SH)ₘ,
   M²-(S-Z¹-O-Z³-CH(NH₂)-Z⁶-SH)ₘ,
   M²-(S-Z¹-O-Z³-O-NH₂)ₘ,
   M²-(S-Z¹-O-Z³-O-NH(CH₃))ₘ,
   M²-(S-Z¹-O-Z³-Z⁴-Z⁵-O-NH₂)ₘ,
   M²-(S-Z¹-O-Z³-Z⁴-Z⁵-O-NH(CH₃))ₘ,
   M²-(S-Z¹-O-Z³-Z⁴-CH(NH₂)-O-Z⁶-SH)ₘ,
   M²-(S-Z¹-O-Z³-Z⁴-CH(NH₂)-Z⁶-SH)ₘ,
   M²-(S-Z¹-Z³-Z⁴-Z⁵-O-NH₂)ₘ,
   M²-(S-Z¹-Z³-Z⁴-Z⁵-O-NH(CH₃))ₘ,
   M²-(S-Z¹-Z³-Z⁴-CH(NH₂)-O-Z⁶-SH)ₘ,
   M²-(S-Z¹-Z³-Z⁴-CH(NH₂)-Z⁶-SH)ₘ,
   or the general formula (VII): 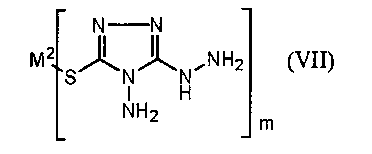
   wherein,
   M² is a metal;
   m indicates a stoichiometric ratio of an organic residue with respect to M² and is an integer equal to or greater than 1, wherein the organic residue comprises a sulfur atom;
   Y, W¹ and W² are independently C1-C12 alkylene, C2-C12 alkenylene or C2-C12 alkynylene;
   W⁴ is C1-C2 alkylene;
   Z¹ is substituted or unsubstituted arylen or heteroarylen;
   Z² is a nitrogen-containing heterocycle;
   Z³ and Z⁵ are independently C1-C12 alkylene;
   Z⁴ is -O-C(=O), -O-C (=S) , -NH-C (=O), -NH-C (=S) , -O- or -S-;
   Z⁶ is C1-C2 alkylene and
   n is an integer between 1 and 10, inclusive.
(Item 48) A composition according to item 46 or 47, wherein the metal has a surface enough to cause a diffuse reflection.
(Item 49) A composition according to item 48, wherein the metal is a fine metal particle.
(Item 50) A kit for mass spectrometry of a sugar chain or a sugar chain-containing substance, including:
   A) a compound represented by the general formula (II):

      R-SH (II)

      or a salt thereof, wherein R is an organic residue; and S is a sulfur atom;
      a compound represented by the general formula (III)

      R-S-S-R (III)

      or a salt thereof, wherein R and S are the same as defined above;
      a compound represented by the general formula (V):

      HS-X-CH (R) -SH (V)

      or a salt thereof, wherein R and S are the same as defined above; and X is lower alkylene or lower alkenylene; or
      a compound represented by the general formula (VI): 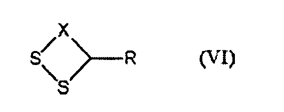 or a salt thereof, wherein R, S and X are the same as defined above; or a mixture thereof; and
   B) a metal.
(Item 51) A kit for mass spectrometry of a sugar chain or a sugar chain-containing substance, including:

A) a sulfur atom containing derivatives of an organic residue, represented by the following formula:
   -(S-Y-(OCH₂CH₂)ₙ-NH-C(=O)-CH₂-O-NH₂)₂,
   -(S-Y-(OCH₂CH₂)ₙ-NH-C(=O)-CH₂-O-NH(CH₃))₂,
   -(S-Y-(OCH₂CH₂)ₙ-NH-C(=O)-CH(NH₂)-W⁴-SH)₂,
   -(S-Y-(OCH₂CH₂)ₙ-NH-C(=S)-CH(NH₂)-W⁴-SH)₂,
   -(S-W¹-O-NH₂)₂,
   -(S-W¹-O-NH(CH₃))₂,
   -(S-W¹-O-W²-O-NH₂)₂,
   -(S-W¹-O-W²-O-NH(CH₃))₂,
   -(S-(CH₂CH₂O)ₙ-W¹-O-W²-O-NH₂)₂,
   -(S-(CH₂CH₂O)ₙ-W¹-O-W²-O-NH(CH₃))₂,
   -(S-W¹-C(=O)-NH-NH₂)₂,
   -(S-W¹-C(=S)-NH-NH₂)₂,
   -(S-W¹-NH-C(=O)-CH(NH₂)-W⁴-SH)₂,
   -(S-W¹-NH-C(=S)-CH (NH₂)-W⁴-SH)₂,
   -(S-Z¹-Z²-Z³-Z⁴-Z⁵-O-NH₂)₂,
   -(S-Z¹-Z²-Z³-Z⁴-Z⁵-O-NH(CH₃))₂,
   -(S-Z¹-Z²-Z³-Z⁴-CH(NH₂)-O-Z⁶-SH)₂,
   -(S-Z¹-Z²-Z³-Z⁴-CH(NH₂)-Z⁶-SH)₂,
   -(S-Z¹-O-Z³-CH(NH₂)-Z⁶-SH)₂.
   -(S-Z¹-O-Z³-O-NH₂)₂,
   -(S-Z¹-O-Z³-O-NH(CH₃))₂,
   -(S-Z¹-O-Z³-Z⁴-Z⁵-O-NH₂)₂,
   -(S-Z¹-O-Z³-Z⁴-Z⁵-O-NH(CH₃))₂,
   -(S-Z¹-O-Z³-Z⁴-CH(NH₂)-O-Z⁶-SH)₂,
   -(S-Z¹-O-Z³-Z⁴-CH(NH₂)-Z⁶-SH)₂,
   -(S-Z¹-Z³-Z⁴-Z⁵-O-NH₂)₂,
   -(S-Z¹-Z³-Z⁴-Z⁵-O-NH(CH₃))₂,
   -(S-Z¹-Z³-Z⁴-CH(NH₂)-O-Z⁶-SH)₂,
   -(S-Z¹-Z³-Z⁴-CH(NH₂)-Z⁶-SH)₂
   or 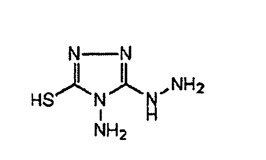 wherein Y, W¹ and W² are independently C1-C12 alkylene, C2-C12 alkenylene or C2-C12 alkynylene;
   W⁴ is C1-C2 alkylene;
   Z¹ is substituted or unsubstituted arylen or heteroarylen;
   Z² is a nitrogen-containing heterocycle;
   Z³ and Z⁵ are independently C1-C12 alkylene;
   Z⁴ is -O-C(=O), -O-C(=S), -NH-C(=O), -NH-C (=S) , -O- or -S-;
   Z⁶ is C1-C2 alkylene; and
   n is an integer between 1 and 10, inclusive; and
B) a metal.
(Item 52) A kit according to item 50 or 51, wherein the metal has a surface enough to cause a diffuse reflection.
(Item 53) A kit according to item 52, wherein the metal is a fine metal particle.
(Item 54) A kit according to item 50 or 51, further including:
   C) a reducing agent, wherein the metal is tetrachloroauric acid.
(Item 55) A kit according to item 42, wherein the reducing agent is sodium borohydride or sodium citrate.
(Item 56) A kit for mass spectrometry of a sugar chain or a sugar chain-containing substance, including:
   a metal-organic residue complex represented by the general formula (I):

      (R-S)ₙ-M¹ (I)

      wherein, R is an organic residue, S is a sulfur atom, M¹ is a metal and n indicates a stoichiometric ratio of (R-S) group with respect to M¹ and is an integer equal to or greater than 1; or
   a metal-organic residue complex represented by the general formula (IV):

      M¹-S-X-CH(R)-S-M¹ (IV)

      wherein R and S are the same as defined above, M¹ at both ends are same metal entities and X is lower alkylene or lower alkenylene.
(Item 57) A kit for mass spectrometry of a sugar chain or a sugar chain-containing substance, including a metal-organic residue complex, represented by the following formula:
   M²-(S-Y-(OCH₂CH₂)ₙ-NH-C(=O)-CH₂-O-NH₂)ₘ,
   M²-(S-Y-(OCH₂CH₂)ₙ-NH-C(=O)-CH₂-O-NH(CH₃))ₘ,
   M²-(S-Y-(OCH₂CH₂)ₙ-NH-C(=O)-CH(NH₂)-W⁴-SH)ₘ,
   M²-(S-Y-(OCH₂CH₂)ₙ-NH-C(=S)-CH(NH₂)-W⁴-SH)ₘ,
   M²-(S-W¹-O-NH²)ₘ,
   M²-(S-W¹-O-NH(CH₃))ₘ,
   M²-(S-W¹-O-W²-O-NH₂)ₘ,
   M²-(S-W¹-O-W²-O-NH(CH₃))ₘ,
   M²-(S-(CH₂CH₂O)ₙ-W¹-O-W²-O-NH₂)ₘ,
   M²-(S-(CH₂CH₂O)ₙ-W¹-O-W²-O-NH(CH₃))ₘ,
   M²-(S-W¹-C(=O)-NH-NH₂)ₘ,
   M²-(S-W¹-C(=S)-NH-NH₂)ₘ,
   M²-(S-W¹-NH-C(=O)-CH (NH₂)-W⁴-SH)ₘ,
   M²-(S-W¹-NH-C(=S)-CH (NH₂)-W⁴-SH)ₘ,
   M²-(S-Z¹-Z²-Z³-Z⁴-Z⁵-O-NH₂)ₘ,
   M²-(S-Z¹-Z²-Z³-Z⁴-Z⁵-O-NH(CH₃))ₘ,
   M²-(S-Z¹-Z²-Z³-Z⁴-CH(NH₂)-O-Z⁶-SH)ₘ,
   M²-(S-Z¹-Z²-Z³-Z⁴-CH (NH₂) -Z⁶-SH)ₘ,
   M²-(S-Z¹-O-Z³-CH(NH₂) -Z⁶-SH)ₘ,
   M²-(S-Z¹-O-Z³-O-NH₂)ₘ,
   M²-(S-Z¹-O-Z³-O-NH(CH₃))m,
   M²-(S-Z¹-O-Z³-Z⁴-Z⁵-O-NH₂)ₘ,
   M²- (S-Z¹-O-Z³-Z⁴-Z⁵-O-NH(CH₃))ₘ,
   M²-(S-Z¹-O-Z³-Z⁴-CH(NH₂) -O-Z⁶-SH)ₘ,
   M²-(S-Z¹-O-Z³-Z⁴-CH(NH₂) -Z⁶-SH)ₘ,
   M²-(S-Z¹-Z³-Z⁴-Z⁵-O-NH₂)ₘ,
   M²-(S-Z¹-Z³-Z⁴-Z⁵-O-NH(CH₃))ₘ,
   M²-(S-Z¹-Z³-Z⁴-CH(NH₂)-O-Z⁶-SH)ₘ,
   M²-(S-Z¹-Z³-Z⁴-CH(NH₂)-Z⁶-SH)ₘ,
   or the general formula (VII): 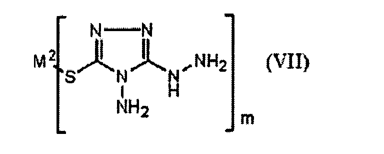
   wherein, M² is a metal, m indicates a stoichiometric ratio of an organic residue with respect to M² and is an integer equal to or greater than 1, the organic residue comprises a sulfur atom, Y, W¹ and W² are independently C1-C12 alkylene, C2-C12 alkenylene or C2-C12 alkynylene, W⁴ is C1-C2 alkylene;
   Z¹ is substituted or unsubstituted arylen or heteroarylen;
   Z² is a nitrogen-containing heterocycle, Z³ and Z⁵ are independently C1-C12 alkylene, Z' is -O-C(=O), -O-C(=S), -NH-C(=O), -NH-C (=S) , -O- or -S-, Z⁶ is C1-C2 alkylene; and n is an integer between 1 and 10, inclusive.
(Item 58) A kit according to item 56 or 57, wherein the metal has a surface enough to cause a diffuse reflection of a laser beam.
(Item 59) A kit according to item 58, wherein the metal is a fine metal particle.
(Item 60) A kit according to item 56 or 57, wherein the metal is a fine gold particle.
(Item 61) A method according to any one of items 1 to 23, wherein the metal is a complex metal containing a layer of gold, silver, cadmium or selenium coating a surface of iron, nickel, cobalt or iron oxide (Fe₃O₄).
(Item 62) A metal-organic residue complex according to any one of items 24 to 26 and 43 to 45, wherein the metal is a complex metal containing a layer of gold, silver, cadmium or selenium coating a surface of iron, nickel, cobalt or iron oxide (Fe₃O₄).
(Item 63) A producing method according to any one of items 27 to 32, wherein the metal is a complexmetal containing a layer of gold, silver, cadmium or selenium coating a surface of iron, nickel, cobalt or iron oxide (Fe₃O₄).
(Item 64) A producing according to any one of items 33 to 40, wherein the metal is a complex metal containing a layer of gold, silver, cadmium or selenium coating a surface of iron, nickel, cobalt or iron oxide (Fe₃O₄).
(Item 65) A composition for trapping a sugar chain according to any one of items 41, 42 and 46 to 49, wherein the metal is a complex metal containing a layer of gold, silver, cadmium or selenium coating a surface of iron, nickel, cobalt or iron oxide (Fe₃O₄).
(Item 66) A kit according to any one of items 50 to 60, wherein the metal is a complex metal containing a layer of gold, silver, cadmium or selenium coating a surface of iron, nickel, cobalt or iron oxide (Fe₃O₄).
(Item 67) A method according to any one of items 6, 26, 36, 40, 45, 49, 53 and 59, wherein the fine metal particle is a fine complex particle containing a layer of gold, silver, cadmium or selenium coating a surface of iron, nickel, cobalt or iron oxide (Fe₃O₄).
(Item 68) A method according to any one of items 1 to 6, wherein the mass spectrometry is carried out by LDI-TOF MS method.
(Item 69) A method according to item 13, wherein the mass spectrometry is carried out by LDI-TOF MS method.

### EFFECTS OF THE INVENTION

According to the present invention, a mass spectrometry method can be provided which has very high sensitivity to a sulfur atom-containing derivative of an organic residue.

The present invention enables accurate real-time monitoring for enzymatic reactions related to sugar chains. By presenting a sugar chain which is meant to be a substrate for the enzymatic reaction on a surface of a metal which enables diffuse reflection of a laser beam, the enzymatic reaction can be tracked with a mass spectrometry method. Even if a buffer or a salt, is present at a high concentration, which is undesired in the case of conventional mass spectrometry, the present invention can enable a highly-sensitive measurement without being affected by the buffer or a salt. Thus a part of the resultant reaction solution can be used for conveniently performing a sequent analysis without addtional processing. Since the resultant reaction products can be directly observed, the present method has an advantage in that kinetic analysis of the enzymatic reaction can be readily carried out.

In conventional MALDI-TOF MS methods, it is considered a problem that a peak of a substance of interest cannot be observed well in the "low molecular weight regions where a candidate compound may often show a peak", due to the intense peak of the matrix per se. However, the present invention has an advantage that matrix-free LDI-TOF MS can be carried out with a high sensitivity and accuracy, by making a low molecular weight compound of interest supported on a metal substrate (in particular, a fine metal particle) as in the present invention.

By providing a metal-organic residue complex particle of the present invention, a sugar chain or various functional groups can be provided on the surface of a fine metal particle, using the bond between a thiol group and a metal. Moreover, solubility of the fine metal particle can be freely controlled. Therefore, a fine metal particle coated with a thiol compound can be used as a solid phase carrier not only for reaction in an aqueous solvent but also for solid phase synthesis in an organic solvent. When a fine particle is suspended in a reaction solvent, it can be separated from the solution component by centrifugal filtration, and thus separate purification can be performed very readily, which is an advantage. Further, the progress of a reaction can be directly tracked by a mass spectrometry, thereby achieving efficiency.

The present invention has an advantage that use of a fine complex particle containing a magnetic particle such as iron, nickel, cobalt or iron oxide (Fe₃O₄) and a layer of gold, silver, cadmium, selenium or the like coating the surface of the particle as a fine metal particle, enables the easy recovery of the particle after the sulfur atom-containing derivative of the organic residue is supported on the fine complex particle, using a magnet or the like, thereby performing mass spectrometry without purification.

According to the present invention, a fine metal particle containing an organic residue without cytotoxicity bound through a sulfur atom to the fine metal particle is provided, thereby enabling direct introduction of a metal-organic residue complex particle into a living cell, and allowing for the accurate analysis of the various chemical reactions (for example, enzymatic reaction) in the cell by performing mass spectrometry of the reaction product.

### BRIEF DESCRIPTION OF THE DRAWINGS

Figure **1** shows a MALDI TOF Mass spectrum of a thiol compound supported on a surface of a fine gold particle.
   a) shows a MALDI-TOF Mass spectrum of a fine gold particle obtained from centrifugal filtration.
   b) shows a MALDI-TOF Mass spectrum of the filtrate.
Figure **2** shows a MALDI-TOF Mass spectrum after a chemical reaction using the fine metal particle as a solid phase carrier.
Figure **3** shows a MALDI-TOF Mass spectrum of a sugar chain-fine gold particle complex.
Figure **4** shows a MALDI-TOF Mass spectrum of a sugar chain (GlcNAc)-fine gold particle complex where the sugar chain density was controlled.
Figure **5** shows an electron micrograph of a sugar chain (GlcNAc)-fine gold particle complex where the sugar chain density is controlled.
Figure **6** shows temporal variation in MALDI-TOF Mass spectrum, obtained by tracking a sugar chain elongation reaction on a sugar chain (GlcNAc)-fine gold particle complex.
Figure **7** shows a time-activity plot in a sugar chain elongation reaction, obtained by the spectrum as shown in Figure **6.** Extent of the reaction on the vertical axis indicates values derived from the following formula: [ (peak intensity of a product)/(peak intensity of a starting substance + peak intensity of the product)].
Figure **8** shows measurement results of inhibiting activity of UDP against GalT reaction.
Figure **9** shows a MALDI-TOF Mass spectrum of a gold nanoparticle having sugar chain-trapping ability.
Figure **10** shows MALDI-TOF Mass spectrum after deprotection reaction of Boc group on a fine gold particle.
Figure **11** shows aMALDI-TOFMass spectrum after sugar chain (mannose)-trapping reaction in an aqueous solution using a sugar chain-trapping fine gold particle.
Figure **12** shows aMALDI-TOFMass spectrum after sugar chain (glucose)-trapping reaction in an aqueous solution using a sugar chain-trapping fine gold particle.
Figure **13** shows a MALDI-TOF Mass spectrum after sugar chain (N-acetylglucosamine) -trapping reaction in an aqueous solution using a sugar chain-trapping fine gold particle.
Figure **14** shows aMALDI-TOFMass spectrum after sugar chain (mannopentaose)-trapping reaction in an aqueous solution using a sugar chain-trapping fine gold particle.

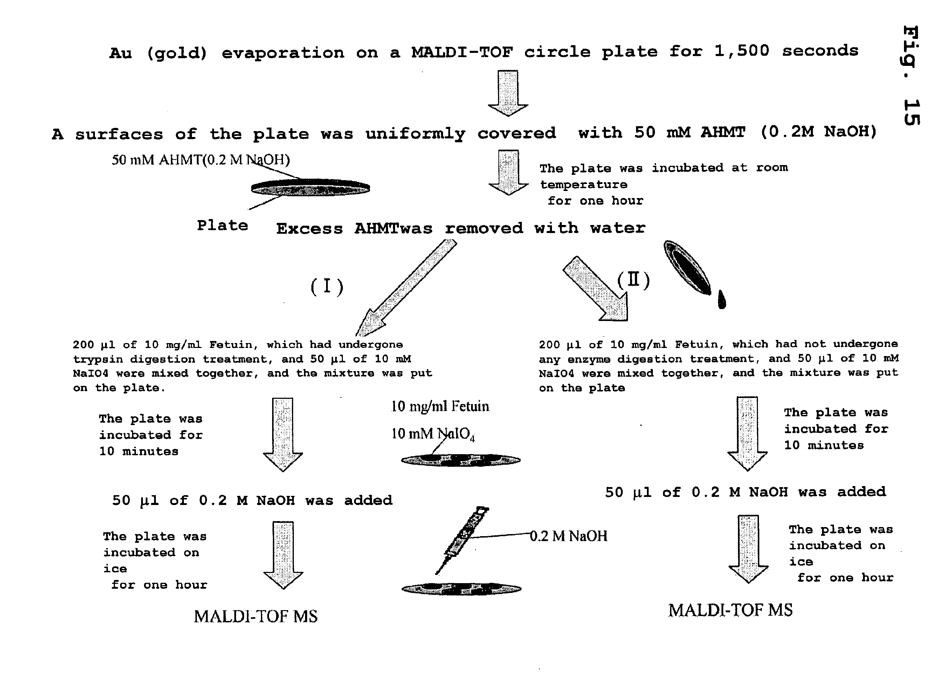

Figure **15** shows protein-trapping experiments of AHTM using a gold substrate (methods (I) and (II)).

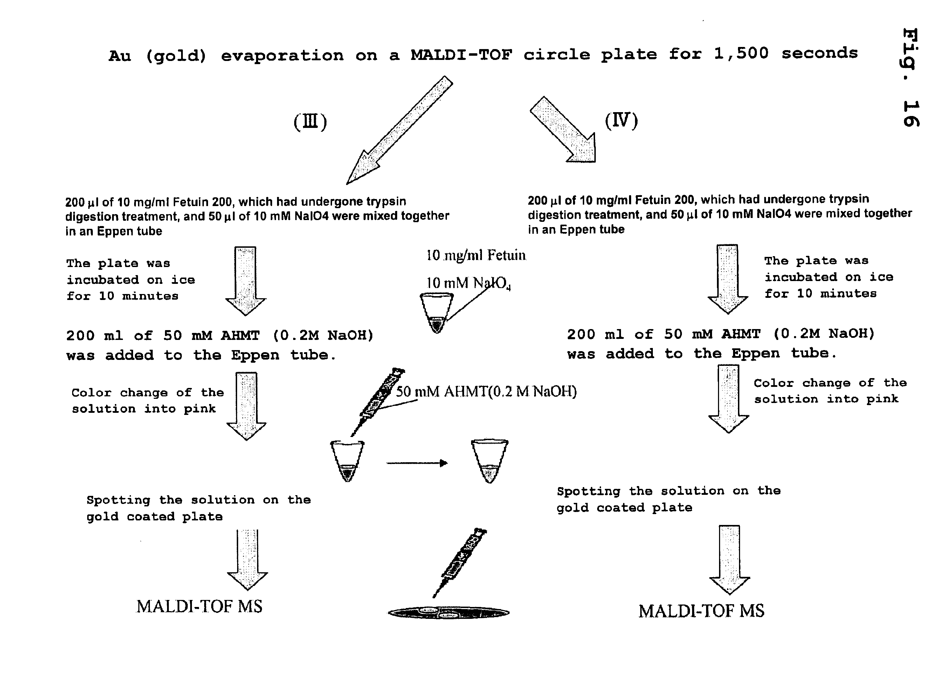

Figure **16** shows protein-trapping experiments of AHTM using a gold substrate (methods (III) and (IV)).
**Figure 17** shows a conceptual diagram of protein-trapping experiments of AHTM using a gold substrate (methods (I)-(IV)) and MALDI-TOF Mass spectrum obtained therein.
Figure **18** shows a schematic diagram of a sugar chain elongation reaction on a sugar chain (GlcNAc)-fine gold particle complex by a glycosyltransferase (galactosyltransferase) expressed in *E. coli,* and MALDI-TOF Mass spectrum obtained after the reaction.
Figure **19** shows a) LDI-TOF Mass spectrum and b) LDI-TOF Mass spectrum of a mixture solution of a sugar chain thiol (GlcNAc) and fine gold particle wherein the thiol compound was liberated by a process using iodine.
Figure **20** shows temporal variation of MALDI-TOFMass spectrum obtained by tracking a sugar chain elongation reaction on a sugar chain (Gal-GlcNAc)-fine gold particle complex by fucosyltransferase (FucT).
Figure **21** shows a time-activity plot in sugar chain elongation reaction, obtained by the spectrum as shown in Figure **20.** Values (activity) in the vertical axis show values calculated by the formula: [peak intensity of a product/(peak intensity of a starting substance+peak intensity of the product)].

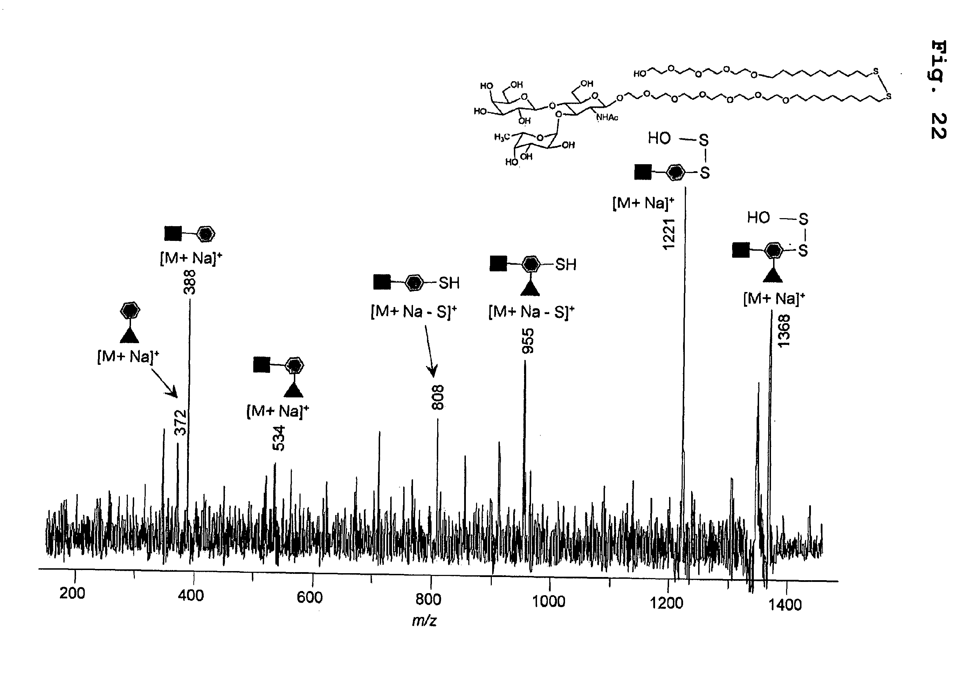

Figure **22** shows an LDI-TOF/TOF Mass spectrum of a sugar chain (Lex)-fine gold particle complex.
Figure 23 shows a) anLDI-TOFMass spectrumof a thiol (compound 1) -gold substrate complex; and b) an LDI-TOF Mass spectrum of a thiol (compound 1)-fine gold particle complex.
Figure **24** shows anLDI-TOF Mass spectrum of a thiol (compound 9)-magnetic fine gold particle complex.
Figure **25** shows a MALDI-TOF Mass spectrum (upper raw) and an LDI-TOF Mass spectrum (lower raw) of a sugar chain (GlcNAc)-magnetic fine gold particle complex.
Figure **26** shows a MALDI-TOF Mass spectrum before (upper raw) and after (lower raw) a sugar chain elongation reaction on a sugar chain (GlcNAc)-magnetic fine gold particle complex by galactosyltransferase.
Figure **27** shows an LDI-TOF Mass spectrum of a thiol (compound 9)-Cds fine particle complex.
Figure **28** shows a MALDI-TOFMass spectrum (upper raw) and LDI-TOF Mass spectrum (lower raw) after a glycosylation reaction on a fine gold particle.
Figure **29** shows aMALDI-TOFMass spectrumof a protein hooked from a mixture of proteins by a sugar chain (GlcNAc)-magnetic fine gold particle complex. The upper raw shows a MALDI-TOF Mass spectrum of a protein hooked by the sugar chain (GlcNAc)-magnetic fine gold particle complex. The middle raw shows a MALDI-TOF Mass spectrum of a standard substance PNA. The lower raw shows a MALDI-TOF Mass spectrum of a standard substance WGA.
Figure **30** shows a MALDI-TOFMass spectrumof a mixture solution of protein (α-amylase) bound to a fine gold particle and acarbose, which is an inhibitor, before the ultrafiltration process.
Figure **31** shows aMALDI-TOFMass spectrumof a mixture solution of protein (α-amylase) bound to a fine gold particle and acarbose, which is an inhibitor, after the ultrafiltration process.
Figure **32** shows aMALDI-TOFMass spectrumof a mixture solution of protein (α-amylase) bound to a fine gold particle and acarbose, which is an inhibitor, before the ultrafiltration process.

### BEST MODE FOR CARRYING OUT THE INVENTION

Hereinafter, the present invention will be described. It should be understood that, throughout the specification, expressions in singular forms also include concepts of plural forms unless otherwise noted. Furthermore, it should be understood that the terms as used herein have the meanings which are generally referred to in the field unless otherwise noted.

### TERMS

Hereinafter, definitions of the terms used herein will be listed.

As used herein, "metal" refers to a metallic element or metallic complex which contains a metal as a part thereof, to which a sulfur atom-containing substance (for example, thiol, disulfide and the like) can bind. Typical examples of a metal, to which a sulfur atom-containing substance can bind, include gold, silver, cadmium, selenium and the like.

As used herein, "a metal which has a surface enough to cause a diffuse reflection of a laser beam" indicates that the metal as defined above has an uneven surface or curved surface which causes a laser beam irradiated and reflected thereon to be irradiated on the surface of the metal for many times. More diffuse reflections of a laser beam enhance the sensitivity of mass spectrometry. Examples of an ideal metal having a surface enough to cause a diffuse reflection include fine metal particles.

As used herein, "fine metal particle" refers to a metal particle of a metal defined above which has an average particle size of 10nm to 5µm, and may be a monodisperse-type or a polydisperse-type. When a fine metal particle of the present invention is a metal complex, the metal complex takes a structure wherein a core particle thereof has a surface covered with a layer of the metal to which the above sulfur atom-containing substance can bind. Examples of typical fine metal particles used in the present invention include fine particles of a metallic element of gold or silver, or fine particles of a metallic complex formed by coating a surface of a magnetic particle such as iron, nickel, cobalt or iron oxide (Fe₃O₄) with gold or silver. A "fine metal particle" of the present invention forms a colloid in solution. In addition to fine metal particles mentioned above, Quantum Dots (trade name, Quantum Dot Corporation, Hayward, CA, USA; hereinafter, represented as "QDs") can also be used. A metal used for QDs may be, for example, ZnSe, CdS, CdSe, CdTe or the like. A typical example is a nanoparticle which has CdSe as a core and ZnS around the core. A -SH compound can be provided on the surface of QDs through bonds between Zn on the surface and -SH (this is a bond of the same type as that of Au-S). As used herein, the term "fine particle" may be used interchangeably with the term "nanoparticle" or "nano particle". Production and applications of a fine gold particle is described in JACS 125, 7790-7791 (2003); Angew Chem Int Ed 40 (12), 2257-2261 (2001); and Chem Eur J 9, 1909-1921 (2003).

As used herein, "organic residue" refers to a "biomolecule" as will be defined below, and to an organic compound synthesized artificially.

As used herein, "analyte" refers to a subject of analysis which is to be ionized in mass spectrometry, and encompasses an "organic residue" as defined above.

As used herein, "metal-organic residue complex" refers to a complex obtained by the bonding of an organic residue through a sulfur atom to a surface of a fine metal particle as defined above.

As used herein, "metal-organic residue complex particle" refers to a fine particle obtained by the bonding of an organic residue through a sulfur atom to a surface of a fine metal particle as defined above. Herein, particularly, a term "sugar chain-gold fine particle" is used for a fine particle obtained by the bonding of a sugar chain or a sugar chain-containing substance through a sulfur atom to a surface of a gold particle.

As used herein, "metal-analyte complex" refers to a complex obtained by the bonding of an analyte through a sulfur atom to a surface of a metal as defined above.

As used herein, "metal-analyte complex particle" refers to a fine particle obtained by the bonding of an analyte through a sulfur atom to a surface of a fine metal particle as defined above.

As used herein, "mass spectrometry" refers to changing a particle such as an atom, a molecule, a cluster and the like into ions in a gaseous state (i.e. ionizing) and accelerating the ions in a vacuum, thereby separating/detecting these ions using electromagnetic force as a ratio of mass with respect to charge. A mass spectrum is defined as a spectrum presented so that m/z is represented on a horizontal axis and the relative intensity of the ions is represented by peaks on a vertical axis based on the ions which have been separated/detected depending on their particular m/z. An ion which provides information of molecular weight is generally referred to as molecular-related ion (e.g. M⁺, M⁻, [M+H]⁺, [M+H]⁻, [M-H]⁺, [M+Na]⁺ and the like, generated from a neutral molecule M, by lack of one electron, addition of one electron, addition of a proton, lack of a proton, lack of a hydride, addition of an alkali metal (such as Na), respectively). Although in some cases no molecular-related ion appears, depending on the specimen or ionizing method (particularly in the case of the EI method), then a molecular-related ion can be confirmed by using a soft ionization method. An ion which appears at a mass lower than a molecular ion is referred to as "fragment ion". Such a "fragment ion" is generated by decomposition of the moleculer ion, and provides information of the structure of a specimen. An ion which is most intense in a spectrum is referred to as base peak, and is used for normalizing the spectrum by setting the relative intensity to 100%.

"Ionization" herein is carried out by a method appropriately selected from the following seven methods.
1) Electron ionization method (EI)
   Electron ionization is achieved by applying thermoelectrons to a vaporized analyte, and is the most general method of ionization. The ionization is carried out by gasifying an analyte, a liquid or solid analyte is required to be previously vaporized. Since heat is applied in order to vaporize an analyte, this method cannot measure a substance which is not thermostable, or a substance which hardly volatilizes. However, when an analyte is a substance which obtains volatility or thermostability through derivatization such as methylation, silylation, acylation, and the like, the analyte can be measured. The ionizing energy typically used is 70 eV, and fragment ions are generated as well as molecular ions due to excess energy (the ionizing energy of general organic compounds is somewhat more than 10eV.) The information of these fragment ions enables structural analysis of the compound. However, in many cases molecular weight information is not obtained because of difficulty in detecting the molecular weight. In such a case, it is required to reduce the ionizing energy to approximately 20eV, or to select a more soft ionization method (CI, DEI, DCI, FAB, and ESI).
2) Chemical ionization method (CI)
   This is a method of ionization by providing vaporized analyte into reactive gas (reagent gas) which has been previously ionized. Since the ionization is achieved using ion molecular reaction, the ionizing energy is approximate to the ionizing energy of an organic compound. Therefore, very few fragment ions are generated, and ions having molecular weight information ((M+H)⁺, (M+NH₄)⁺, (M-H)⁻ and the like) appear as a base ion. Methane, isobutane, and ammonia are generally used as reactive gases.
3) Desorption electron ionization method (DEI)
   This is a method of ionization by instantaneously heating an analyte close to an electron beam and vaporizing the analyte before it causes thermolysis. This method enables measurement of a substance which is not thermostable, or a substance which is difficult to volatilize. Since molecular ions appear more energetic than in a normal direct introduction method (such as an EI method) , molecular weight information can be readily obtained. The measurement is achieved by attaching an analyte solution to a tip of a point filament (platinum wire having a diameter of 100µm), and rapidly heating the filament after insertion into ion sources, by vaporizing the analyte.
4) Desorption chemical ionization method (DCI)
   In DCI, the procedure of DEI is performed using an ion source at a state as in CI.
5) Fast atom bombardment method (FAB)
   This is a method of ionization by mixing an analyte and matrix molecules, which are spread on a target, and collided with fast protons such as Xe. Since the analyte is not required to be vaporized, as compared to EI and CI methods, this method is suitable for measuring a substance which is not thermostable, or a substance which is difficult to volatilize. In some cases, however, a background peak derived from the matrix appears so intense that it is difficult to detect an analyte having a low molecular weight. In such a case, measurement by FRIT-FAB is effective.
6) FRIT-fast atom bombardment method (FRIT-FAB)
   This method is also referred to as Continuous-flow FAB. An analyte dissolved in matrix solution is continuously flown and fast protons are caused to collide with the eluate at an outlet, thereby performing ionization.
7) Electrospray ionization method (ESI)
   This is a method of atmospheric pressure ionization, using a phenomenon whereby an analyte solution is ionized in the form of an electrospray when a high voltage is applied to a capillary. Since the analyte is not required to be vaporized, similar to FAB, this method is suitable for measuring a substance which is not thermostable, or a substance which is difficult to volatilize. It allows a quadrupole mass spectrometer measuring a narrow range of mass to measure peptides or proteins having greater molecular weight. Since fragments having structural information are not obtained in a normal ESI measurement, a voltage higher than usual is applied to the Capillary/Skimmer-1, thereby causing In-source CID. Thus, fragment ions having structural information can be measured.

As used herein, "MALDI-TOF(MS)" refers to the abbreviation of Matrix Assisted Laser Desorption Ionization - Time-of-Flight (Mass spectrometry). MALDI is a method discovered by Tanaka et al., and developed by Hillenkamp et al. (Karas M., Hillenkamp, F., Anal. Chem. 1988, 60, 2299-2301). In this method, after a sample and a matrix solution are mixed to a molar ratio of (10⁻²-5×10⁻⁴) : 1, the mixed solution is dried on a target in a crystallized state. Large energy is given to the matrix by pulse laser irradiation, and thus ions are derived from the sample such as (M+H)⁺, (M+Na)⁺ and the like, and the ions derived from the matrix are dissociated from each other. Analysis is possible even when there is a contamination by a small amount of phosphoric acidbuffer, Trisbuffer, guanidine, and the like. MALDI-TOF (MS) measures mass based on time of flight. When an ion is accelerated at a certain accelerating voltage V, where the mass of the ion is m, velocity of the ion is v, charge number of the ion is z, elementary charge is e, and time of flight of the ion is t, m/z of the ion can be expressed by the formula m/z=2eVt²/L². For such MALDI-TOF measurement, KOMPACT MALDI II/III of Shimazu/Kratos or the like can be used. When such measurement is performed, reference can be made to pamphlets offered by the manufacturers. Irradiation energy of laser irradiation used for measurement by the MALDI-TOF is referred to as the "dissociation energy" herein.

As used herein, "LDI-TOF MS" refers to a method for dissociating/ionizing a molecule of interest by laser beam irradiation instead of using a low molecular weight matrix reagent in contrast to "MALDI-TOF MS" as defined above.

As used herein, "same entity" indicates that entities which are represented herein in plural form for convenience are the same individual.

As used herein, "sugar chain" refers to a compound formed by one or more unit sugars (monosaccharide and/or derivatives thereof) in series. When there are two or more unit sugars in series, each of the unit sugars are bound by dehydrocondensation by a glycosidic bond. Such sugar chains include a wide variety of sugar chains, for example, polysaccharide (glucose, galactose, mannose, fucose, xylose, N-acetylglucosamine, N-acetylgalactosamine, sialic acid and, complexs and derivatives thereof) included in living bodies, decomposed polysaccharides, sugar chains decomposed or derived from complex living body molecules such as glycoproteins, proteoglycans, glycosaminoglycans, glycolipids, and the like, but not limited to these. Thus, as used herein, the term sugar chain is used interchangeably with "polysaccharide", "glucid", and "carbohydrate". Furthermore, unless specifically referred to, "sugar chains" as used herein may include both sugar chains and a sugar chain-containing substance.

As used herein, "sugar chain-containing substance" refers to a substance including a sugar chain and a substance other than the sugar chain. Such a sugar chain-containing substance is found in living bodies in a large quantity, and includes, for example, a wide variety of polysaccharides included in living bodies, decomposed polysaccharides, sugar chains decomposed or derived from complex living body molecules such as glycoproteins, proteoglycans, glycosaminoglycans, glycolipids, and the like, but not limited to these.

As used herein, "monosaccharide" refers to a polyhydroxy aldehyde or polyhydroxy ketone which cannot be hydrolyzed into simpler molecules, and contains at least one hydroxyl group and at least one aldehyde group or ketone group, and derivatives thereof. Normally, monosaccharide are represented by the formula CₙH₂ₙOₙ, but is not limited thereto. Fucose (deoxyhexose), N-acetylglucosamine and the like are also included. The compounds in which, n=2, 3, 4, 5, 6, 7, 8, 9 and 10 in the above formula, are respectively referred to as diose, triose, tetrose, pentose, hexose, heptose, octose, nonose and decose . In general, the compounds correspond to aldehyde or ketone of linear polyvalent alcohols. The former are called aldoses, and the latter is called ketoses.

As used herein, particularly, "derivative of monosaccharide" refers to a substance produced as a result of substitution of one or more hydroxyl group on an unsubstituted monosaccharide by another substituent, which does not falls in the range of a monosaccharide. Such derivatives of monosaccharides include sugars having a carboxyl group (for example, aldonic acid which has the C-1 site oxidized and has became carboxylic acid (for example, D-gluconic acid having D-glucose oxidized), uronic acid having a C atom at the terminal which has become a carboxylic acid (D-glucuronic acid having D-glucose oxidized), sugar having an amino group or a derivative of an amino group (for example, acetylated amino group) (for example, N-acetyl-D-glucosamine, N-acetyl-D-galactosamine and the like) , sugar having both an amino group and a carboxyl group (for example, N-acetyl neuraminic acid (sialic acid), N-acetyl muramic acid and the like), deoxylated sugar (for example, 2-deoxy-D-ribose), sulfated sugar including a sulfuric acid group, phosphorylate sugar including a phosphate group, and the like, but not limited to these. In the present specification, monosaccharides also encompass the above derivative. Glycosides having an acetal structure formed by reacting an alcohol with a sugar forming to hemiacetal structure is also within the range of the monosaccharide.

As used herein, when "interaction" is used for describing two objects, it means that the two objects exert forces on each other. Such interaction includes, for example, covalent bonds, hydrogen bonds, van der Waals forces, ionic interactions, nonionic interactions, hydrophobic interactions, electrostatic interactions, and the like, but are not limited to these. Preferably, the interaction is between hydrogen bonds, hydrophobic interactions or the like. As used herein, "covalent bond" is used as having a normal meaning in the field of the art, and refers to chemical bonds formed when a pair of electrons are shared by two atoms. As used herein, "hydrogen bond" is used as having a normal meaning in the art and refers to a bond generated when an atom having high electronegativity attracts the only extranuclear electron of a hydrogen atom, the hydrogen atom nucleus is exposed, and the exposed hydrogen atom nucleus attracts another atom having high electronegativity. Such hydrogen bonds are generated between, for example, a hydrogen atom and an atom having high electronegativity (such as fluorine, oxygen, nitrogen or the like).

As used herein, "trapping of a sugar chain or a sugar chain-containing substance" indicates that a sugar chain or a sugar chain-containing substance is bound to a sugar chain-trapping site of the above complex by interaction of "metal-organic residue complex" or "metal-analyte complex" of the present invention and "sugar chain" or "sugar chain-containing substance". A sugar chain-trapping site of a complex of the present invention preferably includes a protected or unprotected aminooxy group, a protected or unprotected N-alkylaminooxy group, hydrazid group, azide group, thiosemicarbazide group, cysteine residue, and derivatives thereof.

As used herein, "transferring enzyme" is a generic term referring to enzymes which catalyze group transferring reactions. Herein, "transferring enzyme" may be used interchangeably to "transferase". The group transfer reaction occurs so that a group Y is transferred from a compound (donor) to another compound (receptor), as shown in the following formula (1) :

X-Y+Z⇔X-H+Z-Y (1)

As used herein, "glycosyltransferase" refers to an enzyme which catalyzes the transfer of a sugar (corresponding to group Y in the above formula (1); a saccharide unit or a sugar chain) from one site to another site, which correspond to the compounds X-Y and Z-H, respectively in the formula (1). Examples of glycosyltransferase include, but not limited to, for example, galactosyltransferase, glucosyltransferase, sialyltransferase, mannosyltransferase, fucosyltransferase, xylosyltransferase, N-acetylglucosaminyltransferase, N-acetylgalactosaminyltransferase and the like. Examples of such a glycotransferase include, but not limited to, for example, β1,4-galactosyltransferase, α-1,3- galactosyltransferase, β1,4-galactosyltransferase, β1,3-galactosyltransferase, β1,6-galactosyltransferase, α2,6-sialyltransferase, α1,4-galactosyltransferase, ceramide galactosyltransferase, α1,2-fucosyltransferase, α1,3-fucosyltransferase, α1,4-fucosyltransferase, α1,6-fucosyltransferase, α1,3-N-acetylgalactosaminyltransferase, α1,6-N-acetylgalactosaminyltransferase, β1,4-N-acetylgalactosaminyltransferase, polypeptide N-acetylgalactosaminyltransferase, β1,4-N-acetylgalactosaminyltransferase, β1,2-N-acetylgalactosaminyltransferase, β1,3-N-acetylgalactosaminyltransferase, β1,6-N-acetylgalactosaminyltransferase, α1,4-N-acetylgalactosaminyltransferase, β1,4-mannosyltransferase, α1,2-mannosyltransferase, α1,3-mannosyltransferase, α1,4-mannosyltransferase, α1,6-mannosyltransferase, α1,2-glucosyltransferase, α1,3-glucosyltransferase, α2,3-sialyltransferase, α2,8-sialyltransferase, α1,6-glucosaminyltransferase, α1,6-xylosyltransferase, β-xylosyltransferase (proteoglycan core structure synthesizing enzyme), β1,3-glucuronosyltransferase, hyaluronic acid synthesizing enzyme, a glycosyltransferase using other nucleotide sugar as a sugar donor, a glycosyltransferase using dolichol-phosphate-type sugar donor, and the like.

As used herein, "sugar chain elongation reaction" refers to a reaction where a chain length of a sugar chain elongates in the presence of a glycotransferase as defined above.

As used herein, the term "biomolecule" refers to a molecule related to living bodies. A sample including such biomolecules may be referred to as a biological sample in the present specification. As used herein, "living body" refers to a biological organic body, and includes animals, plants, fungi, virus and the like, but not limited to these. Therefore, a biomolecule includes molecules extracted from living bodies. However, it is not limited to this, and any molecule may fall within the definition of biomolecule as long as it can affect living bodies. Such biomolecules includes proteins, polypeptides, oligopeptides, peptides, polynucleotides oligonucleotides, nucleotides, nucleic acids (including, for example, DNA such as cDNA, genome DNA, RNA such as mRNA), polysaccharides, oligosaccharides, lipid, small molecules (for example, hormone, ligand, signaling substance , organic small molecule and the like), complex molecules thereof, and the like, but not limited to these. As used herein, the biomolecules may be, preferably, complex molecules including sugar chains, or sugar chains (for example, glycoproteins, glycolipids and the like).

The source of such a biomolecule is not particularly limited as long it is a material to which sugar chains derived from living organisms are bound or attached. It may be animal, plant, bacterial, or viral. Abiological sample derived from an animal is preferable. For example, whole blood, blood plasma, blood serum, sweat, saliva, urine, pancreatic fluid, amnionic fluid, cerebrospinal fluid and the like are preferable. More preferably, it may be blood plasma, blood serum, or urine. The biological sample includes a biological sample which has not previously been isolated from a subject. The biological sample may include, for example, mucosal tissue or glandular tissue to which a sample can be attached from the outside, and preferably, the epithelium of ductal tissue attached to the mammary glands, prostate, or pancreas.

As used herein, terms "protein", "polypeptide", "oligopeptide" and "peptide" have the same meaning in the present specification and refer to a polymer of an amino acid having any length. This polymer may be straight, branched or cyclic. An amino acidmaybe natural or unnatural, and may be a modified amino acid. These terms may encompass those assembled with a complex of a plurality of polypeptide chains. These terms further encompass a naturally occurring or artificially modified amino acid polymer. Examples of such a modification include, for example, formation of a disulfide bond, glycosylation, lipidation, acetylation, phpophorylation, or any other manipulation or modification (for example, conjugation with a label component). The definition also encompasses, for example, a polypeptide including one or two or more analog(s) of (including, for example, an unnatural amino acid and the like) peptide-like compounds (for example, peptoid) and other modifications known in the art. A gene product of the present invention usually takes the form of a polypeptide. Such a gene product of the present invention in the polypeptide form is useful as a composition for diagnosis, prophylaxis, treatment or prognosis of the present invention.

The terms "polynucleotide", "oligonucleotide" and "nucleic acid" used herein have the same meaning and refer to a polymer of a nucleotide having any length. These terms also encompass "derivative oligonucleotide" or "derivative polynucleotide". "Derivative oligonucleotide" or "derivative polynucleotide" refers to an oligonucleotide or a polynucleotide which contains a derivative of a nucleotide or which has a bond between nucleotides different from a usual bond, and are used interchangeably. Specific examples of such an oligonucleotide include, for example, 2'-O-methyl-ribonucleotide, a derivative oligonucleotide in which phosphodiester bond in an oligonucleotide is replaced with phosphorothioate bond, a derivative oligonucleotide in which phosphodiester bond in an oligonucleotide is replaced with N3' -P5' phosphoroamidate bond, a derivative oligonucleotide in which ribose and phosphodiester bond in an oligonucleotide are replaced with a peptide nucleic acid bond, a derivative oligonucleotide in which uracil is substituted with C-5 propinyl uracil, a derivative oligonucleotide in which uracil is substituted with C-5 thiazole uracil, a derivative oligonucleotide in which cytosine is substituted with C-5 propinyl cytosine, a derivative oligonucleotide in which cytosine is substituted with phenoxazine-modified cytosine, a derivative oligonucleotide in which the ribose in DNA is substituted with 2'-O-propyl ribose, a derivative oligonucleotide in which the ribose in oligonucleotide is substituted with methoxyethoxy ribose and the like. If not described otherwise, specific nucleic acid sequences are intended to encompass sequences explicitly described and a conservatively varied variant thereof (for example, a degenerate codon substitution) and complementary sequences. Specifically, a degenerate codon substitution is achieved by creating a sequence in which the third position of one or more (or all) codon (s) is substituted with a mixed base and/or a deoxyinosine residue (Batzer et al., Nucleic acid Res. 19: 5081 (1991); Otsuka et al. , J. Biol. Chem. 260: 2605-2608 (1985); Rossolini et al. , Mol. Cell. Probes 8: 91-98 (1994)). A gene in the present invention usually takes a polynucleotide form. A gene or a gene product of the present invention in such a polynucleotide form is useful as a composition for diagnosis, prophylaxis, treatment or prognosis in the present invention.

As used herein, "isolation" of a substance (biological agents, for example, sugar chains , nucleic acids, proteins and the like) refers to substantially separating it or purifying it from other substances in a cell of a living body in which the biological agent naturally exist (for example: when the agent is a sugar chain or a sugar chain-containing substance, agents other than the sugar chains or sugar chain-containing substance, or sugar chains or sugar chain-containing substances other than the target sugar chains or sugar chain-containing substance; when the agent is nucleic acid, agents other than the nucleic acid, and nucleic acids including nucleic acid sequences other than the target nucleic acid; when the agent is a protein, agents other than the protein and proteins including amino acid sequences other than the target protein). The "isolated" sugar chains or sugar chain-containing substance include sugar chains or sugar chain-containing substance purified by a purification method of the present invention. Thus, the isolated sugar chains or sugar chain-containing substance include chemically synthesized sugar chains or sugar chain-containing substance.

As used herein, "purification" of a substance (biological agents, for example, sugar chains, nucleic acids, proteins, and the like) refers to removing at least a part of the agents which are associated to the substance in nature. Thus, purification and separation partially overlap in their forms. Typically, the purity of the purified substance (for example, biological agents such as sugar chains or sugar chain-containing substance), is higher than that of the substance in its usual existing state (i.e., is concentrated) , but, as long as the agents associated in nature are reduced, the state in which the substance is not concentrated is within the concept of "purification".

As used herein, "concentration" of a substance (for example, biological agents such as sugar chains or sugar chain-containing substance) refers to an action to raise the concentration of the substance to be higher than the content of the substance included in the sample before concentration. Thus, the concept of concentration also overlaps those of purification and separation. Typically, concentrated substance (for example, biological agents such as sugar chains or sugar chain-containing substance) have reduced impurities compared to those with the substance in its usual existing state. However, as long as the content of the target substance increases, certain impurities may be increased, and a not "purified" state is also included within the concept of "concentration".

As used herein, "conditions where a metal-organic residue complex and a sugar chain-containing substance may react with each other" refers to conditions sufficient for the metal-organic residue complex to react (preferably, to form covalent bond) with the sugar chain or sugar chain-containing substance (for example, buffers, polarities of solvents, temperature, pH, salt concentration, pressures, and the like). Setting parameters which are necessary for such conditions are within the range of the skills of those skilled in the art. By considering a variety of parameters which are related to the interaction, such as, the type of interaction, the types of sugar chains or sugar chain-containing substance, the type of sugar chain-trapping carriers (for example, as a functional group which can react with an aldehyde group in a fluid, protected or unprotected N-alkylaminooxy group, hydrazide group, azide group, thiosemicarbazide group, cysteine residue and derivatives thereof), those skilled in the art can set the conditions using well-known techniques in the art to perform the interaction reaction. In a preferable embodiment, such conditions may be conditions in which, sugar chains react with aldehyde groups to form specific and stable bonds in an equilibrium between the cyclic hemiacetal type and the non-cyclic aldehyde type states which are formed by sugar chains in fluids such as aqueous solutions, but not limited to these. Alternatively, it may be a preferable condition that a fluid supplied for reaction includes substantially no keto group. Such a condition may be, for example, to use an acetic acid buffer of pH 5.6 at room temperature and atmospheric pressure (for example, 20°C and 1 atmosphere).

### (Organic chemistry)

Organic chemistry is described in, for example, Organic Chemistry, R. T. Morrison, R. N. Boyd 5th ed. (1987) and the like, relevant portions of which are incorporated herein as a reference.

As used herein, "substitution" refers to substituting one or two or more hydrogen atom(s) in an organic compound or a substituent with another atom or atom group, if not particularly mentioned. It is possible to substitute one hydrogen atom with a monovalent substituent, and to substitute two hydrogen atoms with a bivalent substituent.

When an organic residue of a "metal-organic residue complex" or an analyte of "metal-analyte complex" of the present invention is substituted with a substituent R, there is a single or a plurality of such R's, and when there are a plurality of R's, such R's are independently selected from the group consisting of hydrogen, alkyl, substituted alkyl, cycloalkyl, substituted cycloalkyl, alkenyl, substituted alkenyl, cycloalkenyl, substituted cycloalkenyl, alkynyl, substituted alkynyl, alkoxy, substituted alkoxy, carbocyclic group, substituted carbocyclic group, heterocyclic group, substituted heterocyclic group, halogen, hydroxyl, substituted hydroxyl, thiol, substituted thiol, cyano, nitro, amino, substituted amino, carboxy, substituted carboxy, acyl, substituted acyl, thiocarboxy, substituted thiocarboxy, amide, substituted amide, substituted carbonyl, substituted thiocarbonyl, substituted sulfonyl and substituted sulfinyl.

As a substituent R for enhancing the solubility of a "metal-organic residue complex" or "metal-analyte complex" of the present invention with respect to an aqueous solvent, a polar group per se such as a carboxy group or amino group, or a saturated or unsaturated carbon chain having a polar group such as a carboxy group or an amino group as a substituent in the molecule is preferable. In contrast, a substituent R for solubilizing a "metal-organic residue complex" of the present invention in an organic solvent, uses a hydrophobic substituent, for example, C1-C6 alkyl, C1-C5 alkyl, C1-C4 alkyl, C1-C3 alkyl, C1-C2 alkyl or the like.

As used herein, "heterocycle (group") refers to a group having a cyclic structure including carbon and hetero atoms. Herein, hetero atoms may be selected from a group consisting O, S and N, may be the same or different from each other, and one or more of them may be included. A heterocyclic group may be aromatic or nonaromatic, and may be monocyclic or polycyclic. The heterocyclic group may be substituted.

As used herein, "alcohol" refers to an organic compound having 1 or more hydrogen atoms of an aliphatic hydrocarbon substituted by a hydroxyl group. It is also represented as ROH in the present specification. Herein, R is an alkyl group. Preferably, R may be C1-C6 alkyl. Alcohol may be, for example, methanol, ethanol, 1-propanol, 2-propanol and the like, but is not limited to these. As used herein, "heterocycle (group) which may be substituted" means that either "heterocycle (group)" or "substituted heterocycle (group)" as defined above may be used.

As used herein, "alkyl" refers to a monovalent group generated when one hydrogen atom is lost from aliphatic hydrocarbon (alkane) such as methane, ethane, propane, and the like, and is represented by CₙH₂ₙ₊₁- in general (herein, n is a positive integer). Alkyl may be a straight chain or a branched chain. As used herein, "substituted alkyl" refers to an alkyl having the H of an alkyl substituted by a substituent as defined below. Specific examples of such alkyls may be, C1-C2 alkyl, C1-C3 alkyl, C1-C4 alkyl, C1-C5 alkyl, C1-C6 alkyl, C1-C7 alkyl, C1-C8 alkyl, C1-C9 alkyl, C1-C10 alkyl, C1-C11 alkyl or C1-C12 alkyl, C1-C2 substituted alkyl, C1-C3 substituted alkyl, C1-C4 substituted alkyl, C1-C5 substituted alkyl, C1-C6 substituted alkyl, C1-C7 substituted alkyl, C1-C8 substituted alkyl, C1-C9 substituted alkyl, C1-C10 substituted alkyl, C1-C11 substituted alkyl, or C1-C12 substituted alkyl. Herein, for example, C1-C10 alkyl denotes straight chain or branched alkyl having 1-10 carbon atoms, and examples may be methyl (CH₃-), ethyl (C₂H₅-), n-propyl (CH₃CH₂CH₂-), isopropyl ((CH₃)₂CH-), n-butyl (CH₃CH₂CH₂CH₂-), n-pentyl (CH₃CH₂CH₂CH₂CH₂-), n-hexyl (CH₃CH₂CH₂CH₂CH₂CH₂-), n-heptyl (CH₃CH₂CH₂CH₂CH₂CH₂CH₂-), n-octyl (CH₃CH₂CH₂CH₂CH₂CH₂CH₂CH₂-), n-nonyl (CH₃CH₂CH₂CH₂CH₂CH₂CH₂CH₂CH₂-), n-decyl (CH₃CH₂CH₂CH₂CH₂CH₂CH₂CH₂CH₂CH₂-), -C (CH₃)₂CH₂CH₂CH(CH₃)₂, -CH₂CH(CH₃)₂. Further, for example, C1-C10 substituted alkyl refers to C1-C10 alkyl having one or more hydrogen atoms substituted by substituents.

As used herein, "lower alkyl" refers to a C1-C6 alkyl, and is preferably a C1 or C2 alkyl.

As used herein, "cycloalkyl" refers to an alkyl having a cyclic structure. The term "substituted cycloalkyl" refers to a cycloalkyl having the H of the cycloalkyl substituted by a substituent defined below. Specific examples of cycloalkyls may be C3-C4 cycloalkyl, C3-C5 cycloalkyl, C3-C6 cycloalkyl, C3-C7 cycloalkyl, C3-C8 cycloalkyl, C3-C9 cycloalkyl, C3-C10 cycloalkyl, C3-C11 cycloalkyl, C3-C12cycloalkyl, C3-C4substituted cycloalkyl, C3-C5 substituted cycloalkyl, C3-C6 substituted cycloalkyl, C3-C7 substituted cycloalkyl, C3-C8 substituted cycloalkyl, C3-C9 substituted cycloalkyl, C3-C10 substituted cycloalkyl, C3-C11 substituted cycloalkyl or C3-C12 substituted cycloalkyl. For example, cycloalkyl may be cyclopropyl, cyclohexyl, or the like.

As used herein, "alkylene" refers to a bivalent group derived from an "alkyl", and may be, for example, methylene, ethylene, trimethylene, tetramethylene, pentamethylene, hexamethylene, heptamethylene, octamethylene, nonanemethylene, decanemethylene, undecamethylene, dodecamethylene, and the like.

As used herein, "alkenyl" refers to a monovalent group generated when one hydrogen atom is lost from an aliphatic hydrocarbon having one double bond in a molecule, such as ethylene and propylene, and, in general, is represented by CₙH₂ₙ₋₁- (herein, n is a positive integer of 2 or higher). The term "substituted alkenyl" refers to an alkenyl having the H of the alkenyl substituted by a substituent as defined below. Specific examples of alkenyls may be C2-C3 alkenyl, C2-C4 alkenyl, C2-C5 alkenyl, C2-C6 alkenyl, C2-C7 alkenyl, C2-C8 alkenyl, C2-C9 alkenyl, C2-C10 alkenyl, C2-C11 alkenyl or C2-C12 alkenyl, C2-C3 substituted alkenyl, C2-C4 substituted alkenyl, C2-C5 substituted alkenyl, C2-C6 substituted alkenyl, C2-C7 substituted alkenyl, C2-C8 substituted alkenyl, C2-C9 substituted alkenyl, C2-C10 substituted alkenyl, C2-C11 substituted alkenyl or C2-C12 substituted alkenyl. Herein, for example, C2-C10 alkyl refers to a straight chain or branched alkenyl including 2-10 carbon atoms, and examples of alkyls include vinyl (CH₂=CH-), allyl (CH₂=CHCH₂-) , CH₃CH=CH- and the like. Further, for example, C2-C10 substituted alkenyl refers to C2-C10 alkenyl which has 1 or more hydrogen atoms substituted by substituents.

As used herein, "cycloalkenyl" refers to an alkenyl having a cyclic structure. The term "substituted cycloalkenyl" refers to a cycloalkenyl having the H of a cycloalkenyl substituted by a substituent as defined below. Specific examples of cycloalkenyl may be C3-C4 cycloalkenyl, C3-C5 cycloalkenyl, C3-C6 cycloalkenyl, C3-C7 cycloalkenyl, C3-C8 cycloalkenyl, C3-C9 cycloalkenyl, C3-C10 cycloalkenyl, C3-C11 cycloalkenyl, C3-C12 cycloalkenyl, C3-C4 substituted cycloalkenyl, C3-C5 substituted cycloalkenyl, C3-C6 substituted cycloalkenyl, C3-C7 substituted cycloalkenyl, C3-C8 substituted cycloalkenyl, C3-C9 substituted cycloalkenyl, C3-C10 substituted cycloalkenyl, C3-C11 substituted cycloalkenyl or C3-C12 substituted cycloalkenyl. For example, preferable examples of cycloalkenyl include 1-cyclopentenyl, 2- cyclohexenyl or the like.

As used herein, "alkenylene" refers to a bivalent group derived from "alkenyl", and may be, for example, vinylene, propylene, butenylene, and the like.

Asusedherein, "alkynyl" refers to amonovalent group generated when one hydrogen atom is lost from an aliphatic hydrocarbon having one triple bond in a molecule, such as acetylene, and, in general, is represented by CₙH₂ₙ₋₃- (herein, n is a positive integer of 2 or higher). The term "substituted alkynyl" refers to alkynyl having the H of the alkynyl substituted by a substituent as defined below. Specific examples of alkynyls may be C2-C3 alkynyl, C2-C4 alkynyl, C2-C5 alkynyl, C2-C6 alkynyl, C2-C7 alkynyl, C2-C8 alkynyl, C2-C9 alkynyl, C2-C10 alkynyl, C2-C11 alkynyl, C2-C12 alkynyl, C2-C3 substituted alkynyl, C2-C4 substituted alkynyl, C2-C5 substituted alkynyl, C2-C6 substituted alkynyl, C2-C7 substituted alkynyl, C2-C8 substituted alkynyl, C2-C9 substituted alkynyl, C2-C10 substituted alkynyl, C2-C11 substituted alkynyl or C2-C12 substituted alkynyl. Herein, for example, C2-C10 alkynyl refers to, for example, a straight chain or branched alkynyl including 2-10 carbon atoms, and examples of alkynyl may be ethynyl (CH≡C-), 1-propynyl (CH₃C≡C-) or the like. Further, for example, C2-C10 substituted alkynyl refers to C2-C10 alkynyl having 1 or more hydrogen atoms substituted by substituents.

As used herein, "alkynylene" refers to a bivalent group derived from "alkynyl", and may be, for example, propynylene, butynylene, or the like.

As used herein, "alkoxy" refers to a monovalent group generated when a hydrogen atom of a hydroxy group of an alcohol is lost, and in general, is represented by CₙH₂ₙ₊₁O- (herein, n is an integer of 1 or higher). The term "substituted alkoxy" refers to alkoxy having H of the alkoxy substituted by a substituent as defined below. Specific examples of alkoxys may be C1-C2 alkoxy, C1-C3 alkoxy, C1-C4 alkoxy, C1-C5 alkoxy, C1-C6 alkoxy, C1-C7 alkoxy, C1-C8 alkoxy, C1-C9 alkoxy, C1-C10 alkoxy, C1-C11 alkoxy, C1-C12 alkoxy, C1-C2 substituted alkoxy, C1-C3 substituted alkoxy, C1-C4 substituted alkoxy, C1-C5 substituted alkoxy, C1-C6 substituted alkoxy, C1-C7 substituted alkoxy, C1-C8 substituted alkoxy, C1-C9 substituted alkoxy, C1-C10 substituted alkoxy, C1-C11 substituted alkoxy or C1-C12 substituted alkoxy. Herein, for example, C1-C10 alkoxy refers to a straight chain or branched alkoxy including 1-10 carbon atoms, and examples of alkoxys may be methoxy (CH₃O-), ethoxy (C₂H₅O-), n-propoxy (CH₃CH₂CH₂O-), and the like.

As used herein, "carbocyclic group" refers to a group which includes a cyclic structure including only carbons, and which is a group other than the above-mentioned "cycloalkyl", "substituted cycloalkyl", "cycloalkenyl", and "substituted cycloalkenyl". A carbocyclic group may be aromatic or nonaromatic, and may be monocyclic or polycyclic. The term "substituted carbocyclic group" refers to a carbocyclic group having the H of the carbocyclic group substituted by a substituent as defined below. Specific examples of carbocyclic groups may be C3-C4 carbocyclic group, C3-C5 carbocyclic group, C3-C6 carbocyclic group, C3-C7 carbocyclic group, C3-C8 carbocyclic group, C3-C9 carbocyclic group, C3-C10 carbocyclic group, C3-C11 carbocyclic group, C3-C12 carbocyclic group, C3-C4 substituted carbocyclic group, C3-C5 substituted carbocyclic group, C3-C6 substituted carbocyclic group, C3-C7 substituted carbocyclic group, C3-C8 substituted carbocyclic group, C3-C9 substituted carbocyclic group, C3-C10 substituted carbocyclic group, C3-C11 substituted carbocyclic group, or C3-C12substituted carbocyclic group. The carbocyclic group may also be C4-C7 carbocyclic group or C4-C7 substituted carbocyclic group. The examples of carbocyclic group may be a phenyl group having one hydrogen atom deleted. The deletion site of the hydrogen may be any site which is chemically possible, and it may be on an aromatic ring or on a nonaromatic ring.

Asusedherein, "heterocyclicgroup"referstoagroup having a cyclic structure including carbon and hetero atoms. Herein, hetero atoms may be selected from a group consisting of O, S and N, may be the same or different from each other, and one or more atoms may be included. A heterocyclic group may be aromatic or nonaromatic, and may be monocyclic or polycyclic. The term "substituted heterocyclic group" refers to a heterocyclic group having the H of the heterocyclic group substituted by a substituent as defined below. Specific examples of heterocyclic group may be C3-C4 carbocyclic group, C3-C5 carbocyclic group, C3-C6 carbocyclic group, C3-C7 carbocyclic group, C3-C8 carbocyclic group, C3-C9 carbocyclic group, C3-C10 carbocyclic group, C3-C11 carbocyclic group, C3-C12 carbocyclic group, C3-C4 substituted carbocyclic group, C3-C5 substituted carbocyclic group, C3-C6 substituted carbocyclic group, C3-C7 substituted carbocyclic group, C3-C8 substituted carbocyclic group, C3-C9 substituted carbocyclic group, C3-C10 substituted carbocyclic group, C3-C11 substituted carbocyclic group, or C3-C12 substituted carbocyclic group, which has one or more carbon atoms substituted by hetero atoms. The heterocyclic group may also be a C4-C7 carbocyclic group or C4-C7 substituted carbocyclic group, which has one or more carbon atoms substituted with hetero atoms. The examples of heterocyclic groups may be a thienyl group, pyrrolyl group, furyl group, imidazolyl group, pyridyl group, or the like. The deletion site of the hydrogen may be any site which is chemically possible, and it may be on an aromatic ring or on a nonaromatic ring.

As used herein, "phenyl group" refers to a C6 aromatic carbocyclic group and is a functional group lacking one H from benzene. "Substituted phenyl group" refers to a group in which H in a phenyl group is substituted with a substituent as defined below.

As used herein, carbocyclic group or heterocyclic group may be substituted by a bivalent substituent in addition to being able to be substituted by a monovalent substituent as defined below. Such a bivalent substitution may be an oxo substitution (=O) or a thioxo substitution (=S).

Asusedherein, "halogen" refers to a monovalent group of elements such as fluorine (F), chlorine (Cl), bromine (Br), iodine (I) and the like which belong to group 7B of the periodic table.

As used herein, "hydroxy" refers to a group represented by -OH. The term "substituted hydroxy" refers to hydroxy having the H of the hydroxy substituted by a substituent as defined below.

As used herein, "thiol" (mercapto group) is a group having the oxygen atom of a hydroxy group substituted by a sulfur atom, and is represented by -SH. The term "substituted thiol" refers to a group having the H of a mercapto group substituted by a substituent as defined below.

As used herein, "cyano" refers to a group represented by -CN, and "nitro" refers to a group represented by -NO₂. The term "amino" refers to a group represented by -NH₂. The term "substituted amino" refers to amino having an H substituted by a substituent defined below.

As used herein, "carboxy" refers to a group represented by -COOH. The term "substituted carboxy" is carboxy having an H substituted by a substituent as defined below.

As used herein, "thiocarboxy" refers to a group having an oxygen atom of carboxy group substituted with a sulfur atom, and can be represented by -C(=S) OH, -C (=O) SH or -CSSH. The term "substituted thiocarboxy" is thiocarboxy having the H substituted by a substituent as defined below.

As used herein, "acyl" refers to alkylcarbonyl containing the "alkyl" bound to carbonyl, cycloalkylcarbonyl containing the "cycloalkyl" bound to alkyl portion, and arylcarbonyl containing the "aryl" bound to carbonyl. "Acyl" refers to, for example, acetyl, n-propanoyl, i-propanoyl, n-butyloyl, t-butyloyl, cyclopropanoyl, cyclobutanoyl, cyclopentanoyl, cyclohexanoyl, benzoyl, α-naphthoyl, and β-naphthoyl. "Substituted acyl" refers to acyl having hydrogen substituted with a substituent as defined below.

As used herein, "amide" refers to a group having a hydrogen of ammonia substituted with an acid group (acyl group), and, preferably, represented by -CONH₂. The term "substituted amide" refers to amide which is substituted by a substituent as defined below.

As used herein, "carbonyl" refers to a generic term for a substance including - (C=O) -, which is a specific group of aldehydes and ketones. The term "substituted carbonyl" refers to a carbonyl group substituted by a substituent selected as described below.

As used herein, "thiocarbonyl" refers to a group having the oxygen atom of carbonyl substituted by a sulfur atom, and includes a specific group -(C=S)-. The thiocarbonyl includes thioketone and thioaldehyde. The term "substituted thiocarbonyl" refers to a thiocarbonyl substituted by a substituent selected as described below.

As used herein, "sulfonyl" is a generic term for a substance including a specific group, -SO₂-. The term "substituted sulfonyl" refers to a sulfonyl substituted by a substituent selected as described below.

As used herein, "sulfinyl" is a generic term for a substance including a specific group, -SO-. The term "substituted sulfinyl" refers to a sulfinyl substituted by a substituent selected as described below.

As used herein, "aryl" refers to a group generated when one hydrogen atom linked to a ring of aromatic hydrocarbons is disengaged, and included in a carbocyclic group in the present specification. Examples thereof include phenyl, α-naphthyl, β-naphthyl, anthnyl, indenyl, phenanthryl and the like. "Substituted aryl" refers to an aryl substituted with a substituent selected as described below.

As used herein, "heteroaryl" refers to a group generated when one hydrogen atom linked to a ring of aromatic hydrocarbons having hetero atoms is disengaged, and included in a "heterocyclic group" in the present specification. Examples thereof include furanyl, thiophenyl, pyridyl and the like. "Substituted heteroaryl" refers to a heteroaryl substituted with a substituent selected as described below.

As used herein, "arylene" may be, for example, phenylene, naphtylene, or the like. More particularly, examples of arylene include 1,2-phenylene, 1,3-phenylene, 1,4-phenylene and the like.

As used herein, "heteroarylene" may be, for example, thiophendiyl, furandiyl, pyridindiyl, or the like. More particularly, examples of heteroarylene include 2,5-thiophendiyl, 2,5-furandiyl and the like.

As used herein, "hidroxylamino" refers to a monovalent group generated by removing a hydrogen atom from hydroxylamine NH₂OH. "Substituted hydroxylamino" refers to hydroxylamino substituted with a substituent selected as described below.

As used herein, "N-alkylhydroxylamino" refers to hydroxylamino having a hydrogen atom binding to a nitrogen atom of hydroxylamine, substituted with an alkyl group.

As used herein, "hydrazide" is a group represented by -CONHNH₂. "Substituted hydrazide" refers to a hydrazide substituted with a substituent selected as described below.

As usedherein, "thiosemicarbazide" refers to a group represented by H₂NCSNHNH₂. "Substituted thiosemicarbazide" refers to a thiosemicarbazide substituted with a substituent selected as described below.

As used herein, "ester" refers to a generic term for a substance including -COO-, which is a specific group. The term "substituted ester" refers to ester substituted with a substituent selected as describe below.

As used herein, "quaternary ammonium salt" is a group represented by -N⁺(R⁴) (R⁵) (R⁶), and R⁴, R⁵ and R⁶ refer to a lower alkyl group. Here, "lower alkyl" is the same as defined above. Normally, in "quaternary ammonium salt", a salt is formed so that -N⁺(R⁴) (R⁵) (R⁶) and a halide ion are paired.

As used herein, "hydroxyl group" refers to a group represented by -OH. "Hydroxyl group" is interchangeable with "hydroxyl group".

As used herein, "aldehyde" refers to a generic term for a substance including -CHO, which is a specific group. "Substituted aldehyde" refers to an aldehyde substituted with a substituent selected as described below, and may be used interchangeably with "aldehyde derivative".

As used herein, "carboxylic acid" refers to a generic term for a substance including -COOH, which is a specific group. "Substituted carboxylic acid" refers to a carboxylic acid substituted with a substituent selected as described below, and may be used interchangeably with "aldehyde derivative".

As used herein, C1, C2 ... Cn indicates carbon number. Therefore, C1 is used for indicating a substituent having carbon number of 1.

As used herein, "enantiomer" refers to one of a pair of compounds which cannot be superimposed since the structure of their crystals ormolecules are mirror images of one another, or the pair itself. Enantiomers are a type of stereoisomer, and all of their properties, other than optical rotation, are same.

As used herein, unless otherwise noted, the term "substitution" refers to substituting one or more hydrogen atoms in an organic compound or a substituent by another atom or atomic group. It is possible to remove one hydrogen atom and substitute with a monovalent substituent, and remove two hydrogen atoms and substitute with a bivalent substituent.

The substituents may be alkyl, cycloalkyl, alkenyl, cycloalkenyl, alkynyl, alkoxy, carbocyclic group, heterocyclic group, halogen, hydroxy, thiol, cyano, nitro, amino, carboxy, carbamoyl, acyl, acylamino, thiocarboxy, amide, substituted carbonyl, substituted thiocarbonyl, substituted sulfonyl or substituted sulfinyl, but is not limited to these. When a substituent is substituted, it may be substituted with alkyl, cycloalkyl, alkenyl, cycloalkenyl, alkynyl, alkoxy, alkenyloxy, alkynyloxy, alkoxyalkyl, halogenoalkyl, halogen, nitro, cyano, acyl, acyloxy, hydroxy, mercapto, carboxy, thiocarboxy, alkoxycarbonyl, aryloxycarbonyl, carbamoyl, and an amino which may be substituted or unsubstituted.

As used herein, "protection reaction" refers to a reaction to add a protecting group such as Boc (t-buthoxycarbonyl group) to a functional group which is desired to be protected. By protecting a functional group with a protecting group, the reaction of a functional group having high reactivity can be suppressed, and only a functional group having lower reactivity reacts.

As used herein, "deprotection reaction" refers to a reaction to disengage a protecting group such as Boc. The deprotection reaction may be a reaction such as reaction using trifluoroacetic acid (TFA) or a reduction reaction using Pd/C.

Typical examples of "protecting group" used herein include, for example, fluorenyl methoxy carbonyl group (Fmoc) , acetyl group, benzyl group, benzoyl group, t-buthoxycarbonyl group, a t-butyldimethyl group, the silyl group, trimethylsilylethyl ethyl group, N-phthalimidyl group, a trimethylsilylethyl oxycarbonyl group, 2-nitro-4,5-dimethoxy benzyl group, 2-nitro-4,5-dimethoxy benzyloxycarbonyl group, carbamate group and the like. A protecting group can be used for protecting a reactive functional group such as, for example, amino group, carboxyl group and the like. Various protecting groups can be used properly depending on conditions or purposes of a reaction. Acetyl group, benzyl group, silyl group, or derivatives thereof can be used as a protecting group for a hydroxyl group. In addition to an acetyl group, benzyloxycarbonyl group, t-buthoxycarbonyl group or derivatives thereof can be used as a protecting group for an amino group. As a protecting group for an aminooxy group and N-alkylaminoxy group, a trimethylsilylethyl oxycarbonyl group, 2-nitro-4,5-dimethoxy benzyloxycarbonyl group or derivatives thereof are preferable.

In the methods of the present invention, intended products may be isolated by removing foreign substances (unreacted raw material, by-product, solvent and the like) from a reaction solution using a method commonly used in the field of art (for example, extraction, distillation, washing, concentration, precipitation, filtration, drying or the like), and then combining after treatment methods commonly used in the field of art (for example, adsorption, dissolution, elution, distillation, precipitation, deposition, chromatography, or the like).

### (General techniques used in the present specification)

The techniques used in the present specification are, unless otherwise noted specifically, well-known commonly used techniques in organic chemistry, biochemistry, genetic engineering, molecular biology, microbiology, genetics and related fields within the technical range of the field of art. Such techniques are sufficiently disclosed in, for example, documents which will be listed below and documents cited in other parts of the present specification.

Molecular biological methods, biochemical methods, microbiological methods used in the present specification are those well-known and commonly used in the art, and are disclosed in, for example: Maniatis, T. et al. (1989) Molecular Cloning: A Laboratory Manual, Cold Spring Harbor and 3rdEd. Thereof (2001) ; Ausubel, F.M., et al. eds, Current Protocols in Molecular Biology, John Wiley & Sons Inc., NY, 10158(2000); Innis, M.A. (1990). PCR Protocols: A Guide to Methods and Applications, Academic Press; Innis, M.A. et al. (1995) PCR Strategies, Academic Press; Sninsky, J.J. et al. (1999) PCR Applications: Protocols for Functional Genomics, Academic Press; Gait, M.J. (1985) Oligonucleotide Synthesis: A Practical Approach, IRL Press; Gait, M. J. (1990) Oligonucleotide Synthesis: A Practical Approach, IRL Press; Eckstein, F. (1991) Oligonucleotides and Analogues: A Practical Approach, IRL Press; Adams, R.L. et al. (1992) The Biochemistry of the Nucleic Acids, Chapman & Hall; Shabarova, Z. et al. (1994) Advanced Organic Chemistry of NucleicAcids, Weinheim; Blackburn, G.M. etal. (1996) Nucleic Acids in Chemistry and Biology, Oxford University Press; Hermanson, G.T. (1996) Bioconjugate Techniques, Academic Press; Method in Enzymology 230, 242, 247, Academic Press, 1994; Bessatsu Jikkenn Igaku (Separate volume of Experimental Medicine) "Idenshidonyu & Hatsugen Kaiseki Jikkenho (Gene Introduction & Expression Analysis Experimental Method)", Yodosha Co. Ltd., 1997; Hatanaka, Nishimura, et.al., "Toshitsu no Kagaku to Kogaku (Science and engineering of Glucids)", Kodansha Scientific KK, 1997; Tosabunshi no Sekkei to Seirikino (Design and Physiology of Sugar Chain Molecules) , Chemical Society of Japan ed., Japan Scientific Societies Press, 2001; and the like. The relevant portions (these may be the entirety) of these documents are herein incorporated.

### (Description of preferable embodiments)

Hereinafter, preferable embodiments of the present invention will be described. It is appreciated that the embodiments as described below are provided only for better understanding of the present invention, and that the scope of the present invention should not be limited to the following description. Therefore, it is apparent that those skilled in the art can properly modify the invention within the scope of the present invention by considering the description in the present specification.

In one aspect, the present invention provides a method for performing mass spectrometry of sulfur atom-containing derivatives of an organic residue including ionizing a metal-organic residue complex into the derivatives, wherein the complex has the organic residue bound through a sulfur atom to the metal. This method enables a highly-sensitive and accurate analysis of chemical reactions on the surface of a self-organized membrane bound to a metal.

A metal used in the present invention may be, but not limited to, a simple substance of gold, silver, cadmium or selenium, or for example, a fine complex particle containing a magnetic particle of iron, nickel, cobalt or iron oxide (Fe₃O₄) or the like and a layer of gold, silver, cadmium, selenium or the like coated thereon. When a fine complex particle having magnetism as described above is used, a sulfur atom-containing derivative of an organic residue is supported on the fine complex particle, and thereafter the fine particle is readily recovered, thereby performing mass spectrometry without purification. The form of the metal used in the present invention is not particularly limited, and may be a metal substrate for mass spectrometry or a fine metal particle capable of forming colloid. The metal preferably has a surface enough to cause a diffuse reflection of a laser beam. Thus, the sensitivity for mass spectrometry is enhanced, and in spite of the presence of a contaminant (buffer or salt), the possibility that such a contaminant affects mass spectrometry measurement is decreased. From a viewpoint of efficiency in ionization of a thiol compound bound to a gold surface by LDI-TOF Mass, the most preferable form of the metal used in the present invention may be a fine metal particle. A sugar chain or various functional groups can be provided on a surface of the fine metal particle, using the bond between a thiol group and the metal. Moreover, solubility of the fine metal particle can be freely controlled. Therefore, a fine metal particle coated with a thiol compound can be used as a solid phase carrier not only for reaction in an aqueous solvent but also for solid phase synthesis in an organic solvent. While a fine particle is suspended in a reaction solvent, it can be separated from solution by centrifugal filtration, and thus separate purification can be performed very readily, which is an advantage. Further, progress of a reaction can be directly tracked by mass spectrometry methods, thereby achieving efficiency. According to the present invention, a fine metal particle containing an organic residue without cytotoxicity bound through a sulfur atom to the fine metal particle is provided, thereby enabling direct introduction of a metal-organic residue complex particle into a living cell, and accurate analysis of various chemical reactions (for example, enzymatic reaction) in the cell by performing mass spectrometry of a reaction product. A fine gold particle or a fine silver particle, which is a typical example of a fine metal particle, can be produced respectively by reducing tetrachloroauric acid and AgNO₃ in the presence of a reducing agent. Examples of a preferable reducing agent include, but not limited to, sodium borohydride or sodium citrate. By using these metals, a regular self-organized membrane of a sulfur atom-containing derivative of an organic residue can be formed on a surface of the fine metal particle.

In a preferred embodiment, the mass spectrometry of the present invention is carried out by an LDI-TOF MS method. In the present invention, a matrix reagent which has been used in conventional MALDI-TOF MS methods are not particularly required. In conventional MALDI-TOF MS methods, it has been considered as a problem that a peak of a substance of interest cannot be observed well in the"low molecular weight regions where a candidate may often shows a peak", due to an intense peak of the matrix per se. However, the present invention has an advantage that matrix-free LDI-TOF MS can be carried out with a high sensitivity and accuracy, by making a low molecular weight compound of interest supported on a metal substrate (in particular, a fine metal particle) as in the present invention. When using a matrix reagent in the present invention, examples of a preferable reagent include, but not limited to, 2,5-dihydroxybenzoic acid, a-cyano-4-hydroxy-cinnamic acid, sinapic acid, t-rans-3-indole-acrylic acid, 1,5-diamino-naphthalene, 3-amino-4-hydroxybenzoic acid, 9-nitro-anthracene, 2-picolinic acid, 3-hydroxy-picolinic acid, nicotinic acid, anthranilic acid, 5-chloro-salicylic acid, 2'-(4-hydroxyphenyl azo) benzoic acid, dithranol, and 3-amino-quinoline. The most preferable matrix reagent is 2,5-dihydroxybenzoic acid.

In a preferred embodiment, the above organic residue includes a sugar chain or a sugar chain-containing substance.

In another aspect, the present invention provides: a method for performing mass spectrometry of a compound or salt thereof, characterized in that the method includes ionizing a metal-organic residue complex into sulfur atom-containing derivatives,
wherein the metal-organic residue complex is represented by the general formula (I)

(R-S)ₙ-M¹ (I),

wherein R is an organic residue, S is a sulfur atom and n indicates a stoichiometric ratio of (R-S) group with respect to M¹ and is an integer equal to or greater than 1; and
wherein the compound is represented by the general formulae (II) and/or (III):

R-SH (II)

and/or

R-S-S-R (III),

wherein R and S are the same as defined above.

In yet another aspect, the present invention provides: a method for performing mass spectrometry of a compound or salt thereof, characterized in that the method includes ionizing a metal-organic residue complex into sulfur atom-containing derivatives,
wherein the metal-organic residue complex is represented by the general formula (IV):

M¹-S-X-CH(R)-S-M¹ (IV),

wherein R is an organic residue, S is a sulfur atom, M¹ at both ends are same metal entities, X is a lower alkylene or a lower alkenylene;
wherein the compound is represented by the general formulae (V) and/or (VI):

HS-X-CH(R)-SH (V)

and/or 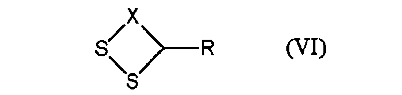 wherein R, S and X are the same as defined above.

In still another aspect, the present invention provides: a method for performing mass spectrometry of a sugar chain or a sugar chain-containing substance, comprising the following steps of:
1) contacting a metal-organic residue complex with a sugar chain or a sugar chain-containing substance under the conditions where the metal-organic residue complex and the sugar chain or sugar chain-containing substance may react with each other, wherein the metal-organic residue complex contains a metal bound to a group represented by the following formula (herein, the following group of formulae is defined as [group X]):
   -S-Y-(OCH₂CH₂)ₙ-NH-C(=O)-CH₂-O-NH₂,
   -S-Y-(OCH₂CH₂)ₙ-NH-C(=O)-CH-O-NH (CH₃),
   -S-Y-(OCH₂CH₂)ₙ-NH-C(=O)-CH (NH₂) -W⁴-SH,
   -S-Y- (OCH₂CH₂)ₙ-NH-C(=S)-CH(NH₂)-W⁴-SH,
   -S-W¹-O-NH₂,
   -S-W¹-O-NH(CH₃),
   -S-W¹-O-W²-O-NH₂,
   -S-W¹-O-W²-O-NH(CH₃),
   -S- (CH₂CH₂O)ₙ-W¹-O-W²-O-NH₂,
   -S- (CH₂CH₂O)ₙ-W¹-O-W²-O-NH(CH₃),
   -S-W¹-C(=O)-NH-NH₂,
   -S-W¹-C(=S)-NH-NH₂,
   -S-W¹-NH-C(=O)-CH(NH₂)-W⁴-SH,
   -S-W¹-NH-C(=S)-CH (NH₂)-W'-SH
   -S-Z¹-Z²-Z³-Z⁴-Z⁵-O-NH₂,
   -S-Z¹-Z²-Z³-Z⁴-Z⁵-O-NH(CH₃),
   -S-Z¹-Z²-Z³-Z⁴-CH(NH₂)-O-Z⁶-SH,
   -S-Z¹-Z²-Z³-Z⁴-CH(NH₂)-Z⁶-SH,
   -S-Z¹-O-Z³-CH(NH₂)-Z⁶-SH,
   -S-Z¹-O-Z³-O-NH₂,
   -S-Z¹-O-Z³-O-NH(CH₃),
   -S-Z¹-O-Z³-Z⁴-Z⁵-O-NH₂,
   -S-Z¹-O-Z³-Z⁴-Z⁵-O-NH(CH₃),
   -S-Z¹-O-Z³-Z⁴-CH(NH₂)-O-Z⁶-SH
   -S-Z¹-O-Z³-Z⁴-CH(NH₂)-Z⁶-SH
   -S-Z¹-Z³-Z⁴-Z⁵-O-NH₂,
   -S-Z¹-Z³-Z⁴-Z⁵-O-NH(CH₃),
   -S-Z¹-Z³-Z⁴-CH(NH₂)-O-Z⁶-SH,
   -S-Z¹-Z³-Z⁴-CH(NH₂) -Z⁶-SH,
   or 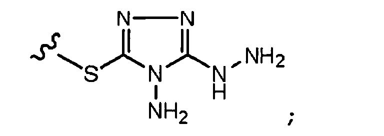 wherein Y, W¹ and W² are independently C1-C12 alkylene, C2-C12 alkenylene or C2-C12 alkynylene;
   W⁴ is C1-C2 alkylene;
   Z¹ is substituted or unsubstituted arylen or heteroarylen;
   Z² is a nitrogen-containing heterocycle;
   Z³ and Z⁵ are independently C1-C12 alkylene;
   Z⁴ is -O-C(=O), -O-C(=S), -NH-C(=O), -NH-C (=S) , -O- or -S-;
   Z⁶ is C1-C2 alkylene and
   n is an integer between 1 and 10, inclusive;
2) obtaining the metal-organic residue complex bound to the sugar chain or the sugar chain-containing substance; and
3) ionizing the metal-organic residue complex bound to the sugar chain or the sugar chain-containing substance into sulfur atom-containing derivatives of the organic residue.

In the above step 1) of the present invention, the method may preferably include the step of liberating an aldehyde group in the above "sugar chain or sugar chain-containing substance", prior to contacting a metal-organic residue complex and "sugar chain or sugar chain-containing substance. This is because a substance capable of specifically interacting with a sugar chain of the present invention can interact advantageously, for example, in a case where an aldehyde group of the sugar chain is protected. Such a step of liberating an aldehyde group preferably includes a proton-donating reaction by an enzyme treatment and/or a chemical method. The enzyme treatment may be, for example, a treatment by glycosidase (for example, in the case of galactose, an aldehyde group is obtained by galactoxydase treatment), and the treatment by a chemical method may be hydrazinolysis. In the method of the present invention, the enzyme treatment and the chemical method can be used separately or in combination. One type of enzyme or a plurality of types of enzymes may be used. In the case of liberating aldehyde from sialic acid contained in a glycoprotein sugar chain (for example, open the sequence between 8 and 9 sites of sialic acid), an oxidant such as sodium periodate (NaIO₄) is used.

In one embodiment, the above organic residue is a group including a sugar chain or a sugar chain-containing substance.

In one aspect, the present invention provides: a method for performing mass spectrometry of a sulfur atom-containing analyte, characterized in that the method includes ionizing a metal-analyte complex wherein the metal is bound through a sulfur atom to the analyte to become a sulfur atom-containing analyte.

In another aspect, the present invention provides: a method for performing mass spectrometry of a sulfur atom-containing analyte including the steps of:
1) reacting tetrachloroauric acid with a sulfur atom-containing analyte in the presence of a reducing agent;
2) obtaining a gold-analyte complex particle which has the analyte bound through the sulfur atom to the gold; and
3) ionizing the obtained gold-analyte complex particles into a sulfur atom-containing analyte derivative.

As a reducing agent used in the above step 1) of the present invention, sodium borohydride or sodium citrate is preferably used, but the reducing agent is not limited to these.

In a preferred embodiment, the above step 1) is carried out in a solvent selected from the group consisting of water, alcoholic solvent, ethyl acetate, ethereal solvent or a mixture solvent thereof. Examples of a preferable alcoholic solvent include methanol, ethanol, n-propanol, isopropanol, and butanol. Examples of a preferable ethereal solvent include dimethyl ether, diethyl ether, and tetrahydrofuran. The above step 1) is carried out at a temperature between -20°C and 200°C, preferably 0°C and 150°C, and more preferably 20°C and 100°C, inclusive. When a compound is stable, the step is preferably carried out at a temperature from room temperature to the reflux temperature of the solvent. The above step 1) is carried out for 0.1 hour to 24 hours, preferably 1 hour to 12 hours.

In another preferred embodiment, a metal-analyte complex particle wherein an analyte is bound through a sulfur atom to a metal, obtained in the above step 2), is purified by centrifugal filtration. However, even if a metal-analyte complex particle is subjected to mass spectrometry without performing centrifugal filtration, the metal-analyte complex particle has a particular advantage in that the sensitivity does not decrease.

In the above step 1), when the reaction occurs in the presence of iron (for example, FeCl₃), the gold-analyte complex particle becomes magnetic, and thus the step 2) is carried out using a magnet. Further, by contacting a magnetic metal-analyte complex particle having a portion of "analyte" a compound used as a substrate for an enzymatic reaction with a living cell, homogenizing the cell after reaction and recovering with a magnet to perform mass spectrometry, various enzymatic reactions occurring in a cell can be analyzed.

When the metal is silver, the method can be carried out using silver nitrate (AgNO₃) instead of tetrachloroauric acid, in the above step 1).

In one aspect, the present invention provides: a method for confirming development of chemical reaction or enzymatic reaction, characterized in that the method includes providing the metal-analyte complex particle obtained in the step 2) to a chemical reaction or enzymatic reaction, and ionizing the metal-analyte complex particle in a reaction system into a sulfur atom-containing analyte.

A preferable example of the above enzymatic reaction is, but not limited to, a sugar chain elongation reaction.

In a preferred embodiment, development of the sugar chain elongation reaction is confirmed using mass spectrometry, thereby confirming the activity of a glycosyltransferase.

In one aspect, the present invention provides: a metal-organic residue complex wherein a metal is bound to a group containing: a) a group selected from the group consisting of protected or unprotected aminooxy group, protected or unprotected N-alkylaminooxy group, hydrazid group, azide group, thiosemicarbazide group, cysteine residue, and derivatives thereof; and b) a sulfur atom.

In another aspect, the present invention provides:
a method for producing a gold-organic residue complex particle including the step of reacting tetrachloroauric acid with a compound containing: a) a group selected from the group consisting of protected or unprotected aminooxy group, protected or unprotected N-alkylaminooxy group, hydrazid group, azide group, thiosemicarbazide group, cysteine residue, and derivatives thereof; and b) a thiol group or disulfide group, or salt thereof in the presence of a reducing agent.

By these methods, a functional group which interacts with a sugar chain or a sugar chain-containing substance can be provided on a fine metal particle.

In one aspect, the present invention provides a metal-organic residue complex, wherein the metal organic residue contains a metal bound to a group represented by the formula in the above [group X]. This metal-organic residue complex can be also represented by the following formula (herein, the following group of formulae is defined as [group Y]):
M²-(S-Y-(OCH₂CH₂)ₙ-NH-C(=O)-CH₂-O-NH₂)ₘ,
M²- (S-Y-(OCH₂CH₂)ₙ-NH-C(=O)-CH₂-O-NH(CH₃))ₘ,
M²-(S-Y-(OCH₂CH₂)ₙ-NH-C(=O)-CH(NH₂)-W⁴-SH)ₘ,
M²-(S-Y-(OCH₂CH₂)ₙ-NH-C(=S)-CH(NH₂)-W⁴-SH)ₘ,
M²-(S-W¹-O-NH₂)ₘ,
M²-(S-W¹-O-NH(CH₃))ₘ,
M²-(S-W¹-O-W²-O-NH₂)ₘ,
M²-(S-W¹-O-W²-O-NH(CH₃))ₘ,
M²-(S- (CH₂CH₂O)ₙ-W¹-O-W²-O-NH₂)ₘ,
M²-(S-(CH₂CH₂O)ₙ-W¹-O-W²-O-NH(CH₃))m,
M²-(S-W¹-C(=O)-NH-NH₂)ₘ,
M²-(S-W¹-C(=S) -NH-NH₂)ₘ,
M²-(S-W¹-NH-C(=O)-CH (NH₂)-W⁴-SH)ₘ,
M²-(S-W¹-NH-C(=S)-CH(NH₂)-W⁴-SH)ₘ,
M²-(S-Z¹-Z²-Z³-Z⁴-Z⁵-O-NH₂)ₘ,
M²-(S-Z¹-Z²-Z³-Z⁴-Z⁵-O-NH(CH₃))ₘ,
M²-(S-Z¹-Z²-Z³-Z⁴-CH(NH₂)-O-Z⁶-SH)ₘ,
M²-(S-Z¹-Z²-Z³-Z⁴-CH(NH₂)-Z⁶-SH)ₘ,
M²-(S-Z¹-O-Z³-CH(NH₂)-Z⁶-SH)ₘ,
M²-(S-Z¹-O-Z³-O-NH₂)ₘ,
M²-(S-Z¹-O-Z³-O-NH(CH₃))ₘ,
M²-(S-Z¹-O-Z³-Z⁴-Z⁵-O-NH₂)ₘ,
M²-(S-Z¹-O-Z³-Z⁴-Z⁵-O-NH(CH₃))ₘ,
M²-(S-Z¹-O-Z³-Z⁴-CH(NH₂)-O-Z⁶-SH)ₘ,
M²-(S-Z¹-O-Z³-Z⁴-CH(NH₂)-Z⁶-SH)ₘ,
M²-(S-Z¹-Z³-Z⁴-Z⁵-O-NH₂)ₘ,
M²-(S-Z¹-Z³-Z⁴-Z⁵-O-NH(CH₃))ₘ,
M²-(S-Z¹-Z³-Z⁴-CH(NH₂)-O-Z⁶-SH)ₘ,
M²-(S-Z¹-Z³-Z⁴-CH(NH₂)-Z⁶-SH)ₘ,
or the general formula (VII): 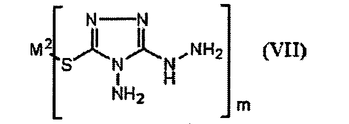 wherein, M² is a metal;
m indicates a stoichiometric ratio of an organic residue with respect to M² and is an integer equal to or greater than 1, wherein the organic residue contains a sulfur atom;
Y, W¹ and W² are independently C1-C12 alkylene, C2-C12 alkenylene or C2-C12 alkynylene;
W' is C1-C2 alkylene;
Z¹ is substituted or unsubstituted arylen or heteroarylen ;
Z² is a nitrogen-containing heterocycle;
Z³ and Z⁵ are independently C1-C12 alkylene;
Z⁴ is -O-C(=O), -O-C(=S), -NH-C(=O), -NH-C (=S) , -O- or -S-;
Z⁶ is C1-C2 alkylene; and
n is an integer between 1 and 10, inclusive.

An example of a preferable complex of the present invention is a complex represented by the general formula (VII): 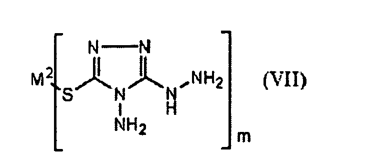 wherein, M² is a metal.

This complex can be represented by the following formula:

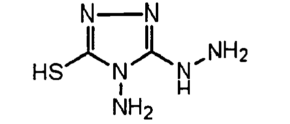

This complex is formed by reaction between the SH group of 4-amino-3-hydrazine-5-mercaptol,2,4-triazol (abbreviation: AHMT) with a metal to bind them to each other. This AHMT is known as a color reagent specifically reacting with formaldehyde or aldehyde (see, for example, patent document 2, 3 and 4, and non-patent document 2).

In another aspect, a method for producing metal-organic residue complex particles, wherein the method includes reacting tetrachloroauric acid with a compound represented by the following formula (herein, the following group of formulae is defined as [group Z]):
(̵S-Y-(OCH₂CH₂)ₙ-NH-C(=O)-CH₂-O-NH₂)₂,
(̵S-Y-(OCH₂CH₂)ₙ-NH-C(=O)-CH₂-O-NH(CH₃))₂,
(̵S-Y-(OCH₂CH₂)ₙ-NH-C(=O)-CH(NH₂)-W⁴-SH)₂,
(̵S-Y-(OCH₂CH₂)ₙ-NH-C(=S)-CH(NH₂)-W⁴-SH)₂,
(̵S-W¹-O-NH₂)₂,
(̵S-W¹-O-NH(CH₃))₂,
(̵S-W¹-O-W²-O-NH₂)₂,
(̵S-W¹-O-W²-O-NH(CH₃))₂,
(̵S-(CH₂CH₂O)ₙ-W¹-O-W²-O-NH₂)₂,
(̵S-(CH₂CH₂O)ₙ-W¹-O-W²-O-NH(CH₃))₂,
(̵S-W¹-C(=O)-NH-NH₂)₂,
(̵S-W¹-C(=S)-NH-NH₂)₂,
(̵S-W¹-NH-C(=O)-CH(NH₂)-W⁴-SH)₂,
(̵S-W¹-NH-C(=S)-CH(NH₂)-W⁴-SH)₂,
(̵S-Z¹-Z²-Z³-Z⁴-Z⁵-O-NH₂)₂,
(̵S-Z¹-Z²-Z³-Z⁴-Z⁵-O-NH(CH₃)₂,
(̵S-Z¹-Z²-Z³-Z⁴-CH(NH₂)-O-Z⁶-SH)₂,
(̵S-Z¹-Z²-Z³-Z⁴-CH(NH₂)-Z⁶-SH)₂,
(̵S-Z¹-O-Z³-CH(NH₂)-Z⁶-SH)₂,
(̵S-Z¹-O-Z³-O-NH₂)₂,
(̵S-Z¹-O-Z³-O-NH(CH₃))₂,
(̵S-Z¹-O-Z³-Z⁴-Z⁵-O-NH₂)₂,
(̵S-Z¹-O-Z³-Z⁴-Z⁵-O-NH(CH₃))₂,
(̵S-Z¹-O-Z³-Z⁴-CH(NH₂)-O-Z⁶-SH)₂,
(̵S-Z¹-O-Z³-Z⁴-CH(NH₂)-Z⁶-SH)₂,
(̵S-Z¹-Z³-Z⁴-Z⁵-O-NH₂)₂,
(̵S-Z¹-Z³-Z⁴-Z⁵-O-NH(CH₃))₂,
(̵S-Z¹-Z³-Z⁴-CH(NH₂)-O-Z⁶-SH)₂,
(̵S-Z¹-Z³-Z⁴-CH(NH₂)-Z⁶-SH)₂
or 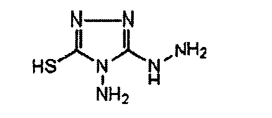 ,or a salt thereof, in the presence of a reducing agent,
wherein, Y, W¹ and W² are independently C1-C12 alkylene, C2-C12 alkenylene or C2-C12 alkynylene;
W⁴ is C1-C2 alkylene;
Z¹ is substituted or unsubstituted arylen or heteroarylen; Z² is a nitrogen-containing heterocycle;
Z³ and Z⁵ are independently C1-C12 alkylene;
Z⁴ is -O-C(=O), -O-C(=S), -NH-C(=O), -NH-C (=S) , -O- or -S-;
Z⁶ is C1-C2 alkylene; and
n is an integer between 1 and 10, inclusive.

According to another aspect, a method for trapping a sugar chain or a sugar chain-containing substance characterized in that the method includes contacting the metal-organic residue complex and a sugar chain or a sugar chain-containing substance under conditions where the metal-organic residue complex and the sugar chain or the sugar chain-containing substance may react with each other is provided.

According to yet another aspect, a method for measuring the molecular weight of a substance which may interact with an organic residue of a metal-organic residue complex, includes the steps of:
1) contacting the metal-organic residue complex with a substance which may interact with the organic residue, wherein the metal is bound through a sulfur atom to an organic residue;
2) obtaining the metal-organic residue complex bound to the substance which may interact; and
3) ionizing the obtained metal-organic residue complex into derivatives of the organic residue, wherein the organic residue contains a sulfur atom is provided.

In one aspect, the method for performing mass spectrometry of a sugar chain or a sugar chain-containing substance of the present invention may be a method including the steps of:
1) contacting a compound with a metal, wherein the compound is represented by the following formula: 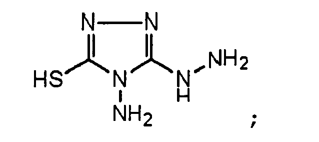
2) contacting the metal-organic residue complex obtained in 1) with a sugar chain or a sugar chain-containing substance under conditions where the metal-organic residue complex and the sugar chain or the sugar chain-containing substance may react with each other; and
3) ionizing the metal-organic residue complex obtained in 2) into derivatives of the organic residue, wherein the organic residue contains a sulfur atom, or
   a method including the steps of:
   1) contacting a compound represented by the following formula: 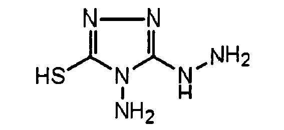 with a sugar chain or a sugar chain-containing substance under conditions where the compound and the sugar chain or the sugar chain-containing substance may react with each other;
   2) contacting the compound obtained in 1) with a metal; and
   3) ionizing the metal-organic residue complex obtained in 2) into derivatives of the organic residue, wherein the organic residue contains a sulfur atom.

In one aspect, the present invention provides: a composition for trapping a sugar chain, including:
a compound represented by the general formula (II):
   R-SH (II) or a salt thereof, wherein R is an organic residue; and S is a sulfur atom;
   a compound represented by the general formula (III):
      R-S-S-R (III) or a salt thereof, wherein, R and S are the same as defined above;
   a compound represented by the general formula (V):
      HS-X-CH(R)-SH (V) or a salt thereof, wherein R and S are the same as defined above; and X is lower alkylene or lower alkenylene; or
   a compound represented by the general formula (VI): 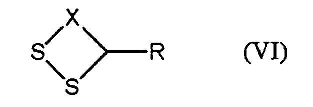 or a salt thereof, wherein, R, S and X are the same as defined above; or a mixture thereof.

In a preferred embodiment, the composition for trapping a sugar chain of the present invention contains compounds represented by the formulae in the above [group Z] .

In one aspect, the present invention provides: a composition for trapping a sugar chain, including: a metal-organic residue complex represented by the general formula (I):

(R-S)ₙ-M¹ (I),

wherein R is an organic residue; S is a sulfur atom; M¹ is a metal; and n indicates a stoichiometric ratio of (R-S) group with respect to M¹ and is an integer equal to or greater than 1; or
a metal-organic residue complex represented by the general formula (IV):

M¹-S-X-CH(R)-S-M¹ (IV),

wherein R and S are the same as defined above, M¹ at both ends are a metal of the same substance and X is lower alkylene or lower alkenylene.

In a preferred embodiment, the composition for trapping a sugar chain contains a metal-organic residue complex represented by the formula in the above [group Y].

In one aspect, the present invention provides: a kit for mass spectrometry of a sugar chain or a sugar chain-containing substance, including:
A) a compound represented by the general formula (II):

   R-SH (II)

   or a salt thereof, wherein R is an organic residue; and S is a sulfur atom;
   a compound represented by the general formula (III)

   R-S-S-R (III)

   or a salt thereof, wherein R and S are the same as defined above;
   a compound represented by the general formula (V):

   HS-X-CH(R)-SH (V)

   or a salt thereof, wherein R and S are the same as defined above; and X is lower alkylene or lower alkenylene; or
   a compound represented by the general formula (VI): 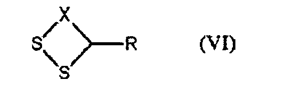 or a salt thereof, wherein R, S and X are the same as defined above; or a mixture thereof; and
B) a metal.

In a preferred embodiment, the kit for mass spectrometry of a sugar chain or a sugar chain-containing substance of the present invention contains:
A) a sulfur atom-containing derivative of an organic residue; and
B) a metal.

In a more preferred embodiment, the above metal is tetrachloroauric acid, and the kit for mass spectrometry of the present invention further contains C) a reducing agent.

In another aspect, the present invention provides: a kit for mass spectrometry of a sugar chain or a sugar chain-containing substance, including:
a metal-organic residue complex represented by the general formula (I):

   (R-S)ₙ-M¹ (I)

   wherein, R is an organic residue, S is a sulfur atom, M¹ is a metal and n indicates a stoichiometric ratio of (R-S) group with respect to M¹ and is an integer equal to or greater than 1; or
   a metal-organic residue complex represented by the general formula (IV):

   M¹-S-X-CH(R)-S-M¹ (IV)

   wherein R and S are the same as defined above, M¹ at both ends are same metal entities and X is lower alkylene or lower alkenylene.

In a preferred embodiment, the kit for mass spectrometry of a sugar chain or a sugar chain-containing substance of the present invention contains a metal-organic residue complex represented by the formula in the above [group Y].

In a more preferred embodiment, the above metal is a fine metal particle.

In a further more preferred embodiment, the above metal is a fine gold particle.

In a preferred embodiment, examples of a preferable combination of "organic residue" and "substance which may interact with an organic residue" include sugar chain-trapping functional group/sugar chain, sugar chain/protein, antigen/antibody, and the like.

References such as scientific documents, patents, and patent applications cited in the present specification are hereby incorporated by as if the entirety thereof are specifically described in the present specification.

The present invention has been illustrated with reference to the preferred embodiments of the present invention. However, the present invention should not be construed to be limited to such embodiments. It is noted that the scope of the present invention should be construed only by the scope of the claims. It is understood that those skilled in the art can carry out equivalent scope from the disclosure of the specific preferred embodiments of the present invention and based on the common technical knowledge. It is noted that patents, patent applications and documents cited in the present specification are herein incorporated by reference as if the contents themselves are specifically described in the present specification.

### EXAMPLES

### EXAMPLE 1 : Production of water-soluble fine gold particles and MALDI TOF mass spectrometry for direct detection of a thiol compound supported on a surface of the particle

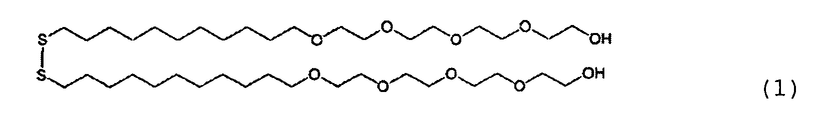

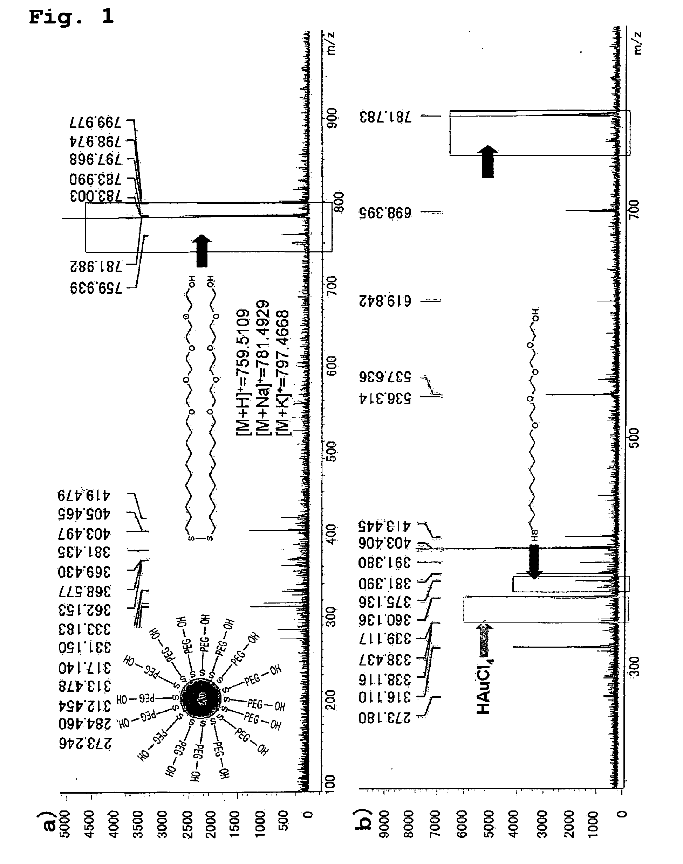

25 mg of tetrachloroauric acid was dissolved in 1 mL of ultrapure water in a flask. 55 mg of ligand (1) was dissolved in 20 mL of methanol and was then added to the tetrachloroauric acid aqueous solution. 56 mg of Sodium borohydride dissolved in 5 mL of ultrapure water was gradually added into the solution with vigorous agitation. After agitation at room temperature for 2 hours, the reaction solution was subjected to centrifugal filtration (20°C; 3,500 rpm) with Centriplus YM50 (Millipore), thereby obtaining a fine gold particle which had ligand (1) introduced on the surface thereof. The fine particles were soluble in water, acetone, and methanol. The fine particles and the filtrate, obtained in the centrifugal filtration process, were subjected to MALDI-TOF Mass spectrometry measurement (respectively, Figure 1 a and b). 2,5-dihydroxybenzoic acid (DHB) was used as a matrix. Observed peaks in Figure 1 indicates that the ligand (1) underwent ionaization or cleavage from the fine gold particle complex.

### EXAMPLE 2:MALDI TOF mass spectrometry for tracking of a chemical reaction using fine gold particles as a solid phase carrier

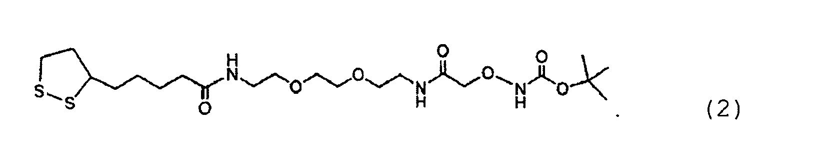

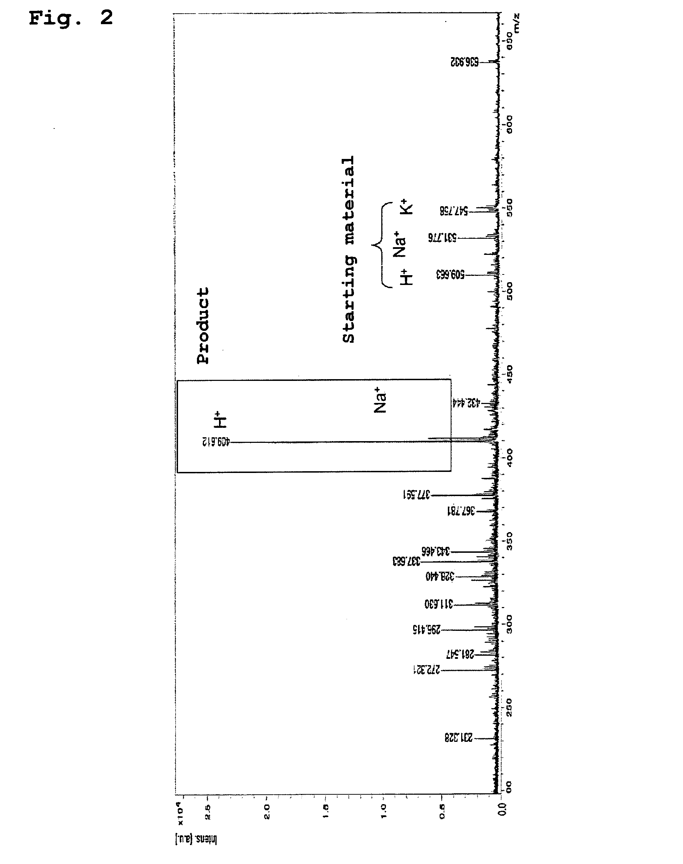

Methanol solution of compound (2) which contained disulfide, was added to a tetrachloroauric acid aqueous solution, and a sodium borohydride aqueous solution was gradually dropped thereto. The solution was stirred at room temperature for 3 hours, and then was subj ected to centrifugal filtration using Centriplus YM-50, thereby obtaining fine gold particles which had compound (2) introduced thereon. The fine particles were dissolved in methanol, with trifluoroacetic acid supplemented thereto up to a concentration of 25%. The solution was agitated at room temperature for 2 hours. Thereafter, a part of the reaction system wassubjected to centrifugal filtration using Microcon. Development of reaction was confirmed by MALDI-TOF mass spectrometry measurement using DHB as a reagent (Figure **2).** Observed peaks in Figure **2** indicates that the product formed through the elimination of a protecting group.

### EXAMPLE 3 : Production of a sugar chain-fine gold particle complex and MALDI-TOF Mass spectrometry for direct measurement of a molecular weight of a ligand

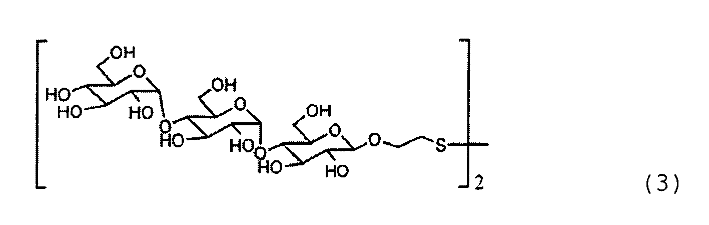

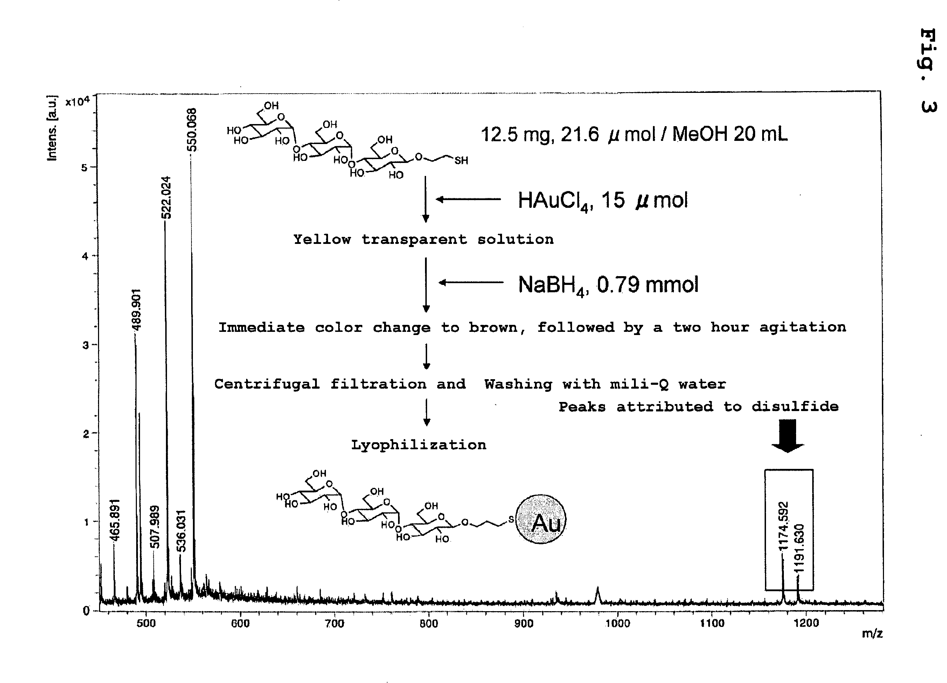

Maltotriose derivative (3) having a thiol group at the aglycone terminus (12.5 mg, 21.6 µmol) was dissolved in 20 mL of methanol, and 15 µL of tetrachloroauric acid was added thereto. Sodium borohydride aqueous solution (30 mg/2 mL, 0.79 mmol) was gradually added to the solution. The solution was stirred at room temperature for 3 hours, and then was subjected to centrifugal filtration using CentriplusYM-50, thereby purifying the fine particles. The fine metal particles were dissolved in ultrapure water, and then subjected to MALDI-TOF mass spectrometry using 2, 5-dihydroxybenzoic acid (DHB) as a matrix (Figure **3).** Figure **3** shows that observed peaks were attributed to the disulfide 3, indicating that mass of a ligand can be readily detected by MALDI-TOF Mass spectroscopy in the case of a fine gold particle modified with a sugar chain ligand.

### EXAMPLE 4 : Production of a sugar chain (GlcNAc) -fine gold particle complex in which the sugar chain density is controlled

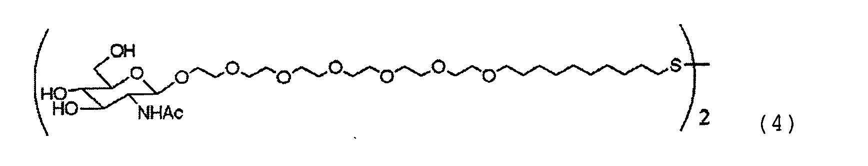

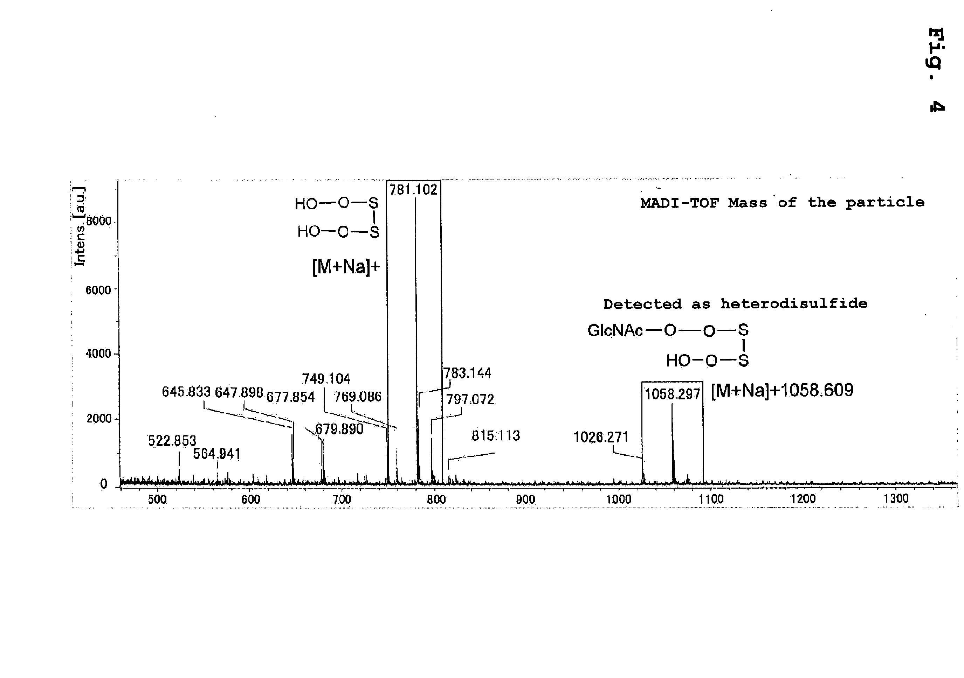

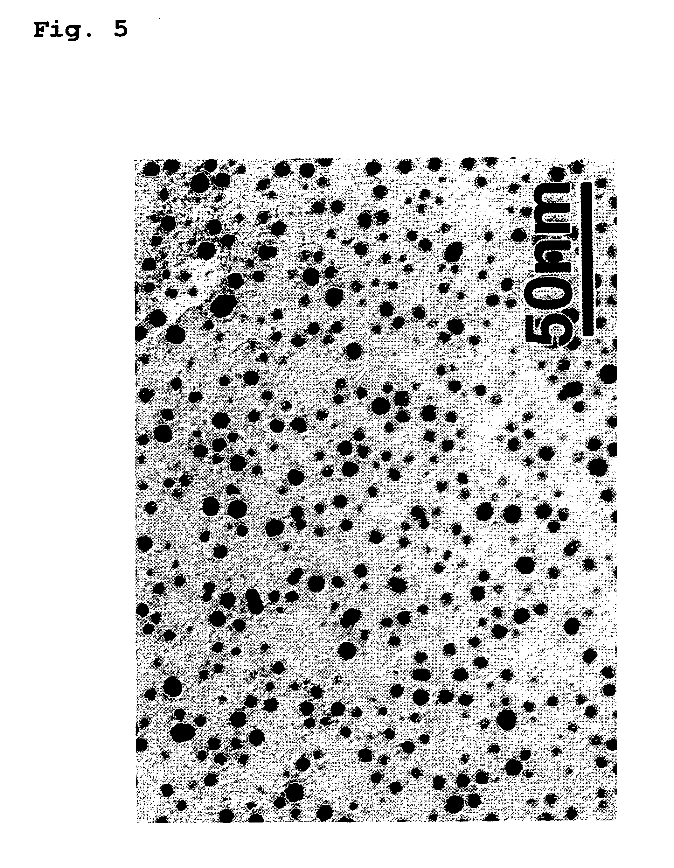

Compound (4) having N-acetylglucosamine (10 mg, 7.6 µmol) at its terminal, and compound (1) (52 mg, 69 µmol) were dissolved in a mixture solvent of methanol (35 mL) and pure water (5 mL), and tetrachloroauric acid (25.5 mg, 75 µmol) was added thereto. Sodium borohydride aqueous solution (70 mg / 5 mL) was gradually added to the solution. The solution was agitated at room temperature for 12 hours, and then was subjected to centrifugal filtration using Centriplus YM-50, thereby purifying a fine particle. The fine metal particles were dissolved in pure water, and then subjected to MALDI-TOF mass spectrometry using 2,5-dihydroxybenzoic acid (DHB) as a matrix (Figure **4).** Figure **4** shows that molecular weight peaks were observed, which correspond to homodisulfide and heterodisulfide of compounds (1) and (4). Here, Figure **5** shows the electron micrographs (TEM) for the fine metal particles purified by centrifugal separation.

### EXAMPLE 5 : Sugar chain elongation reaction by galactosyltransferase (GalT) on sugar chain (GlcNAc)-fine metal particle

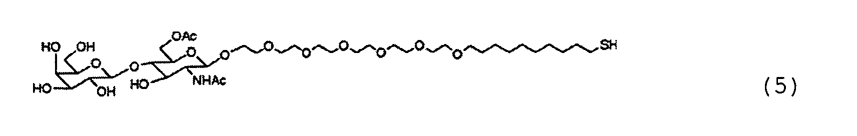

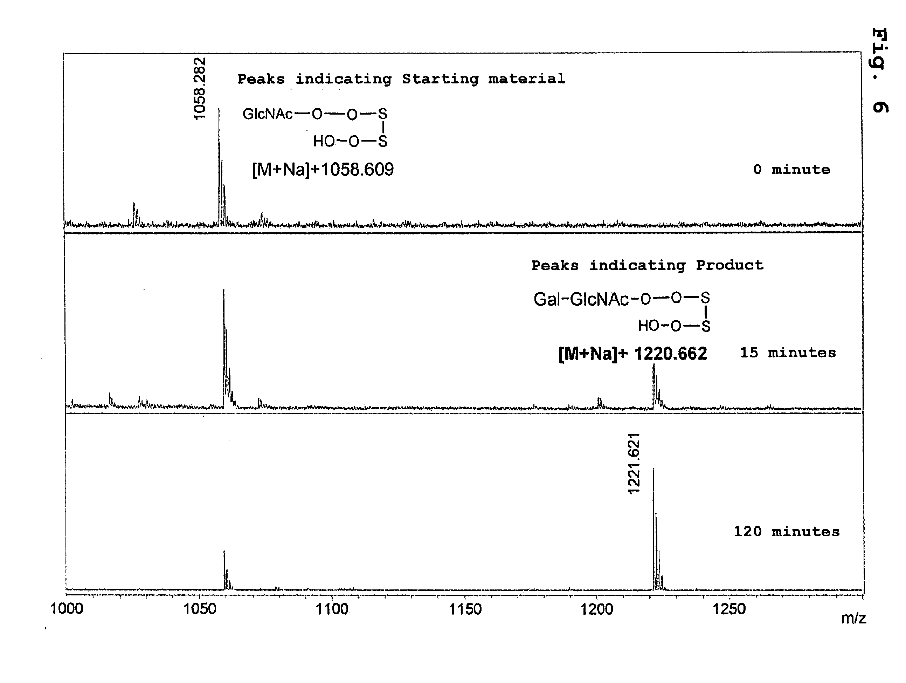

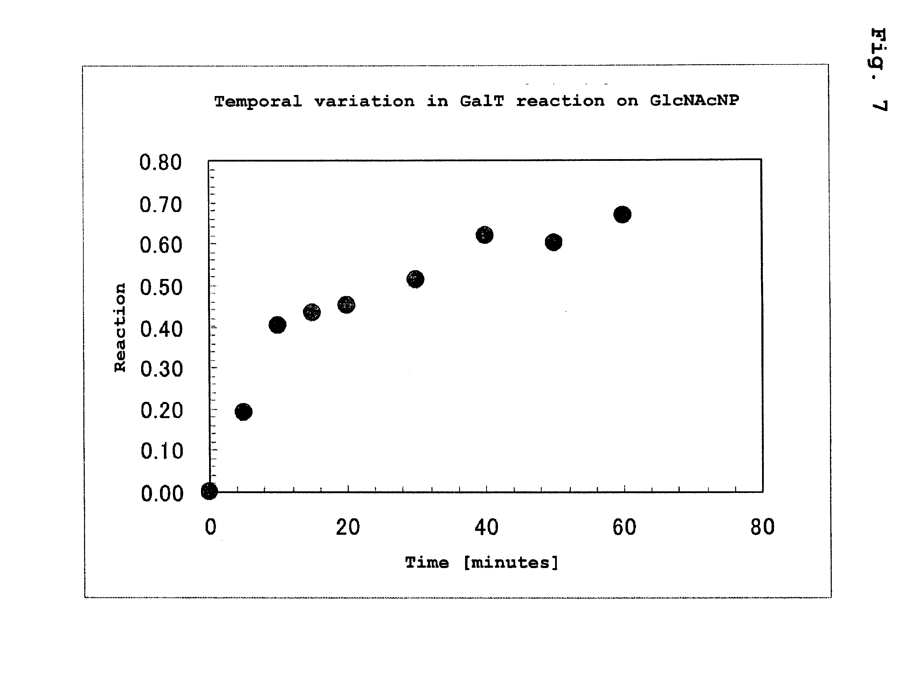

100 µL of MHEPES buffer solution (10 mM MnCl₂, 0.15 M NaCl) of the sugar chain-fine gold particles (GlcNAc-Au: 50 µM) produced in accordance with Example 4 and UDP-Gal (400 µM), was prepared. Four mU of β1-4 galactosyltransferase (GalT) was added to the buffer solution, and the sugar chain elongation reaction was tracked at 25°C by using MALDI-TOF Mass spectroscopy. The reaction solution was not subj ected to centrifugal filtration, but to MALDI-TOF mass spectrometry using 2, 5-dihydroxybenzoic acid (DHB) as a matrix (Figure **6).** Figure **6** shows that although a peak attributed to compound (4) (starting substance) disappeared, another molecular weight peaks were observed at [M+Na]+1220.662, and attributed to a heterodisulfide comprising compound 5 (product) and compound (1) where the compound 5 had a galactose added thereto through the enzymatic reaction. It demonstrated that the enzymatic reaction could be tracked on the sugar chain-fine gold particle complex. Figure **7** indicates a plot representing temporal variation in extent of the sugar-chain elongation reaction, which was derived from the spectrum shown in Figure **6.** This plot has revealed that GalT reaction on GlcNAc-Au complex could be tracked successfully by MALDI-TOF Mass spectrometry.

### EXAMPLE 6: Measurement of inhibiting activity of UDP against GalT reaction on a sugar chain (GlcNAc)-fine gold particle complex

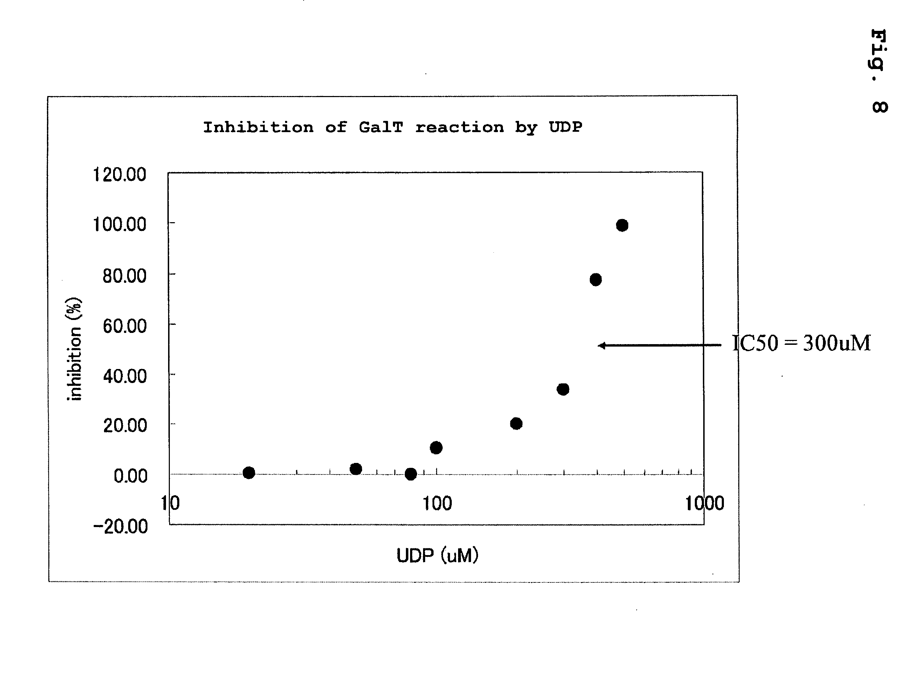

100 µL of MHEPES buffer solution (10 mM MnCl₂, 0.15 M NaCl) of the sugar chain-fine gold particles (GlcNAc-Au:50 µM) produced in accordance with Example 4 and UDP-Gal (400 µM), was prepared. Four mU of β1-4 galactosyltransferase (GalT) and UDP (prepared at nine concentration levels in the range of 0-50 µM) was then added to the buffer solution, and the solution was subjected to reaction at 25°C for 60 minutes. The relationship between UDP concentration (µM) and inhibiting activity (%) is shown in Figure **8.** This plot has revealed that IC50 of UDP is 300µM.

### EXAMPLE 7: Production of a fine gold particle having sugar chain trapping ability

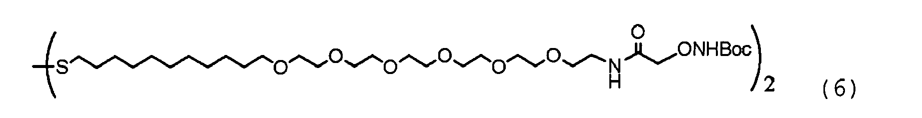

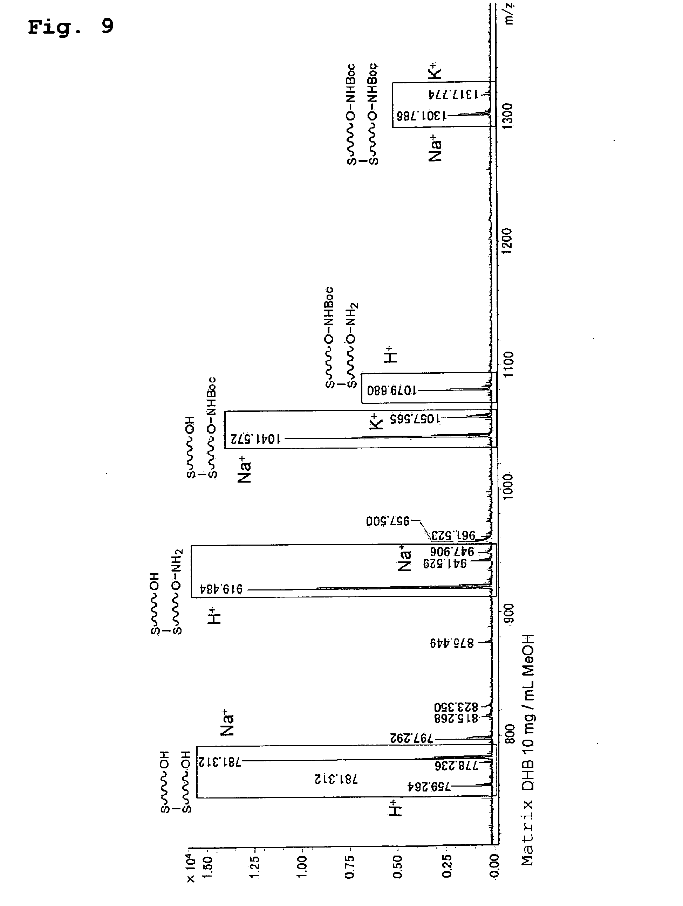

A thiol compound (6) having Boc-protected oxyamino group as a sugar chain-trapping functional group at a terminus (22 mg, 17 µmol) and a compound (1) (54 mg, 71 µmol) were dissolved in 20 mL of methanol, and tetrachloroauric acid (34 mg, 100 µmol) was added thereto. Sodium borohydride aqueous solution (31 mg / 3 mL) was gradually added to the solution. The solution was agitated at room temperature for 12 hours. The reaction solution was then subjected to centrifugal filtration using Centriplus YM-50, thereby purifying a fine particle (100,000 > molecular weight > 50,000). The fine metal particles were dissolved in pure water, and then subjected to MALDI-TOF Mass spectrometry by using 2,5-dihydroxybenzoic acid (DHB) as a matrix (Figure 9). According to Figure 6, peaks were observed, and attributed to a disulfide of compound (6); a heterodisulfide of a compound (7) and a compound (6), wherein the compound (7) had Boc group deprotected; a heterodisulfide of compound (6) and compound (1); as well as a disulfide of compound (7) and compound (1).

### EXAMPLE 8: Deprotection reaction of Boc group on a fine gold particle

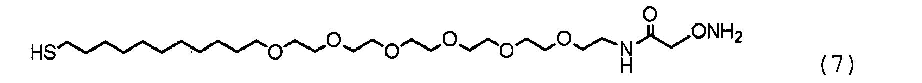

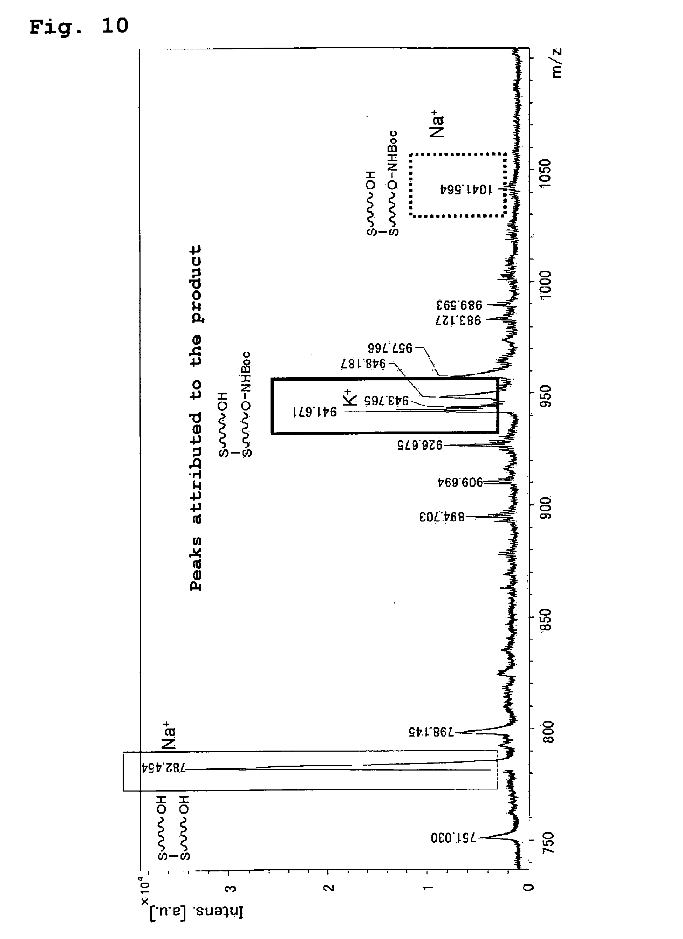

The fine particle produced in Example 7 (10 mg) was dissolved in 2 mL of methanol. One mL of trifluoroacetic acid was added to the solution, and the solution was s agitated at room temperature for 18 hours. The reaction solution was subjected to centrifugal filtration using Centriplus YM-50, thereby purifying a fine particle. The fine particle was dissolved in pure water, and then was subjected to MALDI-TOF mass spectrometry (Figure 10). Figure 10 shows that although a peak derived from compound (6) disappeared, other peaks were observed, and attributed to a disulfide of compound (7) and compound (1), wherein the compound (7) had a deprotected Boc group, indicating that the amino group on the fine gold particle had undergone a deprotection reaction.

### EXAMPLE 9: Sugar chain-trapping reaction in an aqueous solution using sugar chain-trapping fine gold particles.

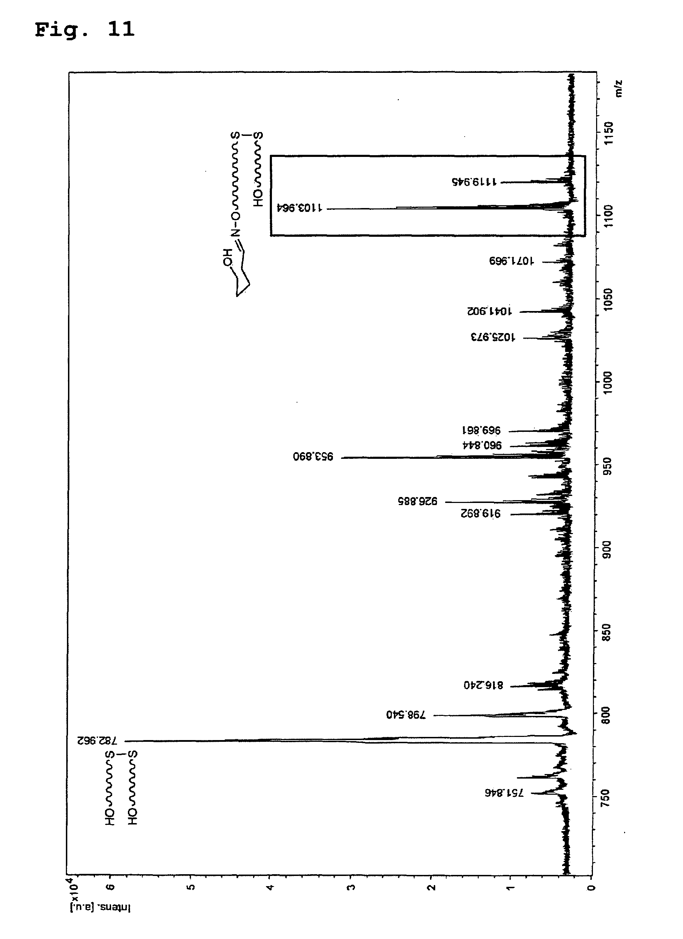

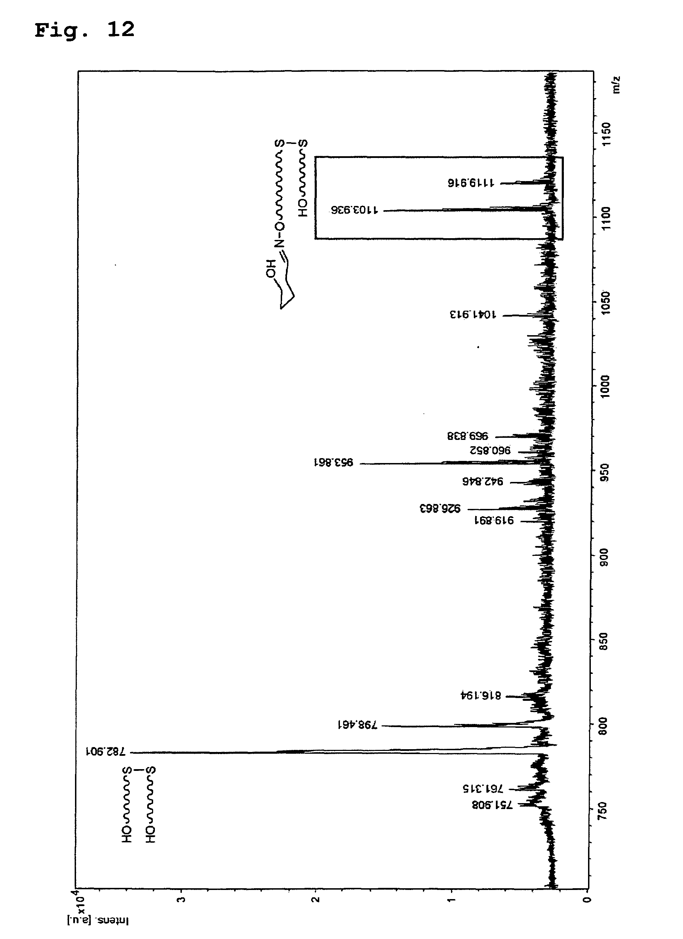

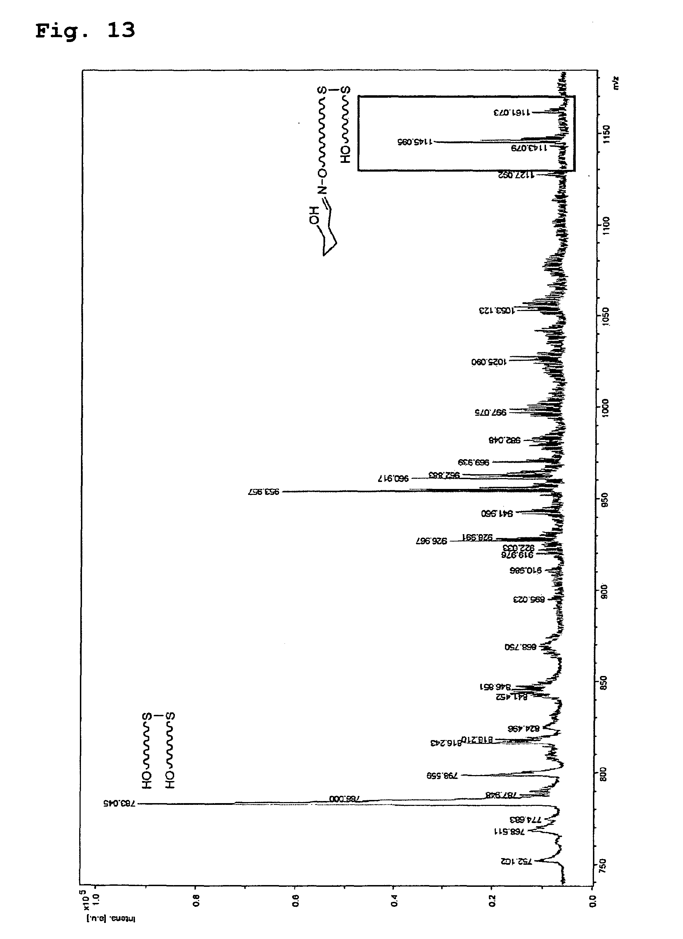

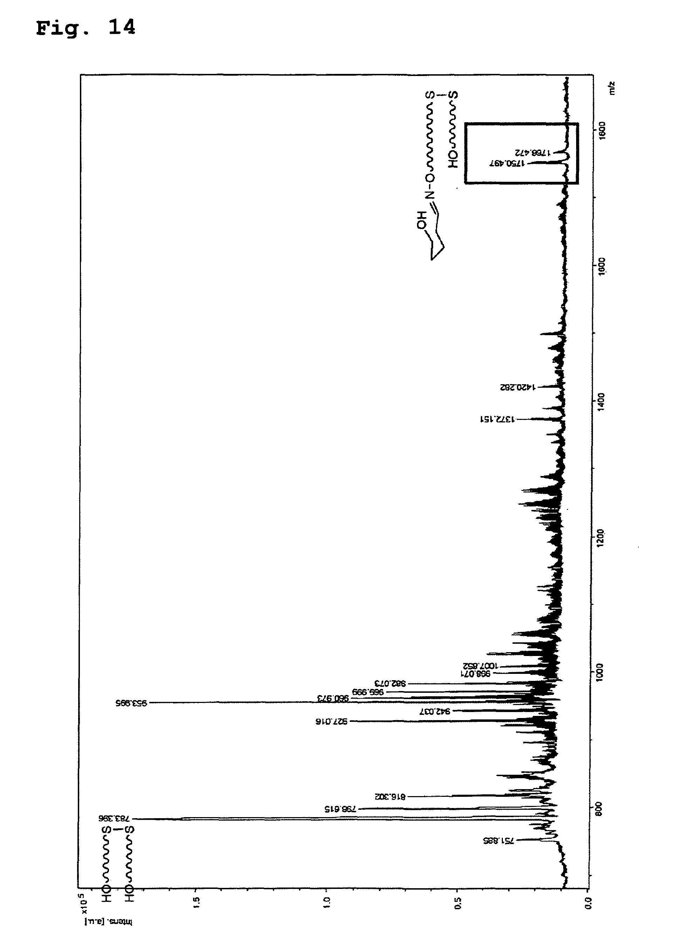

170 µg of sugar chain-trapping fine gold particles produced in accordance with Example 8 was put into respective Eppendorf tubes, and was then dissolved into 100 µL of pure water. One mg of mannose, glucose, N-acetylglucosamine, and mannopentaose were gauged and dissolved into the respective particle solutions. The resultant solutions were incubated at 37 °C for 6 days. After the reaction, the solutions were subjected to centrifugal filtration using CentriplusYM-50, thereby purifying the fine particles. The fine particles were dissolved in pure water, and then subjected to MALDI-TOF Mass spectrometry (mannose (Figure **11),** glucose (Figure **12),** N-acetylglucosamine (Figure **13),** and mannopentaose (Figure **14)).** In each of the Figures **11-14,** observed peaks were attributed to a disulfide which had a compound (1) and resepective sugar-chains covalently bound to the oxiamine. A sugar chain-trapping reaction was exerted by an oxiamino group provided to the fine gold particle.

### EXAMPLE 10: Measurement of absorbance of AHTM using D-glucose

10 mg of D-glucose (0.056 mol) was dissolved in 10 mL of water. The D-glucose solution was put into a 96 well-plate in ten different concenration levels, where the concentration was increased by increment of 1 µl from 1 to 10 µl. Subsequently, 10 mM NaIO₄ was added to the 10 wells, in 50 µL per well. After the plate was set at room temperature for 10 minutes, 50 µL of 41 mM AHMT (4-amino-3-hydrazino-5-mercapto-1,2,4-triazol) (0.5 M HCl) was added to each well, and thereafter, 100 µL of 2 M KOH was added to each well. After the plate was set at room temperature for 2 hours, coloration occurred. A₄₉₂ was measured and thus OD (optical density) at 492 nm were ploted, with respect to resepctive D-glucose concentrations. As a result, the plot showed almost linear correlation. It has revealed that the reaction between AHMT and an aldehyde group of D-glucose will develop in a first-order reaction.

### EXAMPLE 11: Results of coloring test of AHTM using Fetuin were shown.

As a sample for coloring test of AHTM, sialoglycoprotein was used, which had been contained in bovine serum. The following four samples were prepared to observe coloring of AHTM.
1. 200 µl of 10 mg/ml Fetuin and 50 µl of 10 mM NaIO₄ was mixed together, and then placed on ice for 10 minutes. Subsequently, 200 µl of 50 mM AHMT was added, and then the mixture was placed at room temperature for 30 minutes.
2. 200 µl of 10 mg/ml Fetuin was placed on ice for 10 minutes. Subsequently, 200 µl of 50 mM AHMT was added, and then the mixture was placed at room temperature for 30 minutes.
3. 200 µl of 10 mg/ml Fetuin and 50 µl of 10 mM NaIO₄ was mixed together, and then placed on ice for 10 minutes. Subsequently, the mixture was placed at room temperature for 30 minutes.
4. 200 µl of 10 mg/ml Fetuin was placed on ice for 10 minutes. Subsequently, the mixture was placed at room temperature for 30 minutes.

As a result of the above test, the deepest coloration was observed in the sample 1, indicating that NaIO₄ reacted with the sugar in the Fetuin and thus established sufficient reaction with AHTM.

### EXAMPLE 12: Experiment of trapping a protein of AHTM using a metal plate

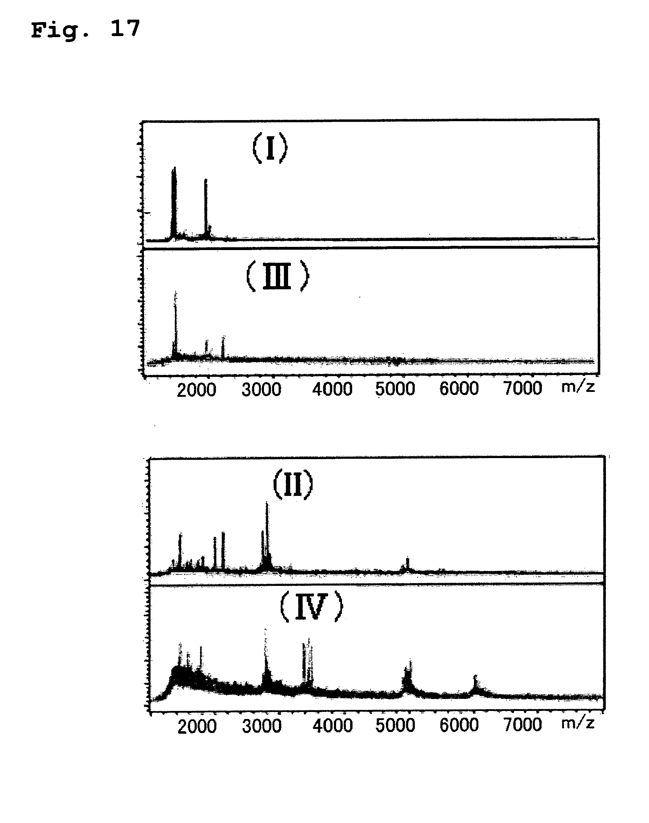

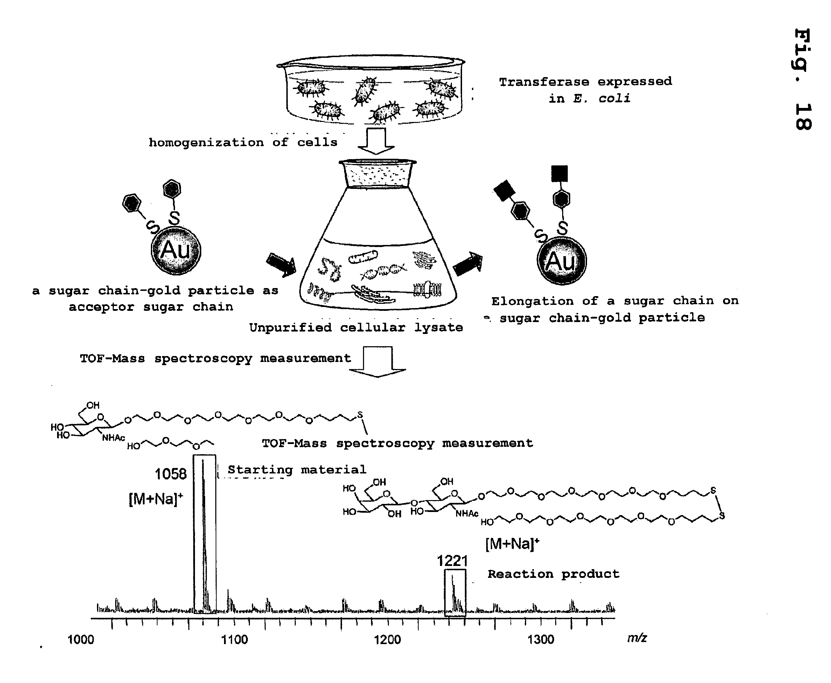

Four circle plates for MALDI-TOF MS were subjected to Au (gold) evaporation for 1,500 seconds. These plates were processed according to the following four types of methods.
(I) The surface of the plate was uniformly covered with 50 mM AHMT (0.2M NaOH). The plate was placed at room temperature for one hour, and thereafter, excess AHMT was removed with water. 200 µl of 10 mg/ml Fetuin, which had undergone trypsin digestion treatment, and 50 µl of 10 mM NaIO₄ were mixed together, and the mixture was put on the plate. After the plate was placed for ten minutes, 50 µl of 0.2 M NaOH was similarly put on the plate, and the plate was placed on ice for one hour (Figure **17).**
(II) The surface of the plate was covered uniformly with 50 mM AHMT (0.2 M NaOH). The plate was placed at room temperature for one hour, and thereafter, excess AHMT was removed with water. 200 µl of 10 mg/ml Fetuin, which had not undergone any enzyme digestion treatment, and 50 µl of 10 mM NaIO₄ were mixed together, and the mixture was put on the plate. After the plate was placed for ten minutes, 50 µl of 0.2 M NaOH was similarly put on the plate, and the plate was placed on ice for one hour (Figure **17).**
(III) 200 µl of 10 mg/ml Fetuin, which had undergone trypsin digestion treatment, and 50 µl of 10 mM NaIO₄ were mixed together in an Eppendorf tube, and then the Eppendorf tube was placed on ice for ten minutes. 200 µl of 50 mM AHMT (0.2M NaOH) was added to the Eppendorf tube. After pink coloration occurred, the mixture was spotted on the plate, and then the plate was placed on ice for one hour (Figure **18**).
(IV) 200 µl of 10 mg/ml Fetuin undigested by enzymes and 50 µl of 10 mM NaIO₄ were mixed together in Eppendorf tube, and then the Eppendorf tube was placed on ice for ten minutes. 200 µl of 50 mM AHMT (0.2M NaOH) was added to the Eppendorf tube. After causing pink coloration, the mixture was spotted on the plate, and then the plate was placed on ice for one hour (Figure **18**).

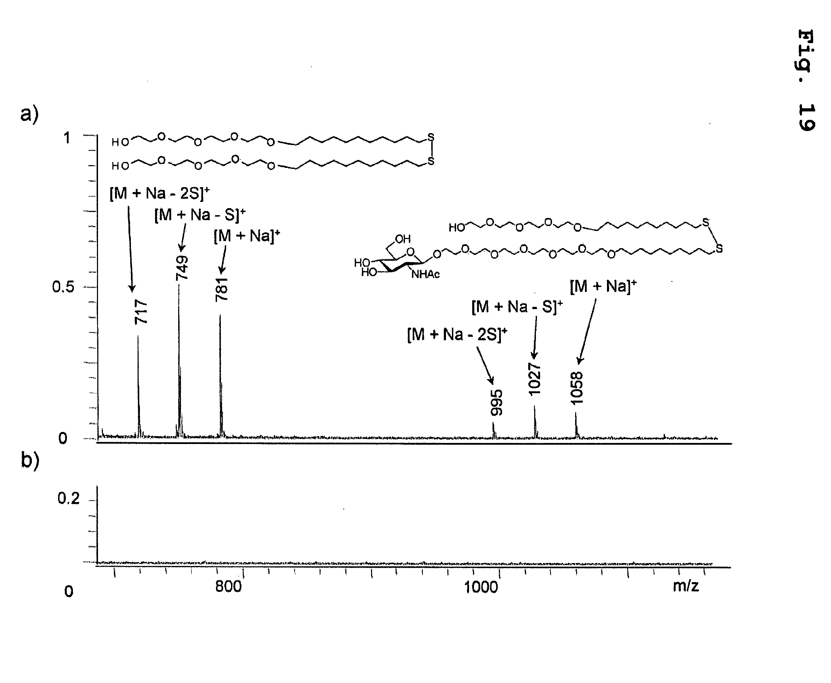

MALDI-TOF MS was carried out using the plates processed in accordance of the above four manners. The results are shown in Figure **19**. Figure **19** shows that glycopeptides having free aldehyde were trapped in the case of Fetuin digested by trypsin, by AHMT on the surface of gold substrate, whether processed by procedure 1 or 2, while in the case of Fetuin not processed with enzymes a glycopeptide could not be successfully trapped whether processed by procedure 1 or 2.

### EXAMPLE 13: Sugar chain elongation reaction on a sugar chain-fine gold particle (GlcNAc-Au) complex with a cellular homogenate of E. coli which had expressed galactosyltransferase (GalT), and tracking of the enzymatic reaction by MALDI-TOF Mass

β1-4 galactosyltransferase (GalT), which is LgtB gene product from *Neisseria meningtids* MC50 strain, was expressed in *E. coli.* The bacteria were homogenized, and then suspended in a buffer containing 0.2% TrionX-100. Total protein concentration in lysate of bacteria was 8.5mg/mL. 120 µL of HEPES buffer solution (10 mM HEPES, 10 mM MnCl₂, 150mM NaCl) (GlcNAc concentration; 500 µM) of the sugar chain-fine gold particle (GlcNAc-AU) complex produced in accordance with Example 4, 20 µL of UDP-Gal (500 µM), and 40 µL of the above lysate of bacteria were mixed together, and then the mixture was incubated at 25°C for 32 hours. 10 µL of the reaction solution was subjected to centrifugal filtration using Centricon YM50, and the obtained fine particles were washed in ultrapure water. When the fine sugar chain particles were subjected to MALDI-TOF Mass measurement, a peak of molecular weight corresponding to product from the enzymatic reaction (compound 5) was detected (Figure **18).** This has proved that the present method is simple, fast, and excellent for measurement of enzymatic activity.

### EXAMPLE 14: Direct detection of thiol compound introduced on a surface of fine gold particle by LDI-TOF Mass spectroscopy

A sugar chain-fine gold particle (GlcNAc-Au) complex produced in accordance with Example 4 was applied to a target for MALDI-TOF Mass, and was directly subjected to LDI-TOF mass spectrometry measurement without using a matrix. In this case, peaks of molecular weight of corresponding to homodisulfide and heterodisulfide of compound (1) and compound (4) were observed (Figure 19a). On the other hand, the same amount of sugar chain-fine particle (GlcNAc-Au) complex was mixed with iodine, the thiol ligand was liberated from the surface of the fine gold particle, and the reaction solution was subjected to TOF-mass spectrometry measurement. In this case, although the reaction solution contains thiol compounds (1) and (4), ionization of a thiol compound was not observed (Figure **19b).** In this example, no low molecular weight matrix, such as 2,5-dihydroxybenzoic acid (DHB) was used. Accordingly, it has proved that a thiol compound introduced on a surface of a fine gold particle can be ionized by direct irradiation of laser beam, without using a low-molecular matrix, and can be detected by TOF Mass spctroscopy (LDI-TOF mass spectrometry).

### EXAMPLE 15: Sugar chain elongation reaction on a sugar chain (Gal-GlcNAc)-fine gold particle complex by a fucosyltransferase (FucT)

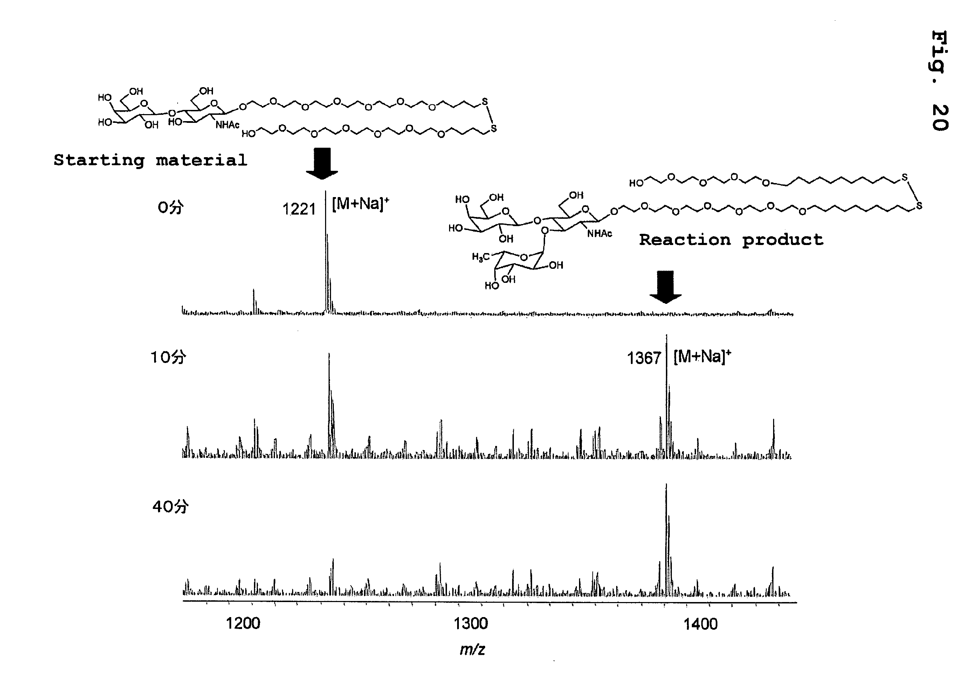

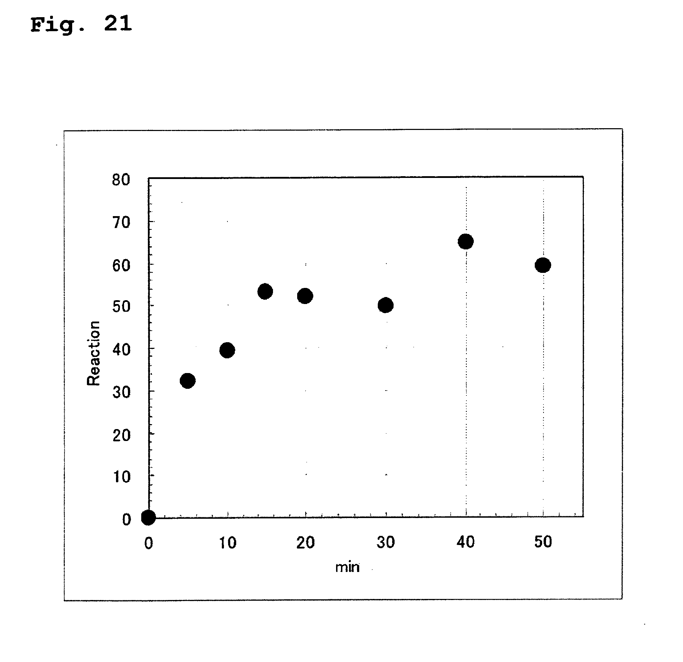

A sugar chain (Gal-GlcNAc)-fine gold particle complex produced in accordance with Example 5 was dissolved in 10 mM HEPES buffer (10 mM MnCl₂, 150 mM NaCl). Five mU of α1,3-FucT VI and GDP fucose (final concentration is 500 µM) were added thereto, and the solution was incubated at a reaction temperature of 25°C. One µL of the solution was extracted from the reaction system at 5, 10, 20, 30, 40, 50 and 60 minutes after initiation of the reaction, and then was subjected to MALDI-TOF mass spectroscopy measurement (Figures **20** and **21).** The peaks corresponding to the reaction products were also observed by LDI-TOF Mass spectroscopy measurement.

### EXAMPLE 16: Structure analysis of a sugar chain by LDI-TOF/TOF measurement of a sugar chain-fine gold particle complex

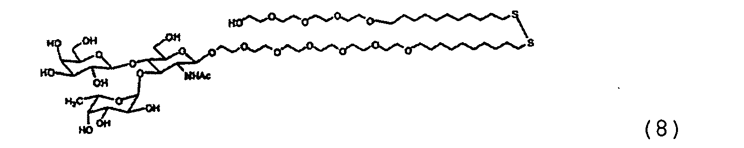

A sugar chain (Galβ1,4(Fucα1,3)GlcNAc; Lex)-fine gold particle complex produced in accordance with Example 15 was subjected to LDI-TOF Mass spectroscopy measurement. With an obtained peak ([M(compound 8)+Na]⁺1368.75) defined as a parent ion, further LDI-TOF/TOF analysis was carried out to obtain a pattern of the fragment ions. The structure of sugar chain of Lex was indetified based on the pattern. This has revealed that mass spectrometry and structure analysis for an organic residue bound through a sulfur atom to a metal can be carried out by LDI-TOF or LDI-TOF/TOF Mass spectroscopy.

### EXAMPLE 17: Ionization efficiency comparisons in LDI-TOF mass spectroscopy measurements of thiol compounds supported on a surface of a fine gold particle or a gold substrate

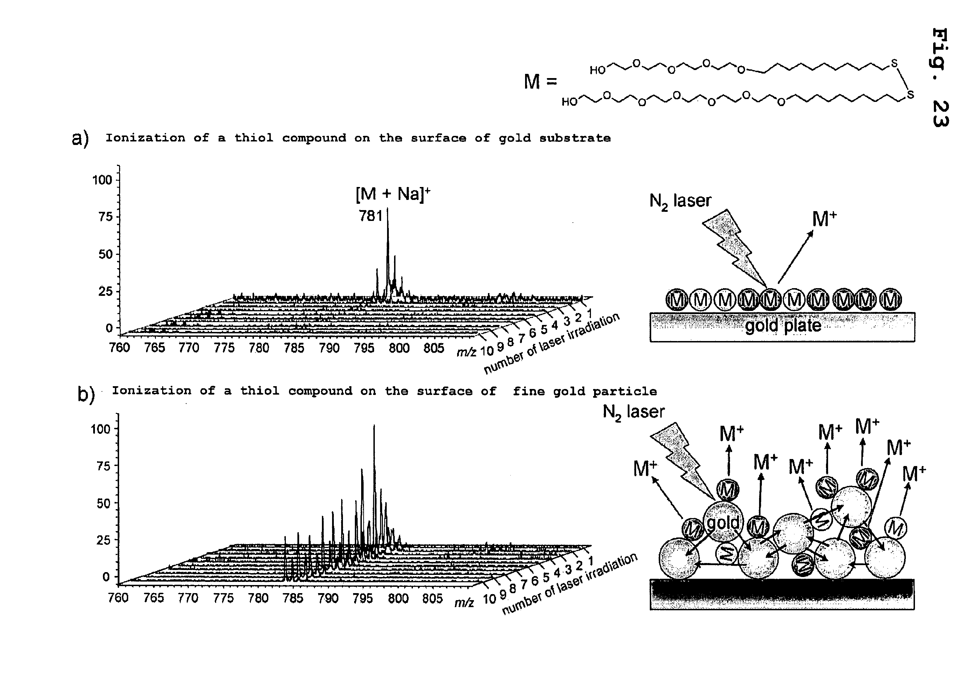

A fine gold particle produced in accordance with Example 1 was dissolved in a buffer so that the thiol compound concentration was 100 µM. 0.2 µL of this solution was applied to a plate for MALDI-TOF mass spectrometry so that the solution formed a circle having a diameter of 2 mm. Meanwhile, compound (1) was dissolved in methanol so that the thiol concentration was 500mM. 0.1 µL of the solution was applied to a gold coated plate for MALDI-TOF mass spectrometry so that the solution formed a circle having a diameter of 2 mm, thereby forming a self-organized film on the gold plate. The gold plate had been obtained by Au (gold) evaporation just before the applications. The above two samples were subjected to LDI-TOF mass spectrometry measurements. One burst of laser beam was irradiated, at the the same intensity, onto the same portion of the respective targeted plates. The same process was carried out repetitively for 10 times in total, and then the obtained peak intensities were recorded (Figure **23).** In the results, in the case of the gold substrate, the intensity of ionization gradually decreased with the increase in the number of laser irradiation shots. After fourth irradiation, the peak completely disappeared. In the case of the fine gold particle, on the other hand, sufficient amount of ionization was observed from the suface of the particles, even after tenth laser irradiation. Further, the peaks attributed to compound (1), were also observed after thirtieth laser irradiation, and even after fiftieth irradiation. Thus, it was revealed that ionization efficiency in LDI-TOF mass spectrometry of a thiol compound bound to a surface of gold carrier depends on a shape of the carrier, and that ionization from a surface of a fine gold particle is superior to that of a tabular gold substrate.

### EXAMPLE 18: Production of a magnetic fine gold particle supporting a thiol compound, and direct observation thereof by (MA) LDI-TOF mass spectrometry

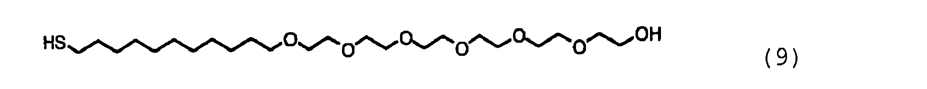

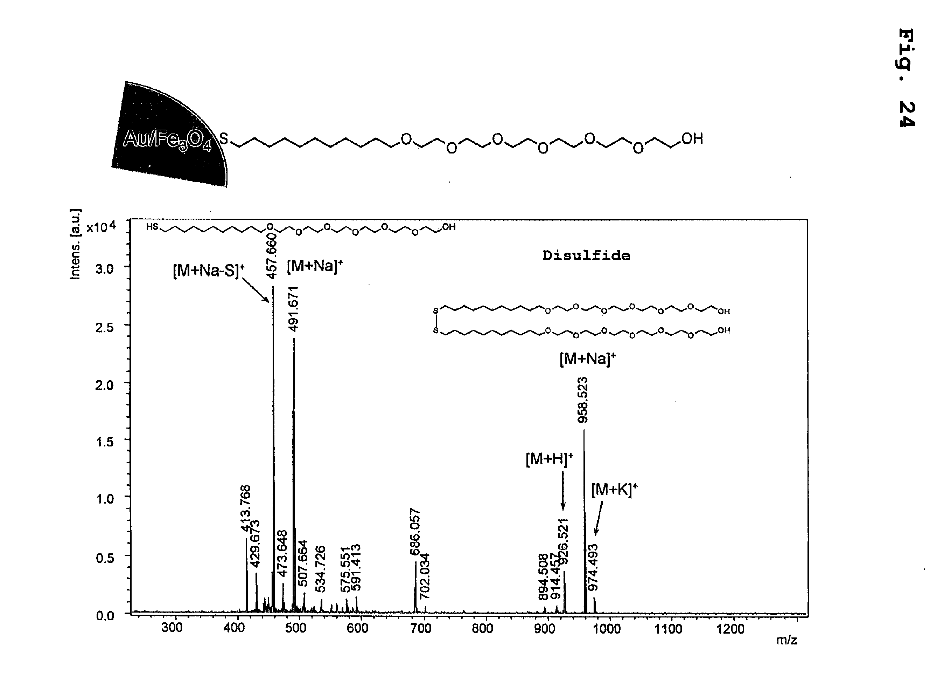

10 mg of a commercially available iron oxide (Fe₃O₄) particle (diameter: approximately 0.2 µm) was suspended in 40 mL of methanol. 50 µL of 17% tetrachloroauric acid hydrochloric acid solution and 10 mg of ligand (9) was added thereto. An aqueous solution obtained by dissolving 20 mg of sodium borohydride in 2 mL of ultrapure water, was gradually added to the solution. The magnetic fine gold particles, which had compounds (9) introduced thereon, were obtained by shaking the above solution at room temperature for one hour, followed by the application of a magnet to the resultant solution. The magnetic fine gold particles were washed in methanol, and then were subjected to MALDI-TOF mass spectrometry measurement using 2,5-dihydroxybenzoic acid (DHB) as a matrix. Peaks were observed, and attributed to the ionized ligand (9). Further, when the fine particles were directly applied to a target plate for MALDI-TOF mass spectoscopy without using a matrix and irradiated with a laser beam, peaks was observed, and attributed to the ionized ligand (9) (Figure **24).** This has revealed that a thiol compound on a fine particle can be ionized without a matrix such as DHB in LDI-TOF mass spectoscopy, by directly irradiating laser beams to the thiol compound bound to a surface of magnetic fine gold particles.

### EXAMPLE 19: Production of a sugar chain-magnetic fine gold particle complex in which the sugar chain density is controlled

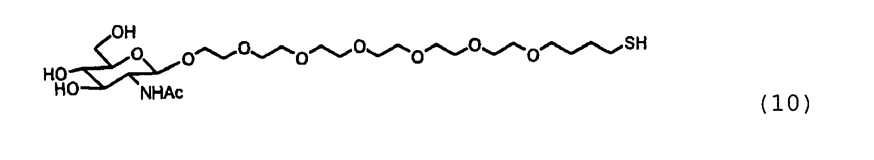

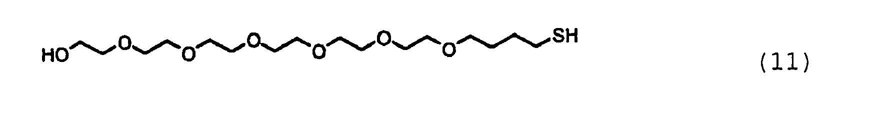

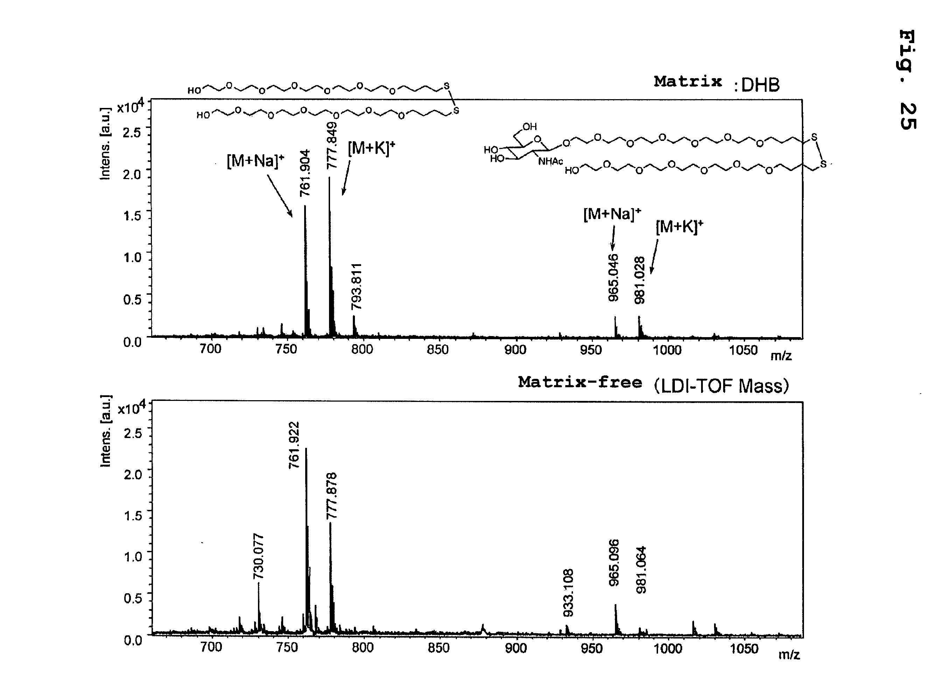

In the same manner as in Example 18, 1 mL of aqueous solution of compound (10) having N-acetylglucosamine at a terminus (10 nmol/µL) and compound (11) (58 mg, 79 µmol) were dissolved in 40 mL of methanol. Six mg of fine iron oxide particle was added to, and was suspended in the solution. 100 µL of 17% tetrachloroauric acid hydrochloric acid solution was added to the solution. An aqueous solution obtained by dissolving 30 mg of sodium borohydride in 4 mL of ultrapure water, was gradually dropped into the above solution. The magnetic fine gold particles, which had ligands (10) and (11) introduced thereon, were obtained by shaking the above solution at room temperature for one hour, followed by the application of a magnet to the resultant solution. The magnetic fine gold particles were washed in methanol, and then subjected to LDI-TOF Mass spectroscopy measurement (Figure **25).** Peaks of molecular weight corresponding to homodisulfides and heterodisulfides of compound (10) and compound (11) were detected. Meanwhile, when MALDI-TOF mass spectrometry measurement was carried out using DHB as a matrix, the same peaks corresponding to molecular weight were also obtained, as those obtained in the LDI-TOF mass spectrometry.

### EXAMPLE 20: Sugar chain elongation reaction on a sugar chain (GlcNAc) presented on a surface of a magnetic fine gold particle by galactosyltransferase (GalT)

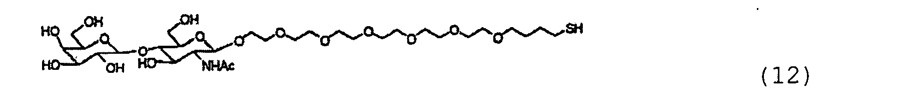

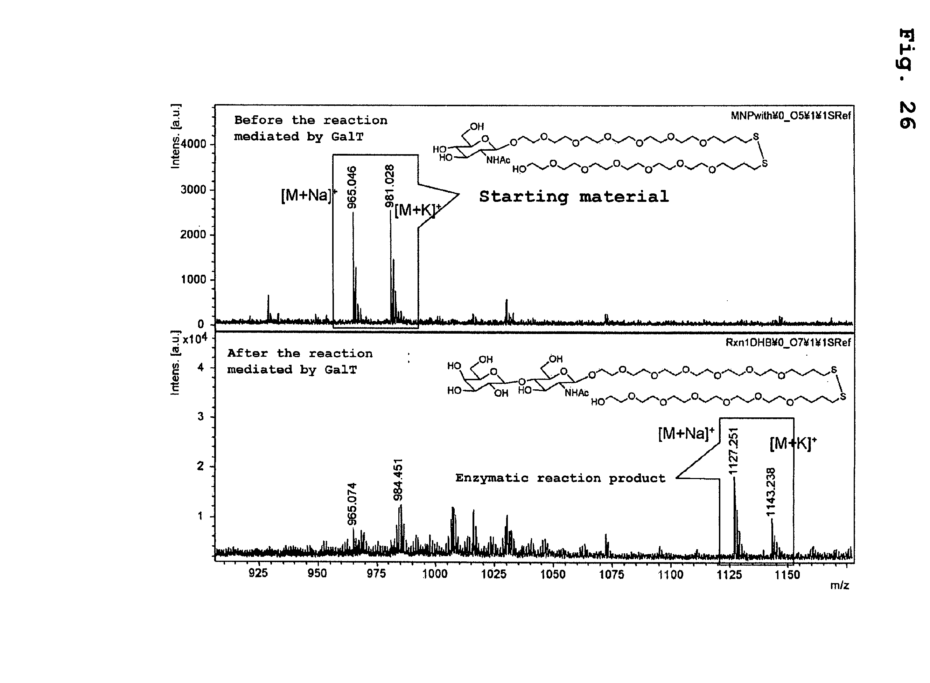

Five µL of a solution which had been obtained by suspending a sugar chain-magnetic fine gold particle complex according to Example 19 in 400 µL of ultrapure water, and 20 µL of UDP-Gal (concentration: 1 mM) dissolved in 10 mM HEPES buffer (containing 10 mM MnCl₂ and 150 mM NaCl), were mixed in an Eppendorf tube having a size of 1.5 mL. Four mU of β1-4 galactosyltransferase was added to the solution, to allow the development of the enzymatic reaction at 37°C. At five hours after the initiation of the reaction, a part of the reaction solution (5 µ) was extracted and washed in ultrapure water. When fine particles were recovered with a magnet, and were subjected to MALDI-TOF mass spectrometry measurements **(Figure 26),** peaks corresponding to the starting substance (disulfides of compounds (10) and (11)) disappeared, and other molecular weight peaks were observed ([M+Na]⁺=1127.251), and attributed to heterodisulfides of compounds (12) and (11), which had a galactose added by the enzymatic reaction. This has revealed that an enzymatic reaction on a surface of magnetic fine gold particles having a sugar chain provided on a surface thereof can be readily tracked.

### EXAMPLE 21: Introduction of thiol compound on a surface of a fine CdS particle and direct detection thereof by (MA) LDI-TOF Mass spectroscopy

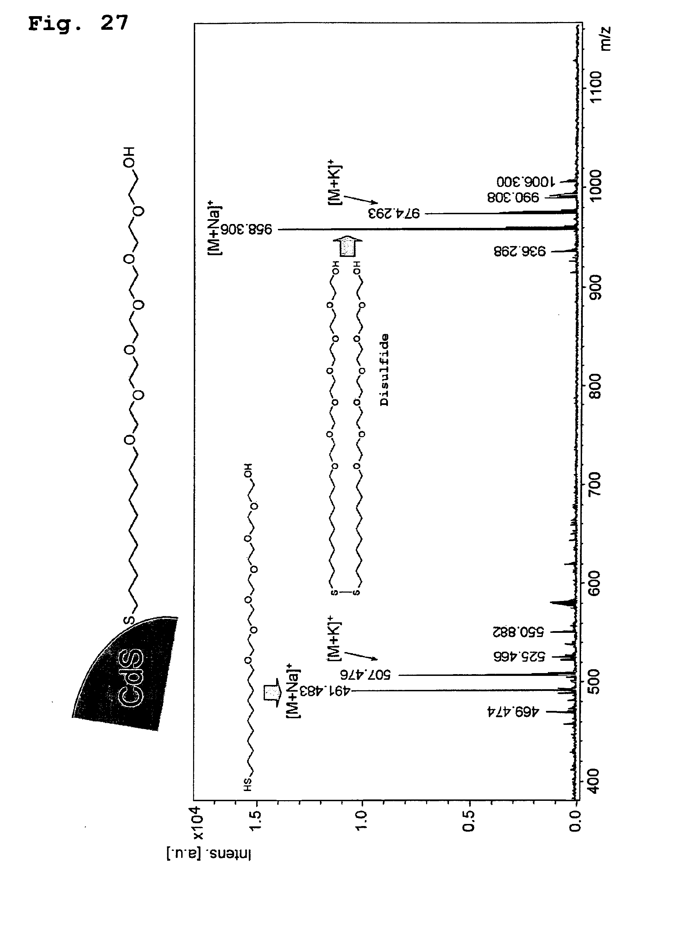

CdCl₂ · 2.5 H₂O (9.6 mg, 42 µmol) was dissolved in 10 mL of ultrapure water. Methanol solution (liquid amount: 2 mL) of compound (9) (10 mg) was added thereto, and the solution was stirred. Solution obtained by dissolving Na₂S·9 H₂O (10 mg, 42 µmol) in 2 mL of ultrapure water was gradually added to the above solution, and was agitated at room temperature for two hours. Yellow precipitates were generated, and removed by centrifugal separation (350 rpm, 5 minutes). The supernatant was subjected to centrifugal filtration using Centriplus YM-50, and the fine particles obtained on the filter were washed in methanol and ultrapure water. When the fine particles were subjected to LDI-TOF Mass spectroscopy measurement, a peak of molecular weight ([M+Na]⁺958.306, [M+K]⁺974.293) corresponding to homodisulfide of compound (9) was observed (Figure **27).** Meanwhile, when MALDI-TOF mass spectrometry measurement was carried out using DHB as a matrix, the same peaks of molecular weight were also obtained, as those obtained in the LDI-TOF mass spectrometry. This has revealed that a thiol compound introduced on a surface of a fine CdS particle can be detected by (MA) LDI-TOF Mass spectroscopy.

### EXAMPLE 22: Glycosylation reaction on a surface of fine gold particles which have a hydroxyl group containing thiol compounds supported thereon

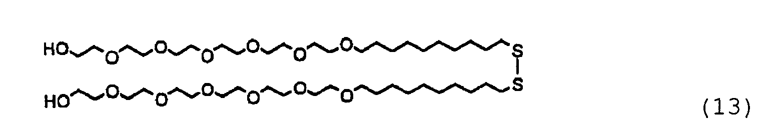

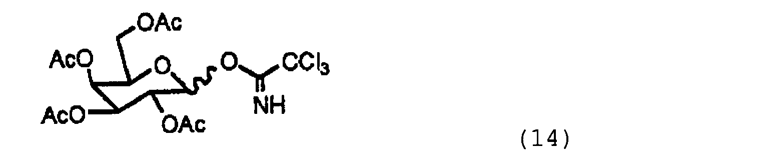

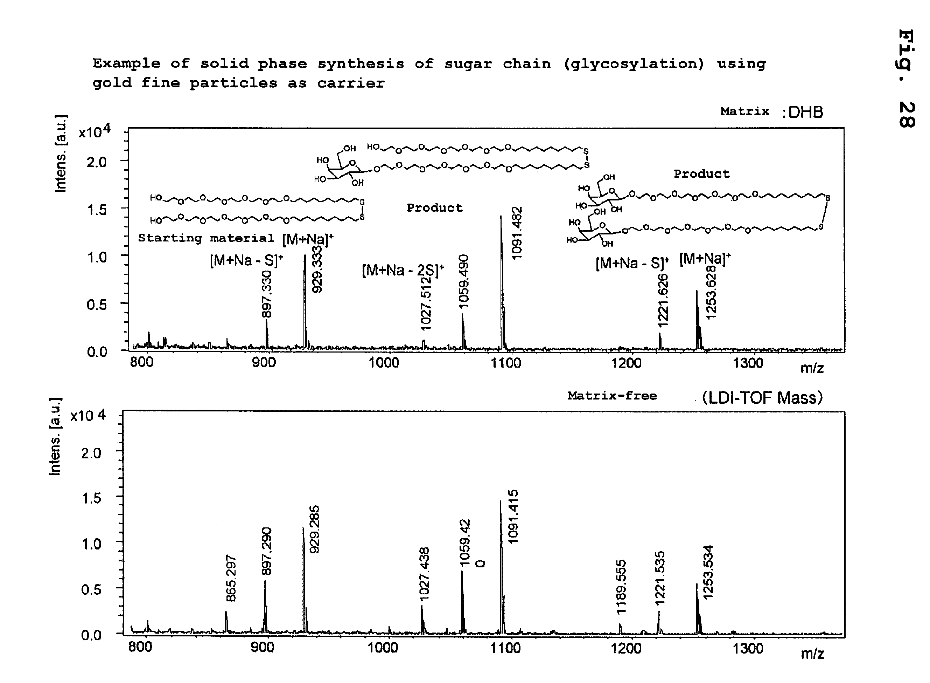

In the same manner as in Example 1, fine gold particles having compound (13) introduced on a surface thereof were produced. 1.5 mg of these fine gold particles were dissolved in 1 mL of dry dichloromethane, and 5 mg of 2, 3, 4, 6-tetra-O-acetyl-D-galactose trichloroacetoimidate (14) was added to the solution. The reaction system was cooled to 0 °C. 10 µL of trimethylsilyl trifluoromethanesulfonate was added to the reaction system, and the solution was stirred for 6 hours. After centrifugal filtration of the reaction system using Centricon YM-50, a fine particle was dissolved in methanol. Four mg of sodium methoxide was added to the fine particle, and the mixture was agitated at room temperature for 20 hours. Since the fine particle was insolubilized in methanol, centrifugation (3,000 rpm, 1 minute) was carried out to precipitate the fine particles, and the supernatant was removed. The fine particles were dissolved in ultrapure water, and then were subjected to (MA) LDI-TOF mass spectrometry measurement. Peaks were observed, which corresponded to molecular weight of a glycosylation reaction product having a galactose residue introduced thereto (Figure **28).** This has revealed that fine gold particles coated with thiol compounds can be used as a solid phase carrier in an organic solvent, and that reaction progress in synthesis of a solid phase can be readily tracked by (MA) LDI-TOF mass spectrometry.

### EXAMPLE 23: Screening of sugar chain-specific protein by using a sugar chain-magnetic fine gold particle complex

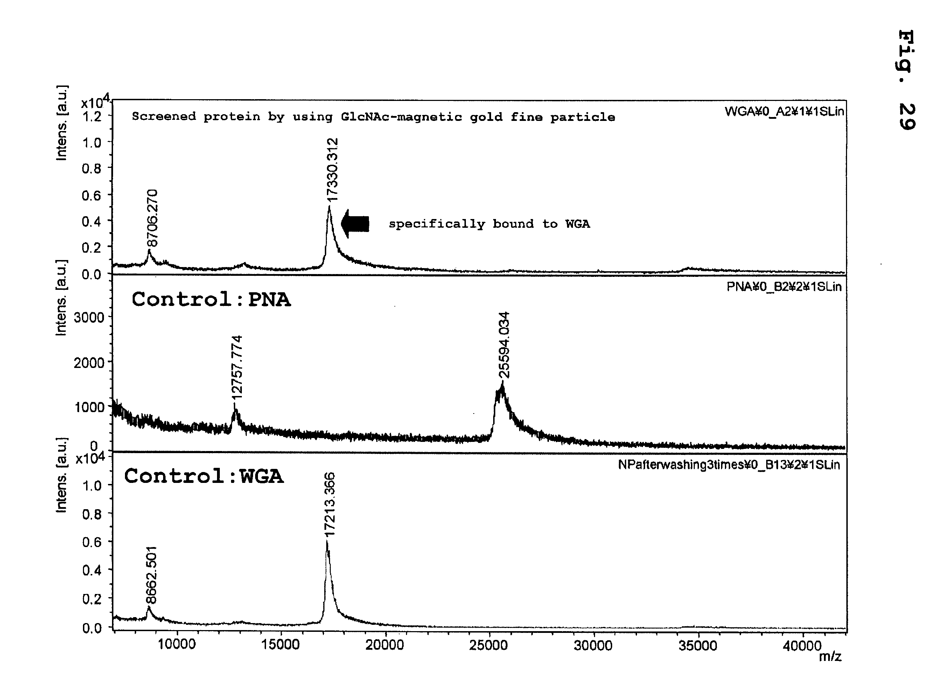

27 µM of wheat germ lectin (WGA), which is a GlcNAc-binding protein, and 20 µM of peanut lectin, which is a galactose-binding protein, were dissolved in 100 µL of phosphoric acid buffer. Two µL of solution obtained by suspending sugar chain(GlcNAc)-magnetic fine gold particles produced in accordance with Example 19, in ultrapure water, was added thereto, and the solution was incubated at room temperature for 30 minutes. Thereafter, fine particles were recovered using a magnet, and then washed in 100 µL of ultrapure water three times. After washing, the magnetic fine particles recovered with the magnet were suspended in 100 µL of ultrapure water. 0.5 µL of the result was mixed with 0.5 µL of DHB (10 mg/mL) and the mixture was subjected to MALDI-TOF mass spectrometry measurement (Figure **29).** In this case, only peaks corresponding to WGA were observed. This has revealed that a sugar chain-magnetic fine gold particle enables screening a protein which specifically interacts with a sugar chain supported on the magnetic fine gold particle among a mixture of proteins as well as identifying the screened protein by mass spectrometry.

### EXAMPLE 24: Detection of an inhibitor by a mass spectrometer using a protein (α-amylase) bound to a fine gold particle

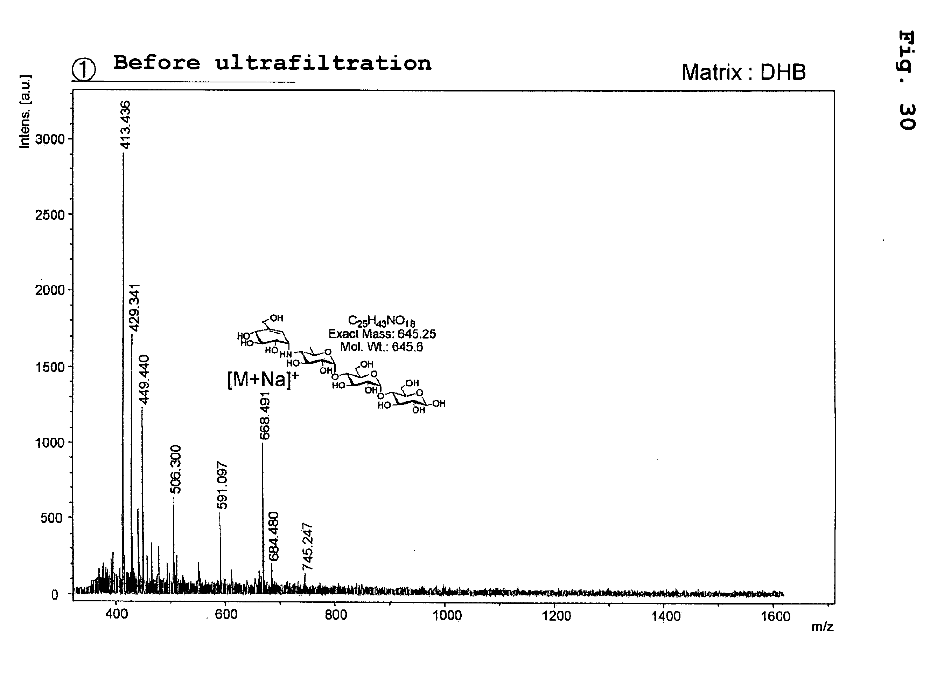

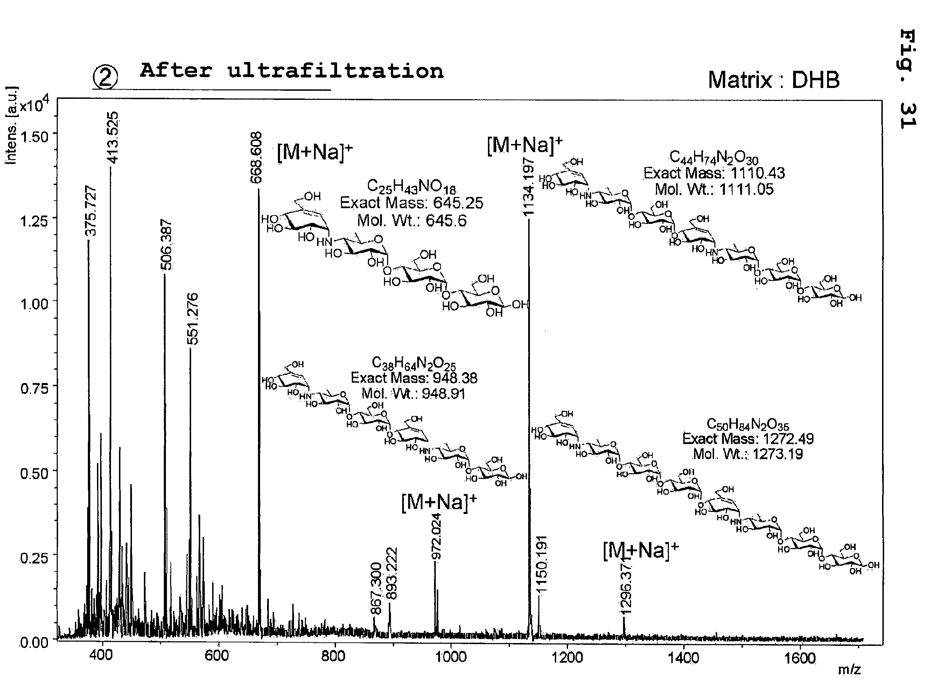

5.8×10⁻⁸ mol of α-amylase (SIGMA A6255) and 5.8×10⁻⁸ mol of 2-iminothiolane hydrochloride (1 equivalent) were added to 400 µL of phosphoric acid buffer (100 mM, pH 7.4). The solution was put into Eppendorf tube and agitated. Subsequently, the solution was incubated at 37 °C for 30 minutes. After 30 minutes, 200 µL of fine gold particles (SIGMA G1652) were added to the resultant solution, and agitated. Thereafter, the solution was placed at room temperature for 12 hours. Subsequently, the reaction solution was subjected to ultrafiltration (Microcon YM-50, Millipore). The α-amylase-fine gold particle complex obtained on ultrafiltration membrane was washed and subjected to centrifugal concentration in 100 µL of ultrapure water. These procedures were repeated 3 times. The α-amylase-fine gold particle complex was dissolved in 100 µL of ultrapure water. Acarbose (5.8×10⁻⁷ mol, 10 equivalents) was added thereto, and the solution was placed at 4°C for 12 hours. The resulting reaction solution was defined as "reaction solution (1) before ultrafiltration process". A part of the reaction solution was subjected to an ultrafiltration process, thereby obtaining a reaction solution with a low-molecular compound having a weak binding ability to α-amylase, which was defined as "reaction solution (2) after ultrafiltration process". 0.5 µL of each of the reaction solutions (1) or (2) were mixed with 0.5 µL of a matrix solution (DHB, 10 mg/mL) , and MALDI-TOF mass spectrometry measurement was carried out (Figures **30** and **31).** It has revealed that hexasaccharide, pentasaccharide, octasaccharide products generated by transglycosylation of acarbose, which binds more strongly to α-amylase, can be selectively detected in the case of the reaction solution (2) after ultrafiltration process, while such products cannot be detected in the case of the reaction solution (1) before ultrafiltration process, due to its slight amount.

### EXAMPLE 25: Production of a magnetic fine gold particle

1.5 mg of commercially available iron particle (particle diameter: 2-3 µm) was suspended in 100 µL of water. 100 µL of water, 1 mg of ligand (1), and 100 µL of potassium tetrachloroaurate solution (10mg/ml) were added thereto. 20 µl of 35 wt% hydrazine was gradually added to this solution while stirring the solution. After settling the solution at room temperature for 3 hours, fine particles were recovered using a magnet, and then washed in methanol and water. 100 µL of methanol, 1mg of ligand (1), and 100 µL of potassium tetrachloroaurate solution were added to the fine particles. 20 µl of 35 wt% hydrazine was gradually added to this solution while stirring the solution. The solution was shaken overnight at room temperature. Thereafter, the fine particles were recovered using a magnet, and washed in methanol and water. The fine particles were applied to a target plate for MALDI-TOF mass spectrometry using 2,5-dihydroxybenzoic acid (DHB) as a matrix, and was irradiated with a laser beam. In this case, a peak corresponding to a homodisulfide of ionized ligand (1).

### EXAMPLE 26: Trapping of a glycosphingolipid by oxylamine-fine gold particle complex

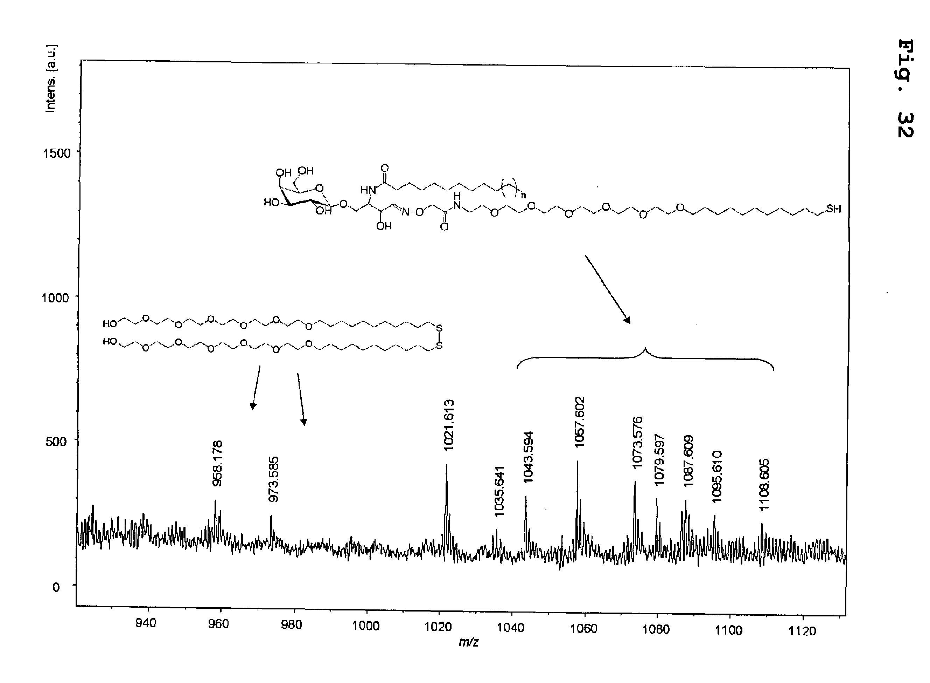

Two mg of galactosylceramide was dissolved in 1 mL of a mixture of the solvents chloroform and methanol (chloroform:methanol=1:1). Ozone was bubbled into the solution, and thus olefin was formed in the long chain base of galactosylceramide through ozonolysis into an aldehyde. This reaction was confirmed by TLC. After 30 minutes, 200 µl of dimethylsulfide was added to the solution and the reaction was terminated. 10 µl of solution of oxylamine-fine gold particles prepared in accordance with Example 8 was added to 10 µl of the reaction solution, and the solution was incubated at 60°C for 12 hours. Thereafter, centrifugal filtration was carried out using Centriplus YM-50. The gold nanoparticles left on the filter was washed in methanol. These gold nanoparticles were subjected to MALDI-TOF Mass measurement using 2, 5-dihydroxybenzoic acid (DHB) as a matrix. In this case, a peak corresponding to compound 7 having galactosylceramide trapped thereby was observed (Figure 32). This has revealed that a glycolipid can be trapped by oxylamine-fine gold particles.

### INDUSTIAL APPLICABILITY

According to the present invention, a mass spectrometry method can be provided which has very high sensitivity to a sulfur atom-containing derivative of an organic residue.

The present invention enables accurate real-time monitoring for enzymatic reactions related to sugar chains. By presenting a sugar chain which is meant to be a substrate for the enzymatic reaction on a surface of a metal which enables diffuse reflection of a laser beam, the enzymatic reaction can be tracked with a mass spectrometry method. Even if a buffer or a salt, is present at a high concentration, which is undesired in the case of conventional mass spectrometry, the present invention can enable a highly-sensitive measurement without being affected by the buffer or a salt. Thus a part of the resultant reaction solution can be used for conveniently performing a sequent analysis without addtional processing. Since the resultant reaction products can be directly observed, the present method has an advantage in that kinetic analysis of the enzymatic reaction can be readily carried out.

In conventional MALDI-TOF MS methods, it is considered a problem that a peak of a substance of interest cannot be observed well in the "low molecular weight regions where a candidate compound may often show a peak", due to the intense peak of the matrix per se. However, the present invention has an advantage that matrix-free LDI-TOF MS can be carried out with a high sensitivity and accuracy, by making a low molecular weight compound of interest supported on a metal substrate (in particular, a fine metal particle) as in the present invention.

By providing a metal-organic residue complex particle of the present invention, a sugar chain or various functional groups can be provided on the surface of a fine metal particle, using the bond between a thiol group and a metal. Moreover, solubility of the fine metal particle can be freely controlled. Therefore, a fine metal particle coated with a thiol compound can be used as a solid phase carrier not only for reaction in an aqueous solvent but also for solid phase synthesis in an organic solvent. When a fine particle is suspended in a reaction solvent, it can be separated from the solution component by centrifugal filtration, and thus separate purification can be performed very readily, which is an advantage. Further, the progress of a reaction can be directly tracked by a mass spectrometry, thereby achieving efficiency.

The present invention has an advantage that use of a fine complex particle containing a magnetic particle such as iron, nickel, cobalt or iron oxide (Fe₃O₄) and a layer of gold, silver, cadmium, selenium or the like coating the surface of the particle as a fine metal particle, enables the easy recovery of the particle after the sulfur atom-containing derivative of the organic residue is supported on the fine complex particle, using a magnet or the like, thereby performing mass spectrometry without purification.

According to the present invention, a fine metal particle containing an organic residue without cytotoxicity bound through a sulfur atom to the fine metal particle is provided, thereby enabling direct introduction of a metal-organic residue complex particle into a living cell, and allowing for the accurate analysis of the various chemical reactions (for example, enzymatic reaction) in the cell by performing mass spectrometry of the reaction product.

## Claims

1. A method for performing mass spectrometry of sulfur atom-containing derivatives of an organic residue, **characterized in that** the method comprises ionizing a metal-organic residue complex into the derivatives, wherein the complex has the organic residue bound through a sulfur atom to the metal.

2. A method for performing mass spectrometry of a compound or salt thereof, **characterized in that** the method comprises ionizing a metal-organic residue complex into sulfur atom-containing derivatives,
wherein the metal-organic residue complex is represented by the general formula (I)
(R-S)ₙ-M¹ (I),
wherein R is an organic residue, S is a sulfur atom and n indicates a stoichiometric ratio of (R-S) group with respect to M¹ and is an integer equal to or greater than 1; and
wherein the compound is represented by the general formulae (II) and/or (III):
R-SH (II)
and/or
R-S-S-R (III),
wherein R and S are the same as defined above.

3. A method for performing mass spectrometry of a compound or salt thereof, **characterized in that** the method comprises ionizing a metal-organic residue complex into sulfur atom-containing derivatives,
wherein the metal-organic residue complex is represented by the general formula (IV):
M¹-S-X-CH(R)-S-M¹ (IV),
wherein R is an organic residue, S is a sulfur atom, M¹ at both ends are same metal entities, X is a lower alkylene or a lower alkenylene;
wherein the compound is represented by the general formulae (V) and/or (VI):
HS-X-CH(R)-SH (V)
and/or 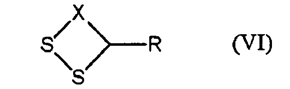 wherein R, S and X are the same as defined above.

4. A method for performing mass spectrometry of a sugar chain or a sugar chain-containing substance, comprising the following steps of:
1) contacting a metal-organic residue complex with a sugar chain or a sugar chain-containing substance under the conditions where the metal-organic residue complex and the sugar chain or sugar chain-containing substance may react with each other, wherein the metal-organic residue complex contains a metal bound to a group represented by the following formula:
- S-Y-(OCH₂CH₂)ₙ-NH-C(=O)-CH₂-O-NH₂,
- S-Y-(OCH₂CH₂)ₙ-NH-C(=O)-CH-O-NH(CH₃),
- S-Y-(OCH₂CH₂)ₙ-NH-C(=O)-CH(NH₂)-W⁴-SH,
- S-Y-(OCH₂CH₂)ₙ-NH-C(=S)-CH(NH₂)-W⁴-SH,
- S-W¹-O-NH₂,
- S-W¹-O-NH(CH₃),
- S-W¹-O-W²-O-NH₂,
- S-W¹-O-W²-O-NH(CH₃),
- S-(CH₂CH₂O)ₙ-W¹-O-W²-O-NH₂,
- S-(CH₂CH₂O)-W¹-O-W²-O-NH(CH₃),
- S-W¹-C(=O)-NH-NH₂,
- S-W¹-C(=S)-NH-NH₂,
- S-W¹-NH-C(=O)-CH(NH₂)-W⁴-SH,
- S-W¹-NH-C (=S) -CH (NH₂) -W⁴-SH
- S-Z¹-Z²-Z³-Z⁴-Z⁵-O-NH₂,
- S-Z¹-Z²-Z³-Z⁴-Z⁵-O-NH(CH₃),
- S-Z¹-Z²-Z³-Z⁴-CH(NH₂) -O-Z⁶-SH,
- S-Z¹-Z²-Z³-Z⁴-CH(NH₂)-Z⁶-SH,
- S-Z¹-O-Z³-CH(NH₂)-Z⁶-SH,
- S-Z¹-O-Z³-O-NH₂,
- S-Z¹-O-Z³-O-NH(CH₃),
- S-Z¹-O-Z³-Z⁴-Z⁵-O-NH₂,
- S-Z¹-O-Z³-Z⁴-Z⁵-O-NH(CH₃),
- S-Z¹-O-Z³-Z⁴-CH(NH₂)-O-Z⁶-SH
- S-Z¹-O-Z³-Z⁴-CH(NH₂)-Z⁶-SH
- S-Z¹-Z³-Z⁴-Z⁵-O-NH₂,
- S-Z¹-Z³-Z⁴-Z⁵-O-NH(CH₃),
- S-Z¹-Z³-Z⁴-CH(NH₂)-O-Z⁶-SH,
- S-Z¹-Z³-Z⁴-CH(NH₂)-Z⁶-SH,
or 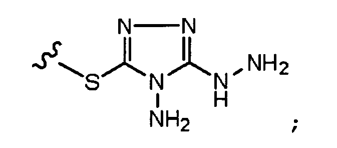
2) obtaining the metal-organic residue complex bound to the sugar chain or the sugar chain-containing substance; and
3) ionizing the metal-organic residue complex bound to the sugar chain or the sugar chain-containing substance into sulfur atom-containing derivatives of the organic residue.

5. A method according to any one of claims 1 to 4, wherein the metal has a surface enough to cause a diffuse reflection of a laser beam.

6. A method according to claim 5, wherein the metal is a fine metal particle.

7. A method according to any one of claims 1 to 6, wherein the metal is gold, silver, cadmium or selenium.

8. A method according to any one of claims 1 to 6, wherein the mass spectrometry is carried out by MALDI-TOF MS method.

9. A method according to any one of claims 1 to 3, wherein the organic residue is a group comprising a sugar chain or a sugar chain-containing substance.

10. A method for performing mass spectrometry of a sulfur atom-containing analyte comprising the steps of:
1) reacting tetrachloroauric acid with a sulfur atom-containing analyte in the presence of a reducing agent;
2) obtaining a gold-analyte complex particle which has the analyte bound through the sulfur atom to the gold; and
3) ionizing the obtained gold-analyte complex particles into a sulfur atom-containing analyte derivative.

11. A metal-organic residue complex containing a metal bound to a group represented by the following formula:
- S-Y-(OCH₂CH₂)ₙ-NH-C(=O)-CH₂-O-NH₂,
- S-Y-(OCH₂CH₂)ₙ-NH-C(=O)-CH₂-O-NH(CH₃),
- S-Y-(OCH₂CH₂)ₙ-NH-C(=O)-CH(NH₂)-W⁴-SH,
- S-Y-(OCH₂CH₂)ₙ-NH-C(=S)-CH(NH₂)-W⁴-SH,
- S-W¹-O-NH₂,
- S-W¹-O-NH(CH₃),
- S-W¹-O-W²-O-NH₂,
- S-W¹-O-W²-O-NH(CH₃),
- S-(CH₂CH₂O)ₙ-W¹-O-W²-O-NH₂,
- S-(CH₂CH₂O)ₙ-W¹-O-W²-O-NH(CH₃),
- S-W¹-C(=O)-NH-NH₂,
- S-W¹-C(=S) -NH-NH₂,
- S-W¹-NH-C (=O) -CH (NH₂) -W⁴-SH,
- S-W¹-NH-C(=S)-CH(NH₂)-W⁴-SH,
- S-Z¹-Z²-Z³-Z⁴-Z⁵-O-NH₂,
- S-Z¹-Z²-Z³-Z⁴-Z⁵-O-NH(CH₃),
- S-Z¹-Z²-Z³-Z⁴-CH(NH₂)-O-Z⁶-SH,
- S-Z¹-Z²-Z³-Z⁴-CH(NH₂)-Z⁶-SH,
- S-Z¹-O-Z³-CH(NH₂)-Z⁶-SH,
- S-Z¹-O-Z³-O-NH₂,
- S-Z¹-O-Z³-O-NH(CH₃),
- S-Z¹-O-Z³-Z⁴-Z⁵-O-NH₂,
- S-Z¹-O-Z³-Z⁴-Z⁵-O-NH(CH₃),
- S-Z¹-O-Z³-Z⁴-CH(NH₂) -O-Z⁶-SH
- S-Z¹-O-Z³-Z⁴-CH(NH₂)-Z⁶-SH
- S-Z¹-Z³-Z⁴-Z⁵-O-NH₂,
- S-Z¹-Z³-Z⁴-Z⁵-O-NH(CH₃),
- S-Z¹-Z³-Z⁴-CH(NH₂) -O-Z⁶-SH,
- S-Z¹-Z³-Z⁴-CH (NH₂) -Z⁶-SH,
or 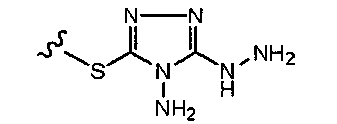
wherein, Y, W¹ and W² are independently C1-C12 alkylene, C2-C12 alkenylene or C2-C12 alkynylene;
w⁴ is C1-C2 alkylene;
Z¹ is substituted or unsubstituted arylen or heteroarylen;
Z² is a nitrogen-containing heterocycle;
Z³ and Z⁵ are independently C1-C12 alkylene;
Z⁴ is -O-C(=O), -O-C(=S), -NH-C(=O), -NH-C (=S) , -O- or -S-;
Z⁶ is C1-C2 alkylene; and
n is an integer between 1 and 10, inclusive.

12. A method for producing metal-organic residue complex particles, wherein the method comprises reacting tetrachloroauric acid with a compound represented by the following formula:
(̵S-Y-(OCH₂CH₂)ₙ-NH-C(=O)-CH₂-O-NH₂)₂,
(̵S-Y-(OCH₂CH₂)ₙ-NH-C(=O)-CH₂-O-NH(CH₃))₂,
(̵S-Y-(OCH₂CH₂)ₙ-NH-C(=O) -CH(NH₂)-W⁴-SH)₂,
(̵S-Y-(OCH₂CH₂)ₙ-NH-C(=S)-CH(NH₂)-W⁴-SH)₂,
(̵S-W¹-O-NH₂)₂,
(̵S-W¹-O-NH(CH₃))₂,
(̵S-W¹-O-W²-O-NH₂)₂,
(̵S-W¹-O-W²-O-NH(CH₃))₂,
(̵S-(CH₂CH₂O)ₙ-W¹-O-W²-O-NH₂)₂,
(̵S-(CH₂CH₂O)ₙ-W¹-O-W²-O-NH(CH³))₂,
(̵S-W¹-C(=O)-NH-NH₂)₂,
(̵S-W¹-C(=S)-NH-NH₂)₂,
(̵S-W¹-NH-C(=O)-CH(NH₂)-W⁴-SH)₂,
(̵S-W¹-NH-C(=S)-CH(NH₂)-W⁴-SH)₂,
(̵S-Z¹-Z²-Z³-Z⁴-Z⁵-O-NH₂)₂,
(̵S-Z¹-Z²-Z³-Z⁴-Z⁵-O-NH(CH₃))₂,
(̵S-Z¹-Z²-Z³-Z⁴-CH(NH₂)-O-Z⁶-SH)₂,
(̵S-Z¹-Z²-Z³-Z⁴-CH(NH₂)-Z⁶-SH)₂,
(̵S-Z¹-O-Z³-CH(NH₂)-Z⁶-SH)₂,
(̵S-Z¹-O-Z³-O-NH₂)₂,
(̵S-Z¹-O-Z³-O-NH(CH₃))₂,
(̵S-Z¹-O-Z³-Z⁴-Z⁵-O-NH₂)₂,
(̵S-Z¹-O-Z³-Z⁴-Z⁵-O-NH(CH₃))₂,
(̵S-Z¹-O-Z³-Z⁴-CH(NH₂)-O-Z⁶-SH)₂,
(̵S-Z¹-O-Z³-Z⁴-CH(NH₂)-Z⁶-SH)₂,
(̵S-Z¹-Z³-Z⁴-Z⁵-O-NH₂)₂,
(̵S-Z¹-Z³-Z⁴-Z⁵-O-NH(CH₃))₂,
(̵S-Z¹-Z³-Z⁴-CH(NH₂)-O-Z⁶-SH)₂,
(̵S-Z¹-Z³-Z⁴-CH(NH₂)-Z⁶-SH)₂
or 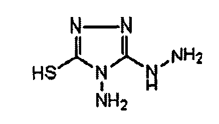 ,or a salt thereof, in the presence of a reducing agent, wherein, Y, W¹ and W² are independently C1-C12 alkylene, C2-C12 alkenylene or C2-C12 alkynylene;
W⁴ is C1-C2 alkylene;
Z¹ is substituted or unsubstituted arylen or heteroarylen;
Z² is a nitrogen-containing heterocycle;
Z³ and Z⁵ are independently C1-C12 alkylene;
Z⁴ is -O-C(=O), -O-C(=S), -NH-C(=O), -NH-C (=S) , -O- or -S-;
Z⁶ is C1-C2 alkylene; and
n is an integer between 1 and 10, inclusive.

13. A method for trapping a sugar chain or a sugar chain-containing substance, **characterized in that** the method comprises contacting a metal-organic residue complex with a sugar chain or a sugar chain-containing substance, under conditions where the metal-organic residue complex and the sugar chain or the sugar chain-containing substance may react with each other,
the metal-organic residue complex has a metal bound to a group represented by the following formula:
-S-Y-(OCH₂CH₂)ₙ-NH-C(=O)-CH₂-O-NH₂,
-S-Y-(OCH₂CH₂)ₙ-NH-C(=O)-CH₂-O-NH(CH₃),
-S-Y-(OCH₂CH₂)ₙ-NH-C(=O)-CH(NH₂)-W⁴-SH,
-S-Y-(OCH₂CH₂)ₙ-NH-C(=S)-CH(NH₂)-W⁴-SH,
-S-W¹-O-NH₂,
-S-W¹-O-NH(CH₃),
-S-W¹-O-W²-O-NH₂,
-S-W¹-O-W²-O-NH(CH₃),
-S-(CH₂CH₂O)ₙ-W¹-O-W²-O-NH₂,
-S-(CH₂CH₂O)ₙ-W¹-O-W²-O-NH(CH₃),
-S-W¹-C(=O)-NH-NH₂,
-S-W¹-C(=S)-NH-NH₂,
-S-W¹-NH-C(=O)-CH(NH₂)-W⁴-SH,
-S-W¹-NH-C(=S)-CH(NH₂)-W⁴-SH,
-S-Z¹-Z²-Z³-Z⁴-Z⁵-O-NH₂,
-S-Z¹-Z²-Z³-Z⁴-Z⁵-O-NH(CH₃),
-S-Z¹-Z²-Z³-Z⁴-CH(NH₂)-O-Z⁶-SH,
-S-Z¹-Z²-Z³-Z⁴-CH(NH₂)-Z⁶-SH,
-S-Z¹-O-Z³-CH(NH₂)-Z⁶-SH,
-S-Z¹-O-Z³-O-NH₂,
-S-Z¹-O-Z³-O-NH(CH₃),
-S-Z¹-O-Z³-Z⁴-Z⁵-O-NH₂,
-S-Z¹-O-Z³-Z⁴-Z⁵-O-NH(CH₃),
-S-Z¹-O-Z³-Z⁴-CH(NH₂)-O-Z⁶-SH
-S-Z¹-O-Z³-Z⁴-CH(NH₂) -Z⁶-SH
-S-Z¹-Z³-Z⁴-Z⁵-O-NH₂,
-S-Z¹-Z³-Z⁴-Z⁵-O-NH(CH₃),
-S-Z¹-Z³-Z⁴-CH(NH₂)-O-Z⁶-SH,
-S-Z¹-Z³-Z⁴-CH(NH₂)-Z⁶-SH,
or 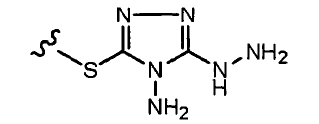 wherein, Y, W¹ and W² are independently C1-C12 alkylene, C2-C12 alkenylene or C2-C12 alkynylene;
W⁴ is C1-C2 alkylene;
Z¹ is substituted or unsubstituted arylen or heteroarylen;
Z² is a nitrogen-containing heterocycle;
Z³ and Z⁵ are independently C1-C12 alkylene;
Z⁴ is -O-C(=O), -O-C(=S), -NH-C(=O), -NH-C (=S) , -O- or -S-;
Z⁶ is C1-C2 alkylene; and
n is an integer between 1 and 10, inclusive.

14. Amethod for measuring the molecular weight of a substance which may interact with an organic residue of a metal-organic residue complex, comprising the steps of:
1) contacting the metal-organic residue complex with a substance which may interact with the organic residue, wherein the metal is bound through a sulfur atom to organic residue;
2) obtaining the metal-organic residue complex bound to the substance which may interact; and
3) ionizing the obtained metal-organic residue complex into derivatives of the organic residue, wherein the organic residue contains a sulfur atom.

15. A method for performing mass spectrometry of a sugar chain or a sugar chain-containing substance, comprising the steps of:
1) contacting a compound with a metal, wherein the compound is represented by the following formula: 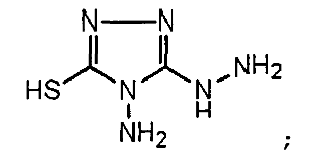
2) contacting the metal-organic residue complex obtained in 1) with a sugar chain or a sugar chain-containing substance under conditions where the metal-organic residue complex and the sugar chain or the sugar chain-containing substance may react with each other; and
3) ionizing the metal-organic residue complex obtained in 2) into derivatives of the organic residue, wherein the organic residue contains a sulfur atom.

16. A method for performing mass spectrometry of a sugar chain or a sugar chain-containing substance, comprising the steps of:
1) contacting a compound represented by the following formula: 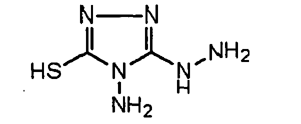 with a sugar chain or a sugar chain-containing substance under conditions where the compound and the sugar chain or the sugar chain-containing substance may react with each other;
2) contacting the compound obtained in 1) with a metal; and
3) ionizing the metal-organic residue complex obtained in 2) into derivatives of the organic residue, wherein the organic residue contains a sulfur atom.

17. A composition for trapping a sugar chain, comprising
a compound represented by the general formula (II):
R-SH (II) or a salt thereof, wherein R is an organic residue; and S is a sulfur atom;
a compound represented by the general formula (III):
R-S-S-R (III) or a salt thereof, wherein, R and S are the same as defined above;
a compound represented by the general formula (V):
HS-X-CH(R)-SH (V) or a salt thereof, wherein R and S are the same as defined above; and X is lower alkylene or lower alkenylene; or
a compound represented by the general formula (VI): 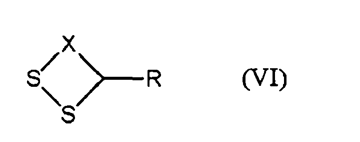 or a salt thereof, wherein, R, S and X are the same as defined above; or a mixture thereof.

18. A composition for trapping a sugar chain, comprising a compound represented by the following formula:
-(S-Y-(OCH₂CH₂)ₙ-NH-C(=O)-CH₂-O-NH₂)₂,
-(S-Y-(OCH₂CH₂)ₙ-NH-C(=O)-CH₂-O-NH(CH₃))₂,
-(S-Y-(OCH₂CH₂)ₙ-NH-C(=O)-CH(NH₂)-W⁴-SH)₂.
-(S-Y-(OCH₂CH₂)ₙ-NH-C(=S)-CH(NH₂)-W⁴-SH)₂,
-(S-W¹-O-NH₂)₂,
-(S-W¹-O-NH(CH₃)₂,
-(S-W¹-O-W²-O-NH₂)₂,
-(S-W¹-O-W²-O-NH(CH₃))₂,
-(S- (CH₂CH₂O)ₙ-W¹-O-W²-O-NH₂)₂,
-(S-(CH₂CH₂O)ₙ-W¹-O-W²-O-NH(CH₃))₂,
-(S-W¹-C(=O)-NH-NH₂)₂,
-(S-W¹-C(=S)-NH-NH₂)₂,
-(S-W¹-NH-C(=O)-CH(NH₂)-W⁴-SH)₂,
-(S-W¹-NH-C(=S)-CH (NH₂)-W⁴-SH)₂,
-(S-Z¹-Z²-Z³-Z⁴-Z⁵-O-NH₂)₂.
-(S-Z¹-Z²-Z³-Z⁴-Z⁵-O-NH(CH₃))₂,
-(S-Z¹-Z²-Z³-Z⁴-CH(NH₂)-O-Z⁶-SH)₂,
-(S-Z¹-Z²-Z³-Z⁴-CH(NH₂)-Z⁶-SH)₂,
-(S-Z¹-O-Z³-CH(NH₂)-Z⁶-SH)₂,
-(S-Z¹-O-Z³-O-NH₂)₂,
-(S-Z¹-O-Z³-O-NH(CH₃))₂,
-(S-Z¹-O-Z³-Z⁴-Z⁵-O-NH₂)₂,
-(S-Z¹-O-Z³-Z⁴-Z⁵-O-NH(CH₃))_{2,}
-(S-Z¹-O-Z³-Z⁴-CH(NH₂)-O-Z⁶-SH)₂,
-(S-Z¹-O-Z³-Z⁴-CH(NH₂)-Z⁶-SH)₂,
-(S-Z¹-Z³-Z⁴-Z⁵-O-NH₂)₂,
-(S-Z¹-Z³-Z⁴-Z⁵-O-NH(CH₃))_{2,}
-(S-Z¹-Z³-Z⁴-CH(NH₂)-O-Z⁶-SH)₂,
-(S-Z¹-Z³-Z⁴-CH(NH₂)-Z⁶-SH)₂
or 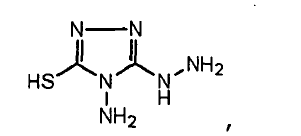 wherein Y, W¹ and W² are independently C1-C12 alkylene, C2-C12 alkenylene or C2-C12 alkynylene;
W⁴ is C1-C2 alkylene;
Z¹ is substituted or unsubstituted arylen or heteroarylen;
Z² is a nitrogen-containing heterocycle;
Z³ and Z⁵ are independently C1-C12 alkylene;
Z⁴ is -O-C(=O), -O-C(=S), -NH-C(=O), -NH-C (=S) , -O- or -S-;
Z⁶ is C1-C2 alkylene; and
n is an integer between 1 and 10, inclusive.

19. A metal-organic residue complex represented by the following formula:
M²-(S-Y-(OCH₂CH₂)ₙ-NH-C(=O)-CH₂-O-NH₂)ₘ,
M²-(S-Y-(OCH₂CH₂) ₙ-NH-C (=O)-CH₂-O-NH (CH₃))ₘ,
M²- (S-Y-(OCH₂CH₂) ₙ-NH-C(=O)-CH(NH₂)-W⁴-SH)ₘ,
M²- (S-Y-(OCH₂CH₂)ₙ-NH-C(=S)-CH(NH₂)-W⁴-SH)ₘ,
M²-(S-W¹-O-NH₂)ₘ,
M²-(S-W¹-O-NH(CH₃))m,
M²- (S-W¹-O-W²-O-NH₂)ₘ,
M²-(S-W¹-O-W²-O-NH(CH₃))ₘ,
M²- (S-(CH₂CH₂O)ₙ-W¹-O-W²-O-NH₂)ₘ,
M²- (S-(CH₂CH₂O)ₙ-W¹-O-W²-O-NH(CH₃))ₘ,
M²-(S-W¹-C(=O) -NH-NH₂)ₘ,
M²-(S-W¹-C(=S)-NH-NH₂)ₘ,
M²-(S-W¹-NH-C(=O)-CH(NH₂)-W⁴-SH)ₘ,
M²-(S-W¹-NH-C(=S)-CH (NH₂)-W⁴-SH)ₘ,
M²-(S-Z¹-Z²-Z³-Z⁴-Z⁵-O-NH₂)ₘ,
M²-(S-Z¹-Z²-Z³-Z⁴-Z⁵-O-NH(CH₃))ₘ,
M²-(S-Z¹-Z²-Z³-Z⁴-CH(NH₂)-O-Z⁶-SH)ₘ,
M²-(S-Z¹-Z²-Z³-Z⁴-CH(NH₂)-Z⁶-SH)ₘ,
M²-(S-Z¹-O-Z³-CH(NH₂)-Z⁶-SH)ₘ,
M²-(S-Z¹-O-Z³-O-NH₂)ₘ,
M²-(S-Z¹-O-Z³-O-NH(CH₃))ₘ,
M²-(S-Z¹-O-Z³-Z⁴-Z⁵-O-NH₂)ₘ,
M²-(S-Z¹-O-Z³-Z⁴-Z⁵-O-NH(CH₃))ₘ,
M²-(S-Z¹-O-Z³-Z⁴-CH(NH₂)-O-Z⁶-SH)ₘ,
M²-(S-Z¹-O-Z³-Z⁴-CH(NH₂)-Z⁶-SH)ₘ,
M²-(S-Z¹-Z³-Z⁴-Z⁵-O-NH₂)ₘ,
M²-(S-Z¹-Z³-Z⁴-Z⁵-O-NH(CH₃))ₘ,
M²-(S-Z¹-Z³-Z⁴-CH(NH₂)-O-Z⁶-SH)ₘ,
m²-(S-Z¹-Z³-Z⁴-CH(NH₂)-Z⁶-SH)ₘ,
or the general formula (VII): 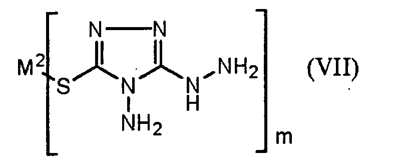
wherein, M² is a metal;
m indicates a stoichiometric ratio of an organic residue with respect to M² and is an integer equal to or greater than 1, wherein the organic residue contains a sulfur atom;
Y, W¹ and W² are independently C1-C12 alkylene, C2-C12 alkenylene or C2-C12 alkynylene;
W⁴ is C1-C2 alkylene;
Z¹ is substituted or unsubstituted arylen or heteroarylen ;
Z² is a nitrogen-containing heterocycle;
Z³ and Z⁵ are independently C1-C12 alkylene;
Z' is -O-C(=O), -O-C(=S) , -NH-C(=O), -NH-C (=S) , -O- or -S-;
Z⁶ is C1-C2 alkylene; and
n is an integer between 1 and 10, inclusive.

20. A composition for trapping a sugar chain, comprising:
a metal-organic residue complex represented by the general formula (I):
(R-S)ₙ-M¹ (I),
wherein R is an organic residue; S is a sulfur atom; M¹ is a metal; and n indicates a stoichiometric ratio of (R-S) group with respect to M¹ and is an integer equal to or greater than 1; or
a metal-organic residue complex represented by the general formula (IV):
M¹-S-X-CH(R) -S-M¹ (IV),
wherein R and S are the same as defined above, M¹ at both ends are a metal of the same substance and X is lower alkylene or lower alkenylene.

21. A composition for trapping a sugar chain, comprising ametal-organic residue complex, represented by the following formula:
M²-(S-Y-(OCH₂CH₂)ₙ-NH-C(=O)-CH₂-O-NH₂)ₘ,
M²-(S-Y-(OCH₂CH₂)ₙ-NH-C(=O)-CH₂-O-NH(CH₃))ₘ,
M²-(S-Y-(OCH₂CH₂)ₙ-NH-C(=O)-CH (NH₂)-W⁴-SH)ₘ,
M²-(S-Y-(OCH₂CH₂)ₙ-NH-C(=S)-CH (NH₂)-W⁴-SH)ₘ,
M²-(S-W¹-O-NH₂)ₘ,
M²-(S-W¹-O-NH(CH₃)ₘ,
M²-(S-W¹-O-W²-O-NH²)ₘ,
M²-(S-W¹-O-W²-O-NH(CH₃))ₘ,
M²-( S- (CH₂CH₂O)ₙ-W¹-O-W²-O-NH₂)ₘ,
M²-( S- (CH₂CH₂O)ₙ-W¹-O-W²-O-NH(CH₃))ₘ,
M²- (S-W¹-C (=O) -NH-NH₂)ₘ,
M²- (S-W¹-C (=S) -NH-NH₂)ₘ,
M²- (S-W¹-NH-C (=O) -CH (NH₂) -W⁴-SH)ₘ,
M²- (S-W¹-NH-C (=S) -CH (NH₂) -W⁴-SH)ₘ,
M²-(S-Z¹-Z²-Z³-Z⁴-Z⁵-O-NH₂)ₘ,
M²-(S-Z¹-Z²-Z³-Z⁴-Z⁵-O-NH(CH₃))ₘ,
M²- (S-Z¹-Z²-Z³-Z⁴-CH (NH₂) -O-Z⁶-SH)ₘ,
M²- (S-Z¹-Z²-Z³-Z⁴-CH (NH₂) -Z⁶-SH)ₘ,
M²-(S-Z¹-O-Z³-CH(NH₂)-Z⁶-SH)ₘ,
M²-(S-Z¹-O-Z³-O-NH₂)ₘ,
M²-(S-Z¹-O-Z³-O-NH(CH₃))ₘ,
M²-(S-Z¹-O-Z³-Z⁴-Z⁵-O-NH₂)ₘ,
M²-(S-Z¹-O-Z³-Z⁴-Z⁵-O-NH(CH₃))ₘ,
M²-(S-Z¹-O-Z³-Z⁴-CH(NH₂)-O-Z⁶-SH)ₘ,
M²-(S-Z¹-O-Z³-Z⁴-CH(NH₂)-Z⁶-SH)ₘ,
M²-(S-Z¹-Z³-Z⁴-Z⁵-O-NH₂)ₘ,
M²-(S-Z¹-Z³-Z⁴-Z⁵-O-NH(CH₃))ₘ,
M²-(S-Z¹-Z³-Z⁴-CH(NH₂)-O-Z⁶-SH)ₘ,
M²-(S-Z¹-Z³-Z⁴-CH(NH₂)-Z⁶-SH)ₘ,
or the general formula (VII): 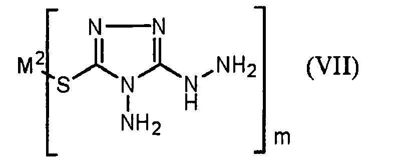
wherein,
M² is a metal;
m indicates a stoichiometric ratio of an organic residue with respect to M² and is an integer equal to or greater than 1, wherein the organic residue comprises a sulfur atom;
Y, W¹ and W² are independently C1-C12 alkylene, C2-C12 alkenylene or C2-C12 alkynylene;
W⁴ is C1-C2 alkylene;
Z¹ is substituted or unsubstituted arylen or heteroarylen;
Z² is a nitrogen-containing heterocycle;
Z³ and Z⁵ are independently C1-C12 alkylene;
Z⁴ is -O-C(=O), -O-C(=S), -NH-C (=O), -NH-C (=S) , -O- or -S-;
Z⁶ is C1-C2 alkylene and
n is an integer between 1 and 10, inclusive.

22. A kit for mass spectrometry of a sugar chain or a sugar chain-containing substance, comprising:
A) a compound represented by the general formula (II):
R-SH (II)
or a salt thereof, wherein R is an organic residue; and S is a sulfur atom;
a compound represented by the general formula (III)
R-S-S-R (III)
or a salt thereof, wherein R and S are the same as defined above;
a compound represented by the general formula (V):
HS-X-CH(R)-SH (V)
or a salt thereof, wherein R and S are the same as defined above; and X is lower alkylene or lower alkenylene; or
a compound represented by the general formula (VI): 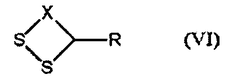 or a salt thereof, wherein R, S and X are the same as defined above; or a mixture thereof; and
B) a metal.

23. A kit for mass spectrometry of a sugar chain or a sugar chain-containing substance, comprising:
A) a sulfur atom containing derivatives of an organic residue, represented by the following formula:
-(S-Y-(OCH₂CH₂)ₙ-NH-C(=O)-CH₂-O-NH₂)₂,
-(S-Y-(OCH₂CH₂)ₙ-NH-C(=O)-CH₂-O-NH(CH₃))₂,
-(S-Y-(OCH₂CH₂)ₙ-NH-C(=O)-CH(NH₂)-W⁴-SH)₂,
-(S-Y-(OCH₂CH₂)ₙ-NH-C(=S)-CH(NH₂)-W⁴-SH)₂,
-(S-W¹-O-NH₂)₂,
-(S-W¹-O-NH(CH₃))₂,
-(S-W¹-O-W²-O-NH₂)₂,
-(S-W¹-O-W²-O-NH(CH₃))₂,
-(S-(CH₂CH₂O)ₙ-W¹-O-W²-O-NH₂)₂,
-(S-(CH₂CH₂O)ₙ-W¹-O-W²-O-NH(CH₃))₂,
-(S-W¹-C(=O)-NH-NH₂)₂,
-(S-W¹-C(=S)-NH-NH₂)₂,
-(S-W¹-NH-C(=O)-CH(NH₂)-W⁴-SH)₂,
-(S-W¹-NH-C(=S)-CH (NH₂)-W⁴-SH)₂,
-(S-Z¹-Z²-Z³-Z⁴-Z⁵-O-NH₂)₂.
-(S-Z¹-Z²-Z³-Z⁴-Z⁵-O-NH(CH₃))₂,
-(S-Z¹-Z²-Z³-Z⁴-CH(NH₂)-O-Z⁶-SH)₂,
-(S-Z¹-Z²-Z³-Z⁴-CH(NH₂)-Z⁶-SH)₂,
-(S-Z¹-O-Z³-CH(NH₂)-Z⁶-SH)₂,
-(S-Z¹-O-Z³-O-NH₂)₂,
-(S-Z¹-O-Z³-O-NH(CH₃))₂,
-(S-Z¹-O-Z³-Z⁴-Z⁵-O-NH₂)₂,
-(S-Z¹-O-Z³-Z⁴-Z⁵-O-NH(CH₃))₂,
-(S-Z¹-O-Z³-Z⁴-CH(NH₂)-O-Z⁶-SH)₂,
-(S-Z¹-O-Z³-Z⁴-CH(NH₂)-Z⁶-SH)₂,
-(S-Z¹-Z³-Z⁴-Z⁵-O-NH₂)₂,
-(S-Z¹-Z³-Z⁴-Z⁵-O-NH(CH₃))₂,
-(S-Z¹-Z³-Z⁴-CH(NH₂)-O-Z⁶-SH)₂,
-(S-Z¹-Z³-Z⁴-CH(NH₂)-Z⁶-SH)₂
or 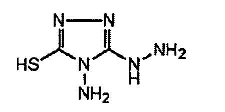 wherein Y, W¹ and W² are independently C1-C12 alkylene, C2-C12 alkenylene or C2-C12 alkynylene;
W⁴ is C1-C2 alkylene;
Z¹ is substituted or unsubstituted arylen or heteroarylen;
Z² is a nitrogen-containing heterocycle;
Z³ and Z⁵ are independently C1-C12 alkylene;
Z⁴ is -O-C(=O), -O-C (=S), -NH-C (=O), -NH-C (=S) , -O- or -S-;
Z⁶ is C1-C2 alkylene; and
n is an integer between 1 and 10, inclusive; and
B) a metal.

24. A kit for mass spectrometry of a sugar chain or a sugar chain-containing substance, comprising:
a metal-organic residue complex represented by the general formula (I):
(R-S)ₙ-M¹ (I)
wherein, R is an organic residue, S is a sulfur atom, M¹ is a metal and n indicates a stoichiometric ratio of (R-S) group with respect to M¹ and is an integer equal to or greater than 1; or
a metal-organic residue complex represented by the general formula (IV):
M¹-S-X-CH(R)-S-M¹ (IV)
wherein R and S are the same as defined above, M¹ at both ends are same metal entities and X is lower alkylene or lower alkenylene.

25. A kit for mass spectrometry of a sugar chain or a sugar chain-containing substance, comprising a metal-organic residue complex, represented by the following formula:
M²-(S-Y-(OCH₂CH₂)ₙ-NH-C (=O) -CH₂-O-NH₂)m,
M²-(S-Y-(OCH₂CH₂)ₙ-NH-C(=O)-CH₂-O-NH(CH₃))ₘ,
M²-(S-Y- (OCH₂CH₂)ₙ-NH-C (=O)-CH(NH₂) -W⁴-SH)ₘ,
M²-(S-Y-(OCH₂CH₂)ₙ-NH-C(=S)-CH (NH₂) -W⁴-SH)ₘ,
M²-(S-W¹-O-NH₂)ₘ,
M²-(S-W¹-O-NH(CH₃))ₘ,
M²-(S-W¹-O-W²-O-NH₂)ₘ,
M²-(S-W¹-O-W²-O-NH(CH₃))ₘ,
M²-(S-(CH₂CH₂O)ₙ-W¹-O-W²-O-NH₂)ₘ,
M²- (S- (CH₂CH₂O)ₙ-W¹-O-W²-O-NH(CH₃))ₘ,
M²-(S-W¹-C(=O)-NH-NH₂)ₘ,
M²-(S-W¹-C(=S)-NH-NH₂)ₘ,
M²-(S-W¹-NH-C(=O)-CH (NH₂)-W⁴-SH)ₘ,
M²-(S-W¹-NH-C(=S)-CH (NH₂)-W⁴-SH)ₘ,
M²-(S-Z¹-Z²-Z³-Z⁴-Z⁵-O-NH₂)ₘ,
M²-(S-Z¹-Z²-Z³-Z⁴-Z⁵-O-NH(CH₃))ₘ,
M²-(S-Z¹-Z²-Z³-Z⁴-CH(NH₂)-O-Z⁶-SH)ₘ,
M²-(S-Z¹-Z²-Z³-Z⁴-CH(NH₂)-Z⁶-SH)ₘ,
M²-(S-Z¹-O-Z³-CH(NH₂) -Z⁶-SH)ₘ,
M²-(S-Z¹-O-Z³-O-NH₂)ₘ,
M²-(S-Z²-O-Z³-O-NH(CH₃))m,
M²-S-Z¹-O-Z³-Z⁴-Z⁵-O-NH₂)ₘ,
M²-(S-Z¹-O-Z³-Z⁴-Z⁵-O-NH(CH₃))ₘ,
M²-(S-Z¹-O-Z³-Z⁴-CH(NH₂)-O-Z⁶-SH)ₘ,
M²-(S-Z¹-O-Z³-Z⁴-CH(NH₂)-Z⁶-SH)ₘ,
M²-(S-Z¹-Z³-Z⁴-Z⁵-O-NH₂)ₘ,
M²-(S-Z¹-Z³-Z⁴-Z⁵-O-NH(CH₃))ₘ,
M²-(S-Z¹-Z³-Z⁴-CH(NH₂)-O-Z⁶-SH)ₘ,
M²-(S-Z¹-Z³-Z⁴-CH(NH₂)-Z⁶-SH)ₘ,
or the general formula (VII): 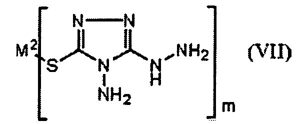
wherein, M² is a metal, m indicates a stoichiometric ratio of an organic residue with respect to M² and is an integer equal to or greater than one, the organic residue comprises a sulfur atom, Y, W¹ and W² are independently C1-C12 alkylene, C2-C12 alkenylene or C2-C12 alkynylene, W⁴ is C1-C2 alkylene; Z¹ is substituted or unsubstituted arylen or heteroarylen;
Z² is a nitrogen-containing heterocycle, Z³ and Z⁵ are independently C1-C12 alkylene, Z⁴ is -O-C(=O), -O-C(=S), -NH-C(=O), -NH-C (=S) , -O- or -S-, Z⁶ is C1-C2 alkylene; and
n is an integer between 1 and 10, inclusive.

26. A method according to any one of claims 1 to 6, wherein the mass spectrometry is carried out by LDI-TOF MS method.

27. A method according to claim 10, wherein the mass spectrometry is carried out by LDI-TOF MS method.
